(19)

Europäisches
Patentamt
European
Patent Office
Office européen
des brevets

(11) **EP 4 674 430 A1**

(12) **EUROPEAN PATENT APPLICATION**

(43) Date of publication:
**07.01.2026 Bulletin 2026/02**

(21) Application number: **24185787.9**

(22) Date of filing: **01.07.2024**

(51) International Patent Classification (IPC):
***A61K 39/00*** *(2006.01)* ***A61P 31/18*** *(2006.01)*
***A61P 31/22*** *(2006.01)*

(52) Cooperative Patent Classification (CPC):
**A61K 40/11; A61K 40/31; A61K 40/4211;**
**A61K 40/4215; A61P 31/18; A61P 31/22;**
**C12N 5/0638;** A61K 40/50; A61K 2239/13;
A61K 2239/46; C12N 15/1138; C12N 2310/20;
C12N 2501/2307; C12N 2501/2315; C12N 2501/51;
(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicants:
 • **Universität zu Köln**
  **50923 Köln (DE)**
 • **Universität Heidelberg**
  **69117 Heidelberg (DE)**

(72) Inventors:
 • **Stripecke, Renata**
  **30175 Hannover (DE)**

 • **Damrat, Michael**
  **51103 Köln (DE)**
 • **Kleid, Jan-Malte**
  **50676 Köln (DE)**
 • **Lusic, Marina**
  **69126 Heidelberg (DE)**

(74) Representative: **Hertin und Partner**
 **Rechts- und Patentanwälte PartG mbB**
 **Kurfürstendamm 63**
 **10707 Berlin (DE)**

Remarks:
The complete document including Reference
Table(s) and the Sequence Listing(s) can be
downloaded from the EPO website

(54) **EDITED CYTOTOXIC CELLS WITH CHIMERIC ANTIGEN RECEPTORS TARGETING CD30 FOR THE TREATMENT OF INFECTIONS**

(57) The invention relates to a gene-edited cytotoxic cell comprising a chimeric antigen receptor (CAR) that binds CD30, for use in the treatment of an infection, in which infected cells express CD30. In embodiments, the treatment comprises the cytotoxic cell binding to a human target cell, which is infected with a chronic inflammatory virus and expresses CD30. In embodiments, the treatment comprises cytotoxic activity against a reservoir of infected human CD30-expressing target cells in a subject with a chronic viral infection with associated pathologies, immune dysregulations and autoimmune diseases. Corresponding medical uses and methods of treatment are encompassed by the present invention. In another aspect, the invention relates to the CD30-CAR-expressing gene-edited cytotoxic cell as described herein. In other aspects, the invention relates to the CD30-CAR nucleic acid construct and the CAR polypeptide as described herein.

**EP 4 674 430 A1**

(52) Cooperative Patent Classification (CPC): (Cont.)
C12N 2710/16211; C12N 2740/16011

## Description

The invention relates to the field of molecular biology and recombinant technology, chimeric antigen receptors and cell therapy of medical conditions.

The invention relates to a genetically modified cytotoxic cell comprising a chimeric antigen receptor (CAR) that binds CD30, for use in the treatment of an infection, in which infected cells express CD30. The invention further relates to a gene-edited cytotoxic cell comprising a chimeric antigen receptor (CAR) that binds CD30, for use in the treatment of an infection, in which infected cells express CD30.

In embodiments, in infected target cells, expression and/or presentation of CD30 is upregulated over uninfected cells. In embodiments, the treatment comprises the cytotoxic cell binding to a human target cell, which is infected with a chronic or acute inflammatory virus and expresses CD30, and activation of said cytotoxic cell, thereby inducing cytotoxic activity against said human target cell and/or a bystander inflammatory cell.

In embodiments, the treatment comprises the cytotoxic activity of the cytotoxic cell against a reservoir of infected human CD30-expressing target cells in a subject with a chronic or acute viral infection, said reservoir comprising a latent and lytic viral reservoir, preferably wherein said treatment prevents viral reactivation.

In embodiments, the treatment comprises autologous or allogeneic cell therapy.

In further embodiments, the cytotoxic cell is administered in a pharmaceutical composition with one or more pharmaceutically acceptable excipients. Corresponding medical uses and methods of treatment are encompassed by the present invention.

In another aspect, the invention further relates to the CD30-CAR-expressing genetically modified cytotoxic cell as described herein. In other aspects, the invention further relates to the CD30-CAR nucleic acid construct and the CAR polypeptide as described herein.

## BACKGROUND OF THE INVENTION

Latent viruses have the capacity to remain dormant within host cells, refraining from producing new virus particles while the viral genome persists within the infected cell. Despite lying dormant, these viruses can reactivate under specific conditions, resulting in the resumption of viral replication and the recurrence of symptoms. Several viruses, including herpesviruses (such as herpes simplex virus 1 and 2 / HSV-1 and HSV-2, Epstein-Barr virus /EBV, and human cytomegalovirus/ HCMV), human immune deficiency virus/HIV, human T-lymphotropic virus /HTLV and hepatitis B virus/HBV, can establish latent infections.

EBV infects over 95% of the adult population and is linked to approximately 2% of all human malignancies. Immune-compromised or immune-deficient patients are susceptible to EBV or HCMV infections, which can lead to severe complications. Managing EBV-positive post-transplant lymphoproliferative disease (LPD) or HCMV reactivation poses challenges due to the absence of approved treatments. Adoptive transfer of autologous or allogeneic T cells reactive against EBV or HCMV serves as a salvage therapy for patients who have relapsed or are refractory to drugs. This approach relies on the existence of donor memory T cells recognizing viral antigenic epitopes via the T cell receptors (TCR) and human leukocyte antigen (HLAs) interactions. In the allogenic context, the HLAs between the donor and recipient have to be optimally matched.

While many individuals harboring latent infections of EBV, HCMV, or HTLV remain asymptomatic, these viruses are linked to various serious diseases, including cancer. For example, EBV is associated with conditions such as mononucleosis and lymphomas affecting B, T, and natural killer (NK) cells, as well as nasopharyngeal or gastric epithelial cancers. HCMV is implicated in mononucleosis and glioblastoma, while HTLV is associated with T-cell leukemia/lymphoma and inflammatory diseases such as the HTLV-1 associated myelopathy/tropical spastic paraparesis (HAM/TSP). These associations underscore the potential health risks posed by latent infections of these viruses.

HIV establishes latent infections primarily in memory $CD4^+$ T cells, forming a reservoir that persists indefinitely despite potent antiretroviral therapy (ART). This reservoir serves as a major obstacle to curing HIV-1 infection due to its ability to evade immune detection and viral replication. Untreated, HIV can lead to acquired immunodeficiency syndrome (AIDS), and the patients are at higher risk of developing lymphomas. Although some drugs are used to reduce the viral load, no marketed treatments are available to provide long-term immune control of the latent virus reservoir.

Furthermore, viral infections like EBV and HCMV have been linked to the development of autoimmune disorders such as rheumatoid diseases, systemic lupus erythematosus, and multiple sclerosis. Similarly, HTLV infections are associated with conditions like uveitis, Sjögren's syndrome, and rheumatoid arthritis, while HIV infections can lead to rheumatoid diseases, systemic lupus erythematosus, anti-phospholipid syndrome, and vasculitis. Despite advancements in medical care, only palliative treatments exist to manage the autoimmune inflammation associated with these conditions, with no definitive curative approaches available.

CD30, a member of the TNF receptor superfamily 8, plays multiple roles in regulating immune responses. It acts as a positive regulator of apoptosis and limits the proliferation of auto-reactive $CD8^+$ T cells (van der Weyden, Blood Cancer J., 2017). CD30 is expressed in a subset of memory T and B cells and is notably upregulated in some EBV-positive lymphomas, as well as on polyclonal B cells infected with EBV (van der Weyden, Blood Cancer J., 2017). The EBV protein LMP1 mediates this upregulation, although its precise contribution to EBV tumorigenesis remains unclear. Moreover, CD30 expression on CD4[+] T cells increases significantly before HIV-1 rebound, indicating its involvement in HIV infection dynamics (Prator, J Infect Dis., 2020). Additionally, CD30 is expressed in a subset of adult T cell leukemia/lymphoma cases associated with HTLV-1 infection (Watanabe, Int J Hematol., 2023).

Further analysis of the CD30-positive T cell subsets in peripheral blood (PB) and synovial fluid (SF) of rheumatoid arthritis suggested the involvement of CD30/CD30L signaling in the polarization of T cells towards a Th17 phenotype with pro-inflammatory features. An elevated expression of CD30 has been observed on regulatory T cells (Tregs) of rheumatoid arthritis patients (Barbieri, J Immunol Res., 2015).

Several CD30-CAR constructs are known in the art, however, none of which have been applied in the treatment of infections and associated pathologies. WO2017066122A1 discloses a CAR-T that recognizes CD30 for treating cancer, specifically lymphoma. Choi et al. discloses CD30-CAR-T cells used in the treatment of Hodgkin lymphomas. Seif et al. discuss various strategies for using CAR-T cells to treat viral infections. However, the approach involves targeting viral antigens, which pose a challenge due to the common occurrence of mutant escape variants in viruses, rendering the CAR-T cells potentially ineffective over time.

Adoptive immunotherapy using virus-specific T cells (VSTs), either autologous or allogeneic, presents a potential avenue for controlling latent viral reservoirs and preventing viral reactivations. VSTs, activated against viral antigens via the TCR, can be expanded in specialized laboratories and administered to patients. In the autologous approach, VSTs rely on the presence of functional memory T cells, often compromised or exhausted in chronic viral infections. However, challenges such as complex manufacturing processes and personalized product requirements hinder widespread use. In the allogeneic setting, matching HLAs between donor and recipient is crucial to prevent graft-versus-host disease (GVHD) and ensure VST cell engraftment. Obstacles like donor availability, prolonged product generation times, and potential GVHD effects from nonspecific TCRs limit the off-the-shelf applicability of allogeneic VSTs.

Hanley et al. demonstrated the feasibility of deriving HCMV-specific T cells from naive cells derived from cord blood for T cell therapy, offering a potential solution to the restricted availability of virus-specific T cells for immunoprophylaxis in HCMV-seronegative donors. Icheva et al. presented a protocol for isolating polyclonal EBV nuclear antigen 1 (EBNA-1)-specific T cells using an interferon gamma (IFN-γ) capture technique to address EBV reactivation after allogeneic stem-cell transplantation (SCT). However, both procedures depend on intrinsic properties of the T cells obtained from allogeneic donors (such as the TCR and HLA) and the large-scale manufacturing is hard to standardize.

Thus, although a number of potential allogeneic therapies are in development for viral infections, a significant need remains for providing effective and standardized means for addressing such medical disorders.

## SUMMARY OF THE INVENTION

In light of the prior art, one technical problem underlying the invention was providing alternative or improved means for treating a chronic infection. The present invention seeks to provide such means while avoiding the disadvantages of the prior art.

A further object of the invention was to provide alternative or improved means for treating a chronic infection, which is caused by bacteria or a virus, such as a herpesvirus or retrovirus.

Another object of the invention was to provide alternative or improved means of controlling or eliminating a reservoir of latent viral infections associated with severe pathologies.

Another object of the invention was to provide alternative or improved means for preventing viral reactivation in subjects with chronic infections.

A further object of the invention was to provide alternative or improved means for an off-the-shelf product with low GvHD risk and/or effects to be used in the treatment of acute or chronic infections

A further object of the invention was to provide alternative or improved means for treating autoimmune diseases associated with an inflammatory or chronic infection.

These problems are solved by the subject matter of the independent claims. Preferred embodiments of the present invention are provided by the dependent claims.

The present invention, in one aspect, relates to a genetically modified cytotoxic cell comprising a chimeric antigen receptor (CAR) that binds CD30 for use in the treatment of an infection in which infected cells express CD30.

The invention further relates to a gene-edited cytotoxic cell comprising a chimeric antigen receptor (CAR) that binds CD30, for use in the treatment of an infection, in which infected cells express CD30.

Hence, the genetically modified/gene-edited cytotoxic cell of the present invention represents a surprising and beneficial approach toward treating infections in which infected cells express CD30.

WO2017/066122 discloses a T cell expressing via a retroviral vector a CAR, while still expressing the endogenous TCR, that immunologically recognizes CD30 for autologous use in the treatment of cancer. However, the gene-edited cytotoxic cell comprising a CAR and lacking TCR of the present invention represents a surprising and beneficial approach toward treating the medical conditions described herein. To the knowledge of the inventors, the employment of an allogeneic CD30-CAR-T cell lacking the TCR has not been previously attempted or described as a promising approach to treating infections. The minimal (or even non-existent) unwanted side effects, due to the selectivity of the marker, also represent a beneficial and surprising aspect of the present invention.

The present invention therefore exploits human CD30 expression in infected target cells as a marker for infection and inflammation. This reduces the risks of targeting viral antigens, which may also be presented on infected cells but undergo mutation or change overtime, thus potentially rendering a CAR directed against a viral antigen to lose effectiveness. CD30 appears as a reliable and specific marker for infected cells, especially in the reservoir of infected cells that exist in chronic (preferably viral) infections in the body of an infected subject. CD30 appears to be expressed in both the lytic and latent viral reservoirs, for example in cases of e.g. EBV and/or HIV infection.

In preferred embodiments, the cytotoxic cell according to the present invention is not intended for use in the treatment of cancer, or is not intended for use in the treatment of cancer as the primary disease *per se*. In embodiments, the subject of treatment does not have cancer. In embodiments of the invention, if the subject develops cancer that may be treated by the invention, it is considered a secondary pathology due to the primary chronic infection with the virus and associated immune dysregulations. Thus, any treatment of cancer as may be encompassed by embodiments of the invention, preferably comprises treatment of the infection as such, in addition to any treatment of cancer. In embodiments, the target cell to be lysed by the cytotoxic cell expressing a CD30-CAR is not a cancerous cell.

It was further surprising that the CRISPR/Cas gene-edited CD30-CAR-T cell of the present invention, in which the CAR is inserted in the TCR alpha constant chain *(TRAC)* genomic locus expanded continuously. Although the T cells are activated during the gene editing procedure and express CD30, they did not show fratricide effects previously reported for CD30-CAR-T cells produced with retroviral vectors. Current approaches face limitations, such as the need for high initial CAR-T cell dosing and the potential for relapse due to a diminishing or non-persistent CAR-T cell population. Thus, the continuously expanding CD30-CAR-T cells with undetectable fratricide effects enable a persistent and potent immune response against infections in which CD30 is chronically upregulated. Additionally, by continuous expansion, the CD30-CAR-T cell population could provide long-term control of chronic diseases, thereby reducing the risk of relapse.

Moreover, by preventing fratricide of CD30-positive cytotoxic CD30-CAR-T cells, the safety of these cytotoxic cells in treating chronic infections is greatly improved. Fratricide can contribute to severe side effects like cytokine release syndrome (CRS) and neurotoxicity. Eliminating fratricide could significantly reduce these potentially life-threatening complications.

In embodiments, the invention relates to a genetically modified cytotoxic cell comprising a chimeric antigen receptor (CAR) that binds CD30, for use in the treatment of an infection in which CD30 is upregulated. In embodiments, CD30 is upregulated to levels above a non-infected cell. In other embodiments, CD30 is upregulated over a cell infected with a latent virus.

In embodiments, the inventive treatment comprises the cytotoxic cell binding to a human target cell, which is infected with a chronic inflammatory virus and expresses CD30, and activation of said cytotoxic cell, thereby inducing cytotoxic activity against said human target cell and/or a bystander inflammatory cell.

In embodiments, the inventive treatment comprises the cytotoxic cell binding to a human target cell, which is infected with an acute inflammatory virus and expresses CD30, and activation of said cytotoxic cell, thereby inducing cytotoxic activity against said human target cell and/or a bystander inflammatory cell.

In embodiments, the treatment comprises cytotoxic activity of the cytotoxic cell against a reservoir of infected human CD30-expressing target cells in a subject with a chronic viral infection, said reservoir comprising a latent and lytic viral reservoir, preferably wherein said treatment prevents viral pathology or reactivation.

In embodiments, the treatment comprises cytotoxic activity of the cytotoxic cell against a reservoir of infected human CD30-expressing target cells in a subject with an acute viral infection, said reservoir comprising a latent and lytic viral reservoir, preferably wherein said treatment prevents viral pathology or reactivation.

In embodiments, the cytotoxic cell targets a reservoir of human CD30-expressing cells with a chronic viral infection. In embodiments, the cytotoxic cell eliminates reservoirs of human CD30-expressing cells with a chronic viral infection. In embodiments, the cytotoxic cell targets a reservoir of human CD30-expressing cells with an acute viral infection. In embodiments, the cytotoxic cell eliminates reservoirs of human CD30-expressing cells with an acute viral infection. In further embodiments, the cytotoxic cell eradicates a human target cell and/or a bystander inflammatory cell.

While currently available drugs can reduce viral load, to the best of the inventors' knowledge, no treatments on the market offer long-term immune control of viral reservoirs. Hence, a notable advantage of this invention lies in the cytotoxic cell of the present invention, which, in embodiments, can eliminate reservoirs of cells infected with pathogens, preferably chronic inflammatory viruses, wherein said cells express CD30. It was surprising that the cytotoxic cell of the present invention could target these reservoirs, thus enabling either (almost) complete eradication of the virus, thereby preventing reactivation and subsequent flare-ups of illness. Additionally, by targeting these reservoirs, the transmission of the virus to others, e.g., viruses like HIV transmitted through bodily fluids, may be significantly impeded.

In embodiments, the reservoir comprises CD30-expressing B cells, T cells and/or myeloid cells.

In embodiments, the treatment comprises the cytotoxic cell targeting a human CD30-expressing cell with a chronic viral infection or present in the inflammatory microenvironment. In embodiments, the cytotoxic cell targets a human CD30-expressing cell with a chronic viral infection or present in the inflammatory microenvironment.

Advantageously, the cytotoxic cell of the present invention not only targets human CD30-expressing cells with a chronic infection, but also cells present in the inflammatory microenvironment. Chronic inflammatory conditions often involve a dysregulated inflammatory microenvironment. The CD30-CAR cell of the present invention is able to target specific activated immune cell types within this environment, thus offering a novel approach for treating chronic infections and their associated autoimmune diseases.

In embodiments, the treatment comprises cytotoxic activity of the cytotoxic cell against a pre-cancerous and/or non-cancerous human CD30-expressing target cell.

In embodiments, the treatment comprises cytotoxic activity of the cytotoxic cell in a host with an immune-compromised immune system that predisposes the occurrence of cancer or auto-immunity.

In embodiments, the chronic viral infection is a herpes virus infection, preferably EBV or HCMV infection.

In embodiments, the cytotoxic cell targets a human CD30-expressing cell with a chronic viral infection, wherein the chronic viral infection is a herpes virus. In a preferred embodiment, the cytotoxic cell targets a human CD30-expressing cell with a chronic viral infection, wherein the chronic viral infection is an EBV infection. In another preferred embodiment, the cytotoxic cell targets a human CD30-expressing cell with a chronic viral infection, wherein the chronic viral infection is a HCMV infection.

In embodiments, the chronic viral infection is a retrovirus infection, preferably HIV or HTLV infection.

In embodiments, the chronic viral infection is a HIV infection and/or an EBV infection. In embodiments, the cytotoxic cell targets a human CD30-expressing cell with a latent or lytic viral infection, preferably an HIV and/or EBV infection.

In embodiments, the chronic infection is a bacterial infection, preferably a tuberculosis infection.

In embodiments, the treatment is effective against an autoimmune disease that results from or is associated with a chronic infection, the autoimmune disease preferably being rheumatoid arthritis, systemic lupus erythematosus (SLE), multiple sclerosis (scleroderma), Sjörgen's syndrome, Hashimoto's thyroiditis, Wegner's granulomatosis, primary biliary cirrhosis, anti-phospholipid syndrome, uveitis and/or vasculitis; an allergic condition, preferably atopic dermatitis, asthma, and/or allergic rhinitis; and/or a disease associated with inflammation.

In embodiments, the cytotoxic cell is used in the treatment of patients with EBV, HCMV, HIV or HTLV reactivations, infectious mononucleosis, Chronic Active EBV (CAEBV), PTLD, EBV-related, HIV-related, and/or HTLV-related malignancies resulting from chronic virus infections.

As such, the cytotoxic cell comprising a CD30-CAR of this invention represents a remarkable and beneficial approach to the treatment of various diseases described herein, susceptible to the targeted activity of a modified immune cell. The minimal (if any) adverse side effects due to the selectivity and specificity of the expression regulation also represent a beneficial and surprising aspect of the present invention. In embodiments, the cytotoxic cell comprises a CAR that binds CD30 and lacks a T cell receptor (TCR), preferably lacks an endogenous T cell receptor (TCR).

In embodiments, the genetically modified cytotoxic cell comprises a chimeric antigen receptor (CAR), comprising:

i. an extracellular antigen-binding domain, comprising a binding agent, preferably an antibody or antibody fragment, that binds CD30,

ii. a transmembrane domain, and

iii. an intracellular signaling domain, and

iv. an insertion of the CAR into one of the genomic loci needed for expression of the TCR resulting into a TCR knock-out.

In one embodiment, the invention relates to a chimeric antigen receptor polypeptide (CAR), comprising:

- an extracellular antigen-binding domain comprising an antibody or antibody fragment that binds CD30 protein, wherein said antibody or antibody fragment comprises VH and VL domains of a single chain antibody fragment, wherein preferably a linker polypeptide is positioned between the VH and VL domains, wherein said linker is preferably configured to not interfere with the antibody fragment-CD30 antigen interaction;

- a spacer polypeptide (also referred to as a hinge) positioned between the extracellular antigen-binding domain and a transmembrane domain, wherein said spacer polypeptide is preferably configured to not interfere with the antibody fragment-CD30 antigen interaction and/or with cytotoxic cell activation when said CAR is expressed in a cytotoxic cell expressing said CAR;

- a transmembrane domain, wherein said transmembrane domain is preferably configured to not interfere with the antibody fragment- CD30 antigen interaction and/or with cytotoxic cell activation when said CAR is expressed in a cytotoxic cell expressing said CAR; and/or

- and an intracellular domain, wherein said intracellular (signaling) domain comprises a co-stimulatory domain and an activation domain, wherein said intracellular domain is preferably configured to provide signals to stimulate cytotoxic cell activation upon binding to the CD30 target, for example, by increasing cytokine production and/or facilitating cytotoxic cell replication, thus leading to a cytotoxic effect.

In one embodiment, the CAR comprises a hinge domain that allows the immune detection of CAR-positive cells via flow cytometry, mass spectroscopy, or immunofluorescence methods.

In one embodiment, the CAR comprises a hinge domain that allows the enrichment of the CAR-positive TCR-negative-edited cells via flow cytometry or immune-magnetic beads selection.

Therefore, the CAR of the genetically modified cytotoxic cell according to the invention may employ various formats comprising potentially different protein sequences for each functional domain described herein. A skilled person is capable of selecting and testing the desired function of the CARs, for example, based on the experimental approaches demonstrated in the examples below. As such, the election of any given specific protein sequence to be used in the CAR of the invention in any of the functional domains discussed herein can be assessed by a skilled person using routine methods for functional efficacy. For example, various linker polypeptide sequences positioned between the VH and VL domains, various spacer polypeptide sequences (also referred to as a hinge) positioned between the extracellular antigen-binding domain and a transmembrane domain, various transmembrane domains, and various intracellular domains, preferably comprising co-stimulatory and signaling domains, may be employed.

In embodiments of the invention, the CAR and each of the elements or domains mentioned herein are configured to not detrimentally interfere with the antibody fragment-CD30 antigen interaction, to not detrimentally interfere with cytotoxic cell activation when said CAR is expressed in a cytotoxic cell expressing said CAR, and to not detrimentally interfere with the CAR providing signals to stimulate cytotoxic cell activation upon binding to the CD30 target.

Experimental approaches are described herein for assessing these properties of a CD30-CAR, such that the invention is considered to encompass various functional sequence variants and combinations of domains of the types described herein without being limited to the particular sequences disclosed by way of example in the following.

To the knowledge of the inventors, the present invention relates to the first described cytotoxic cell comprising a CD30-CAR with functional evidence of a desired therapeutic effect of a CD30-CAR in the treatment of chronic infections in which infected cells express CD30.

*Embodiments relating to the antigen-binding domain of the CAR*

*CD30-(**HRS-3** V$_H$-V$_L$)-CAR*

In one embodiment, the invention relates to a genetically modified cytotoxic cell comprising a chimeric antigen receptor (CAR) polypeptide as described herein, wherein the antigen-binding domain comprises a variable heavy chain (VH), said VH comprising:

- a heavy chain complementary determining region 1 (H-CDR1) according to SEQ ID NO 1 (TYTIH)

- a heavy chain complementary determining region 2 (H-CDR2) according to SEQ ID NO 2 (YINPSSGCSDYNQNFKG), and

- a heavy chain complementary determining region 1 (H-CDR3) according to SEQ ID NO 3 (RADYGNYEYTWFAY),

and a variable light chain (VL), said VL comprising:

- a light chain complementary determining region 1 (L-CDR1) according to SEQ ID NO 4 (KASQNVGTNVA),

- a light chain complementary determining region 2 (L-CDR2) according to SEQ ID NO 5 (SASYRYS), and

- a light chain complementary determining region 3 (L-CDR3) according to SEQ ID NO 6 (QQYHTYPLT).

In one embodiment, the invention relates to a genetically modified cytotoxic cell comprising a chimeric antigen receptor (CAR) polypeptide as described herein, wherein the antigen-binding domain comprises

- a variable heavy chain (VH) according to SEQ ID NO 7 (RMAQVQLQQSGAELARPGASVKMSCKASGYTFTTY TIHWVRRRPGHDLEWIGYINPSSG CSDYNQNFKGKTTLTADKSSNTAYMQLNSLTSEDSAVYYCARRADYGNYE YTWFAYWGQ GTTVTVSSS) or a VH with at least 80% sequence identity to SEQ ID NO 7, and

- a variable light chain (VL) according to SEQ ID NO 8 (VIELTQSPKFMSTSVGDRVNVTYKASQNVGTN VAWFQQKPGQSPKVLIYSASYRYSGVPD RFTGSGSGTDFTLTISNVQSEDLAEYFCQQYHTYPLTFGGGTKLEIK RSDP) or a VL with at least 80% sequence identity to SEQ ID NO 8.

In one embodiment, the invention relates to a genetically modified cytotoxic cell comprising a chimeric antigen receptor (CAR) polypeptide as described herein, comprising a VH domain that comprises CDR sequences of SEQ ID NO. 1, SEQ ID NO. 2, and SEQ ID NO. 3, and a VL domain that comprises CDR sequences of SEQ ID NO. 4, SEQ ID NO 5, and SEQ ID NO. 6.
In preferred embodiments, the sequence variants with 80% or more sequence identity to the specific CDR sequences of SEQ ID 1-6 maintain CD30 binding with essentially the same or similar functional properties as VH and VL domains with the specific CDR sequences of SEQ ID NO 1-6, i.e. the CD30 binding is essentially the same or similar with respect to affinity, specificity and/or epitope binding mode.
Furthermore, the order of the light and heavy chain fragments may be inverted upon the desired configuration of the antigen-binding fragment.

## CD30-(5F11 V_L-V_H)-CAR

In one embodiment, the invention relates to a genetically modified cytotoxic cell comprising a chimeric antigen receptor (CAR) polypeptide as described herein, wherein the antigen-binding domain comprises a variable heavy chain (VH), said VH comprising:

- a heavy chain complementary determining region 1 (H-CDR1) according to SEQ ID NO 9 (AYYWS)

- a heavy chain complementary determining region 2 (H-CDR2) according to SEQ ID NO 10 (DINHGGGTNYNPSLKS), and

- a heavy chain complementary determining region 1 (H-CDR3) according to SEQ ID NO 11 (LTAY),

and a variable light chain (VL), said VL comprising:

- a light chain complementary determining region 1 (L-CDR1) according to SEQ ID NO 12 (RASQGISSWLT),

- a light chain complementary determining region 2 (L-CDR2) according to SEQ ID NO 13 (AASSLQS), and

- a light chain complementary determining region 3 (L-CDR3) according to SEQ ID NO 14 (QQYDSYPIT).

In one embodiment, the invention relates to a genetically modified cytotoxic cell comprising a chimeric antigen receptor (CAR) polypeptide as described herein, wherein the antigen-binding domain comprises

- a variable heavy chain (VH) according to SEQ ID NO 15 (QVQLQQWGAGLLKPSETLSLTCAVYGGSFSAYYWS WIRQPPGKGLEWIGDINHGGGTNY NPSLKSRVTISVDTSKNQFSLKLNSVTAADTAVYYCASLTAYWGQGSLVTV SS) or a VH with at least 80% sequence identity to SEQ ID NO 15, and

- a variable light chain (VL) according to SEQ ID NO 16 (DIQMTQSPTSLSASVGDRVTITCRASQGISSWLTWYQQK PEKAPKSLIYAASSLQSGVPSR FSGSGSGTDFTLTISSLQPEDFATYYCQQYDSYPITFGQGTRLEIK) or a VL with at least 80% sequence identity to SEQ ID NO 16.

In one embodiment, the invention relates to a genetically modified cytotoxic cell comprising a chimeric antigen receptor (CAR) polypeptide as described herein, comprising a VH domain that comprises CDR sequences of SEQ ID NO.

9, SEQ ID NO. 10, and SEQ ID NO. 11, and a VL domain that comprises CDR sequences of SEQ ID NO. 12, SEQ ID NO 13, and SEQ ID NO. 14.

In preferred embodiments, the sequence variants with 80% or more sequence identity to the specific CDR sequences of SEQ ID 9-14 maintain CD30 binding with essentially the same or similar functional properties as VH and VL domains with the specific CDR sequences of SEQ ID NO 9-14, i.e. the CD30 binding is essentially the same or similar with respect to affinity, specificity and/or epitope binding mode.

Furthermore, the order of the light and heavy chain fragments may be inverted upon the desired configuration of the antigen-binding fragment.

*CD30-(**2H9** $V_H$ $V_L$)-CAR*

In one embodiment, the invention relates to a genetically modified cytotoxic cell comprising a chimeric antigen receptor (CAR) polypeptide as described herein, wherein the antigen-binding domain comprises a variable heavy chain (VH), said VH comprising:

- a heavy chain complementary determining region 1 (H-CDR1) according to SEQ ID NO 17 (GYYWS)

- a heavy chain complementary determining region 2 (H-CDR2) according to SEQ ID NO 18 (EINHSGSTKYTPSLKS), and

- a heavy chain complementary determining region 1 (H-CDR3) according to SEQ ID NO 19 (ETVYYFDL),

and a variable light chain (VL), said VL comprising:

- a light chain complementary determining region 1 (L-CDR1) according to SEQ ID NO 20 (RASQSVSSNLA),

- a light chain complementary determining region 2 (L-CDR2) according to SEQ ID NO 21 (DASNRAT), and

- a light chain complementary determining region 3 (L-CDR3) according to SEQ ID NO 22 (QQRSNWPWT).

In one embodiment, the invention relates to a genetically modified cytotoxic cell comprising a chimeric antigen receptor (CAR) polypeptide as described herein, wherein the antigen-binding domain comprises

- a variable heavy chain (VH) according to SEQ ID NO 23 (QVQLQQWGAGLLKPSETLSLTCAVYGGSFSGYYWS WIRQPPGKGLEWIGEINHSGSTKY TPSLKSRVTISVDTSKHQFSLKLSSVTAADTAVYYCARETVYYFDLWGRGT LVTVSS) or a VH with at least 80% sequence identity to SEQ ID NO 23, and

- a variable light chain (VL) according to SEQ ID NO 24 (EIVLTQSPATLSLSPGERATLSCRASQSVSSNLAWYQQKPG QAPRLLIYDASNRATGIPARL SGSGSGTDFTLTISSLEPEDFAVYYCQQRSNWPWTFGQGTKVEIK) or a VL with at least 80% sequence identity to SEQ ID NO 24.

In one embodiment, the invention relates to a genetically modified cytotoxic cell comprising a chimeric antigen receptor (CAR) polypeptide as described herein, comprising a VH domain that comprises CDR sequences of SEQ ID NO. 17, SEQ ID NO. 18, and SEQ ID NO. 19, and a VL domain that comprises CDR sequences of SEQ ID NO. 20, SEQ ID NO 21, and SEQ ID NO. 22.

In preferred embodiments, the sequence variants with 80% or more sequence identity to the specific CDR sequences of SEQ ID 17-22 maintain CD30 binding with essentially the same or similar functional properties as VH and VL domains with the specific CDR sequences of SEQ ID NO 17-22, i.e. the CD30 binding is essentially the same or similar with respect to affinity, specificity, and/or epitope binding mode.

Furthermore, the order of the light and heavy chain fragments may be inverted upon the desired configuration of the antigen-binding fragment.

Additionally, in embodiments, the linker sequence between heavy and light chains has been modified, for example by extending or shortening, in order to maintain and/or enhance the CAR function.

Additionally, in embodiments, the nucleic acid sequence encoding the CAR has been codon-optimized in order to maintain and/or improve expression of the CAR.

These modifications enable sufficient surface expression on the cytotoxic cell and maintain proper antigen binding. High affinity and high avidity enable the cytotoxic cell comprising the CD30 CAR to i) recognize, ii) be activated against, and iii) kill tumor target cells with high, intermediate, or low CD30 surface expression.

The anti-CD30-CAR cell product described herein is, thus, characterized by unique properties.

The anti-CD30-CAR, as described herein, has a high affinity and confers high specificity and avidity to CD30-expressing infected cells and/or bystander inflammatory cells. These properties enable CD30-CAR cells to i) recognize, ii) be activated against, and iii) kill tumor target cells with high and intermediate CD30 surface expression.

In further aspects, the invention relates to the CAR polypeptide itself (independent of the cell expressing said CAR) and/or a nucleic acid molecule encoding said CAR.

_Embodiments relating to further components of the CAR_

In further embodiments, the invention relates to a chimeric antigen receptor (CAR) polypeptide that comprises one or more linkers, spacers, transmembrane, and/or signalling domains.

In one embodiment, the CAR comprises an intracellular domain comprising a co-stimulatory domain and a signaling (activation) domain.

In one embodiment, the invention relates to a chimeric antigen receptor (CAR) polypeptide expressed after integration in a cell genome, as described herein. In embodiments, the nucleic acid molecule encoding said CAR comprises Homology Directed Repair Templates (HDRTs) with homology arms flanking a target sequence for the TRAC locus of the target (cytotoxic cell), for example:

- Left homology arm SEQ ID NO 25

    TATTAAATAAAAGAATAAGCAGTATTATTAAGTAGCCCTGCATTTCAGGTTTCCTTGAGT

    GGCAGGCCAGGCCTGGCCGTGAACGTTCACTGAAATCATGGCCTCTTGGCCAAGATTG
    ATAGCTTGTGCCTGTCCCTGAGTCCCAGTCCATCACGAGCAGCTGGTTTCTAAGATGCT
    ATTTCCCGTATAAAGCATGAGACCGTGACTTGCCAGCCCCACAGAGCCCCGCCCTTGT
    CCATCACTGGCATCTGGACTCCAGCCTGGGTTGGGGCAAAGAGGGAAATGAGATCATG
    TCCTAACCCTGATCCTCTTGTCCCACAGATATCCAGAACCCTGACCCTGCCGTGTACCA
    GCTGAGAGACTCTAAATCCAGTGACAAGTCTGTCTGCCTATTCACC), or

- Right homology arm SEQ ID NO 26

    (ATTTTGATTCTCAAACAAATGTGTCACAAAGTAAGGATTCTGATGTGTATATCACAGACA
    AAACTGTGCTAGACATGAGGTCTATGGACTTCAAGAGCAACAGTGCTGTGGCCTGGAG
    CAACAAATCTGACTTTGCATGTGCAAACGCCTTCAACAACAGCATTATTCCAGAAGACAC
    CTTCTTCCCCAGCCCAGGTAAGGGCAGCTTTGGTGCCTTCGCAGGCTGTTTCCTTGCTT
    CAGGAATGGCCAGGTTCTGCCCAGAGCTCTGGTCAATGATGTCTAAAACTCCTCTGATT
    GGTGGTCTCGGCCTTATCCATTGCCACCAAAACCCTCTTTTTACTAAGAAACAGTGAGC
    CTTGTTCTGGCAGTCCAGAGAATGACACGGGAAAAAAGCAGATG), or

- homology arms with at least 80% sequence identity to SEQ ID 25 or 26.

In one embodiment, the invention relates to a chimeric antigen receptor (CAR) polypeptide as described herein, wherein the extracellular antigen-binding domain comprises a linker polypeptide positioned between the VH and VL domains, wherein said linker is preferably selected from:

- a Whitlow (SEQ ID NO 27 GSTSGSGKPGSGEGSTKG), or

- Gly-Ser (SEQ ID NO 28; SGGGGSGGGGSGGGGS) linker, or

- linkers with at least 80% sequence identity to SEQ ID NO 27 or 28.

In preferred embodiments, the linker is a Gly-Ser linker.

In one embodiment, the invention relates to a chimeric antigen receptor (CAR) polypeptide as described herein, comprising additionally a linker positioned between the extracellular antigen-binding domain and the hinge domain, wherein said linker comprises a sequence according to SEQ ID NO 29 (GDPA) or a linker with at least 80% sequence identity to SEQ ID NO 29.

In one embodiment, the invention relates to a chimeric antigen receptor (CAR) polypeptide as described herein, comprising additionally a linker positioned the hinge domain and the transmembrane domain, wherein said linker comprises a sequence according to SEQ ID NO 30 (KDPK) or a linker with at least 80% sequence identity to SEQ ID NO 30.

In one embodiment, the invention relates to a chimeric antigen receptor (CAR) polypeptide as described herein, comprising additionally a spacer polypeptide positioned between the extracellular antigen-binding domain and the transmembrane domain, wherein said spacer is selected from:

IgG1 spacer (SEQ ID NO 31;

PAEPKSPDKTHTCPPCPAPPVAGPSVFLFPPKPKDTLMIARTPEVTCVVVDVSHEDPEVKF
NWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGKKDPK), and/or

IgG1Δ spacer (SEQ ID NO 32;

PAEPKSPDKTHTCPPCPAPPVAGPSVFLFPPKPKDTLMIARTPEVTCVVVDVSHEDPEVKF
NWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSSLSPGKK), and/or

- IgG1 (CH3) spacer (SEQ ID NO 33;

GQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK), and/or

IgG4 (Hi-CH2-CH3) spacer (SEQ ID NO 34;

ESKYGPPCPPCPAPEFEGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK), and/or

- IgG4 (Hi-CH3) spacer (SEQ ID NO 35;

ESKYGPPCPPCPGQPREPQVYTLPPSQEEMTKNQVSLTCLVKGFYPSDIAVEWESNGQPE
NNYKTTPPVLDSDGSFFLYSRLTVDKSRWQEGNVFSCSVMHEALHNHYTQKSLSLSLGK),

and/or

- IgG4 (Hi) spacer (SEQ ID NO 36; ESKYGPPCPPCP), and/or

- a truncated tCD34 spacer (SEQ ID NO 37;

ELPTQGTFSNVSTNVSYQETTTPSTLGSTSLHPVSQHGNEATTNITETTVKFTSTSVITSVYG
NTNSSVQSQTSVISTVFTTPANVSTPETTLKPSLSP), and/or

- a spacer with at least 80% sequence identity to any one of SEQ ID NO 31, 32, 33, 34, 35, 36 to 37.

In preferred embodiments, the spacer is an IgG4, and/or IgG1 (CH3), and/or tCD34 spacer. In more preferred embodiments, the spacer is an IgG4 and IgG (CH3) spacer. In other preferred embodiments, the spacer is an IgG4 and tCD34 spacer.

In one embodiment, the invention relates to a chimeric antigen receptor (CAR) polypeptide as described herein, wherein the transmembrane domain is selected from:

- a CD28 domain (SEQ ID NO 38; FWVLVVVGGVLACYSLLVTVAFIIFWVRS), or

- a CD8$\alpha$ domain (SEQ ID NO 39; IYIWAPLAGTCGVLLLSLVITLYC), or

- transmembrane domains with at least 80% sequence identity to SEQ ID NO 38 or 39.

In preferred embodiments, the transmembrane domain is a CD28 domain.

In one embodiment, the invention relates to a chimeric antigen receptor (CAR) polypeptide as described herein, wherein the intracellular domain comprises:

- a CD28 co-stimulatory domain (SEQ ID NO 40; KRSRLLHSDYMNMTPRRPGPTRKHYQPYAPPRDFAAYRS), and/or

- a co-stimulatory domain selected from a 4-1BB co-stimulatory domain (SEQ ID NO 41; KRGRKKLLYIFKQPFMRPVQTTQEEDGCSCRFPEEEEGGCEL), and/or

- a co-stimulatory domain comprising both a 4-1BB (SEQ ID NO 41) and a CD28 co-stimulatory domain (SEQ ID NO 40) arranged adjacently, or

- a co-stimulatory domain with at least 80% sequence identity to SEQ ID NO 40 or 41.

In preferred embodiments, the intracellular domain comprises a CD28 co-stimulatory domain. In contrast to CARs based on the 4-1BB co-stimulatory domain, those employing CD28 demonstrate a more rapid effector profile. This heightened efficiency is associated with substantial alterations in lymphocyte-specific protein tyrosine kinase (Lck) phosphorylation within the signaling pathway. Consequently, CD28-triggered signals elicit increased levels of key immune effectors, including interferon-$\gamma$ (IFN-$\gamma$), granzyme B, and tumor necrosis factor $\alpha$ (TNF-$\alpha$). This differential signaling pathway activation suggests that CD30-based CARs may contribute to enhanced cytotoxicity and immune response.

In one embodiment, the invention relates to a chimeric antigen receptor (CAR) polypeptide as described herein, comprising additionally a signaling domain (otherwise known as an activation domain), wherein said signaling domain is:

- a CD3zeta (4-1BB or CD28) signaling domain (SEQ ID NO 42;

    RVKFSRSADAPAYQQGQNQLYNELNLGRREEYDVLDKRRGRDPEMGGKPRRKNPQEGLY
    NELQKDKMAEAYSEIGMKGERRRGKGHDGLYQGLSTATKDTYDALHMQALPPR), or

- a signaling domain with at least 80% sequence identity to SEQ ID NO 42.

In preferred embodiments, the invention relates to a chimeric antigen receptor (CAR) polypeptide as described herein:

- wherein the extracellular antigen-binding domain comprises a linker polypeptide positioned between the VH and VL domains; and/or

- comprising additionally a spacer polypeptide positioned between the extracellular antigen-binding domain and the transmembrane domain, wherein said spacer is preferably an IgG4 (Hi) spacer (SEQ ID NO 36; ESKYGPPCPPCP) and an IgG1 (CH3) spacer (SEQ ID NO 33; GQPREPQVYTLPPSRDELTKNQVSLTCLVKGFYPSDIAVEWESNG QPENNYKTTPPVLDSD GSFFLYSKLTVDKSRWQQGNVFSCSVMHEALHNHYTQKSLSLSPGK), or a spacer with at least 80% sequence identity to SEQ ID NO 33 or 36; and/or

- wherein the transmembrane domain is a CD28 domain (SEQ ID NO 38; FWVLVVVGGVLACYSLLVTVAFIIFWVRS), or transmembrane domain with at least 80% sequence identity to SEQ ID NO 38; and/or

- wherein the intracellular domain comprises a CD28 co-stimulatory domain (SEQ ID NO 40; KRSRLLHSDYMNMTPRRPGPTRKHYQPYAPPRDFAAYRS), or a co-stimulatory domain with at least 80% sequence identity to SEQ ID NO 40; and/or

- comprising additionally a signaling domain (activation domain), wherein said signaling domain is preferably a CD3zeta signaling domain (SEQ ID NO 42; RVKFSRSADAPAYQQGQNQLYNELNLGRREEYDVLDKRRGRDPE MGGKPRRKNPQEGLY NELQKDKMAEAYSEIGMKGERRRGKGHDGLYQGLSTATKDTYDALHMQALPPR) or a signaling domain with at least 80% sequence identity to SEQ ID NO 42; and/or

- comprising additionally a signal peptide.

In preferred embodiments, the invention relates to a chimeric antigen receptor (CAR) polypeptide as described herein:

- wherein the extracellular antigen-binding domain comprises a linker polypeptide positioned between the VH and VL domains; and/or

- comprising additionally a spacer polypeptide positioned between the extracellular antigen-binding domain and the transmembrane domain, wherein said spacer is preferably an IgG4 (Hi) spacer (SEQ ID NO 36; ESKYGPPCPPCP) and a tCD34 spacer (SEQ ID NO 37; ELPTQGTFSNVSTNVSYQETTTPSTLGSTSLHPVSQHGNEATTNITETTVK FTSTSVITSVYG NTNSSVQSQTSVISTVFTTPANVSTPETTLKPSLSP), or a spacer with at least 80% sequence identity to SEQ ID NO 36 or 37; and/or

- wherein the transmembrane domain is a CD28 domain (SEQ ID NO 38; FWVLWVGGVLACYSLLVTVAFIIFWVRS), or transmembrane domain with at least 80% sequence identity to SEQ ID NO 38; and/or

- wherein the intracellular domain comprises a CD28 co-stimulatory domain (SEQ ID NO 40; KRSRLLHSDYMNMTPRRPGPTRKHYQPYAPPRDFAAYRS), or a co-stimulatory domain with at least 80% sequence identity to SEQ ID NO 40; and/or

- comprising additionally a signaling domain (activation domain), wherein said signaling domain is preferably a CD3zeta signaling domain (SEQ ID NO 42; RVKFSRSADAPAYQQGQNQLYNELNLGRREEYDVLDKRRGRDPE MGGKPRRKNPQEGLY NELQKDKMAEAYSEIGMKGERRRGKGHDGLYQGLSTATKDTYDALHMQALPPR) or a signaling domain with at least 80% sequence identity to SEQ ID NO 42; and/or

- comprising additionally a signal peptide.

In one embodiment, the invention relates to a chimeric antigen receptor (CAR) polypeptide as described herein, or a nucleic acid encoding said CAR construct, comprising a P2A element for protein translation of downstream cistron according to SEQ ID NO 43
(GSGATNFSLLKQAGDVEENPGP) or a P2A element with at least 80% sequence identity to SEQ ID 43.
In one embodiment, the invention relates to a chimeric antigen receptor (CAR) polypeptide as described herein, or a nucleic acid encoding said CAR construct, comprising a sequence with the presence of a poly-adenylation signal (SEQ ID NO 44;

CTAGAGCTCGCTGATCAGCCTCGACTGTGCCTTCTAGTTGCCAGCCATCTGTTGTTTGCCCCTC CCCCGTGCCTTCCTTGACCCTGGAAGGTGCCACTCCCACTGTCCTTTCCTAATAAAATGAGGAA

ATTGCATCGCATTGTCTGAGTAGGTGTCATTCTATTCTGGGGGGTGGGGTGGGGCAGGACAGC AAGGGGGAGGATTGGGAAGAGAATAGCAGGCATGCTGGGGA).

In preferred embodiments, the chimeric antigen receptor (CAR) polypeptide described herein comprises or consists of an amino acid sequence according to MEFGLSWLFLVAILKGVQCDIQMTQSPTSLSASVGDRVTITCRAS QGISSWLTWYQQKPEKAPKSLI YAASSLQSGVPSRFSGSGSGTDFTLTISSLQPEDFATYYCQQYDSYPITFGQGTRL

EIKSGGGGSG GGGSGGGGSQVQLQQWGAGLLKPSETLSLTCAVYGGSFSAYYWSWIRQPPGKGLEWIGDINHGG GTNYNPSLKSRVTISVDTSKNQFSLKLNSVTAADTAVYYCASLTAYWGQGSLVTVSSGDPAESKYG PPCPPCPGQPR EPQVYTLPPSRDELTKNQVSLTCLVKGFYPSDIAVEWESNGQPENNYKTTPPVLD SDGSFFLYSKLTVDKSRWQQGN VFSCSVMHEALHNHYTQKSLSLSPGKKDPKFVWLVWGGVLAC YSLLVTVAFIIFVWRSKRSRLLHSDYMNMTPRRPG PTRKHYQPYAPPRDFAAYRSRVKFSRSADAP AYQQGQNQLYN ELN LG RREEYDVLDKRRG RDPEMGG KPRRKN PQEG LYN ELQKDKMAEAYSE IG MKGERRRGKGHDGLYQGLSTATKDTYDALHMQALPPR (SEQ ID NO 45, CD30-(5F11)-IgG) or a sequence with at least 80% sequence identity to SEQ ID NO 45.

In preferred embodiments, the chimeric antigen receptor (CAR) polypeptide described herein comprises or consists of a nucleotide sequence according to TATTAAATAAAAGAATAAGCAGTATTATTAAGTAGCCCTGCATTTCA GGTTTCCTTGAGTGGCAG GCCAGGCCTGGCCGTGAACGTTCACTGAAATCATGGCCTCTTGGCCAAGATTGATA GCTTGTG CCTGTCCCTGAGTCCCAGTCCATCACGAGCAGCTGGTTTCTAAGATGCTATTTCCCGTATAAAG CATG AGACCGTGACTTGCCAGCCCCACAGAGCCCCGCCCTTGTCCATCACTGGCATCTGGACT CCAGCCTGGGTTGG GGCAAAGAGGGAAATGAGATCATGTCCTAACCCTGATCCTCTTGTCCCA CAGATATCCAGAACCCTGACCCTGCC GTGTACCAGCTGAGAGACTCTAAATCCAGTGACAAGT CTGTCTGCCTATTCACCGGCAGCGGCGCCACCAACTT CAGCCTGCTGAAGCAGGCCGGCGAC GTGGAAGAGAACCCCGGGCCCATGGAGTTTGGCCTGAGCTGGCTGTT TCTGGTGGCCATTCTG AAGGGCGTGCAGTGCGACATCCAGATGACCCAGTCTCCAACCTCACTGTCTGCATCTG TAGGA GACAGAGTCACCATCACTTGTCGGGCGAGTCAGGGTATTAGCAGCTGGTTAACCTGGTATCAG CAGAAA CCAGAGAAAGCCCCTAAGTCCCTGATCTATGCTGCATCCAGTTTGCAAAGTGGGGTC CCATCAAGGTTCAGCGG CAGTGGATCTGGGACAGATTTCACTCTCACCATCAGCAGCCTGCAG CCTGAAGATTTTGCAACTTATTACTGCCA ACAGTATGATAGTTACCCTATCACCTTCGGCCAAGG GACACGACTGGAGATTAAAAGCGGAGGCGGAGGAAGTG GTGGCGGAGGTTCTGGCGGCGGA GGATCTCAGGTGCAGCTACAGCAGTGGGGCGCAGGACTGTTGAAGCCTT CGGAGACCCTGTC CCTCACCTGCGCTGTCTATGGTGGGTCCTTCAGTGCTTACTACTGGAGCTGGATCCGCCAG CC CCCAGGGAAGGGGCTGGAGTGGATTGGGGACATCAATCATGGTGGAGGCACCAACTACAACC CGTCCCTC AAGAGTCGAGTCACCATATCAGTAGACACGTCCAAGAACCAGTTCTCCCTGAAGCT GAACTCTGTAACCGCCGC GGACACGGCTGTGTATTACTGTGCGAGCCTAACTGCCTACTGGGG CCAGGGAAGCCTGGTCACCGTCTCCTCA GGGGATCCCGCCGAGTCTAAATATGGCCCACCTTG CCCACCGTGCCCAGGGCAGCCCCGAGAACCACAGGTGT ACACCCTGCCCCCATCCCGGGATG AGCTGACCAAGAACCAGGTCAGCCTGACCTGCCTGGTCAAAGGCTTCTA TCCCAGCGACATCG CCGTGGAGTGGGAGAGCAATGGGCAACCGGAGAACAACTACAAGACCACGCCTCCCGT GCTG GACTCCGACGGCTCCTTCTTCCTCTACAGCAAGCTCACCGTGGACAAGAGCAGGTGGCAGCAG GGGAA CGTCTTCTCATGCTCCGTGATGCATGAGGCTCTGCACAACCACTACACGCAGAAGAGC CTCTCCCTGTCTCCGG GTAAAAAAGATCCCAAATTTTGGGTGCTGGTGGTGGTTGGTGGAGTC CTGGCTTGCTATAGCTTGCTAGTAACAG TGGCCTTTATTATTTTCTGGGTGAGGAGTAAGAGGA GCAGGCTCCTGCACAGTGACTACATGAACATGACTCCCC GCCGCCCCGGGCCCACCCGCAAG CATTACCAGCCCTATGCCCCACCACGCGACTTCGCAGCCTATCGCTCCAG AGTGAAGTTCAGC AGGAGCGCAGACGCCCCCGCGTACCAGCAGGGCCAGAACCAGCTCTATAACGAGCTCAAT CT AGGACGAAGAGAGGAGTACGATGTTTTGGACAAGAGACGTGGCCGGGACCCTGAGATGGGGG GAAAGCCG AGAAGGAAGAACCCTCAGGAAGGCCTGTACAATGAACTGCAGAAAGATAAGATGG CGGAGGCCTACAGTGAGAT TGGGATGAAAGGCGAGCGCCGGAGGGGCAAGGGGCACGATGG CCTTTACCAGGGTCTCAGTACAGCCACCAA GGACACCTACGACGCCCTTCACATGCAGGCCCT GCCCCCTCGCTGACTAGAGCTCGCTGATCAGCCTCGACTG TGCCTTCTAGTTGCCAGCCATCT GTTGTTTGCCCCTCCCCCGTGCCTTCCTTGACCCTGGAAGGTGCCACTCCC ACTGTCCTTTCCT AATAAAATGAGGAAATTGCATCGCATTGTCTGAGTAGGTGTCATTCTATTCTGGGGGGTGGGG T GGGGCAGGACAGCAAGGGGGAGGATTGGGAAGAGAATAGCAGGCATGCTGGGGAATTTTGAT TCTCAAACAA ATGTGTCACAAAGTAAGGATTCTGATGTGTATATCACAGACAAAACTGTGCTAGA CATGAGGTCTATGGACTTCAA GAGCAACAGTGCTGTGGCCTGGAGCAACAAATCTGACTTTGCA TGTGCAAACGCCTTCAACAACAGCATTATTCC AGAAGACACCTTCTTCCCCAGCCCAGGTAAGG GCAGCTTTGGTGCCTTCGCAGGCTGTTTCCTTGCTTCAGGAA TGGCCAGGTTCTGCCCAGAGC TCTGGTCAATGATGTCTAAAACTCCTCTGATTGGTGGTCTCGGCCTTATCCATT GCCACCAAAA CCCTCTTTTTACTAAGAAACAGTGAGCCTTGTTCTGGCAGTCCAGAGAATGACACGGGAAAAAA GCAGATG (SEQ ID NO 46, CD30-(5F11)-IgG) or a sequence with at least 80% sequence identity to SEQ ID NO 46.

In preferred embodiments, the chimeric antigen receptor (CAR) polypeptide described herein comprises or consists of an amino acid sequence according to MEFGLSWLFLVAILKGVQCDIQMTQSPTSLSASVGDRVTITCRAS QGISSWLTWYQQKPEKAPKSLI YAASSLQSGVPSRFSGSGSGTDFTLTISSLQPEDFATYYCQQYDSYPITFGQGTRL EIKSGGGGSG GGGSGGGGSQVQLQQWGAGLLKPSETLSLTCAVYGGSFSAYYWSWIRQPPGKGLEWIGDINHGG GTNYNPSLKSRVTISVDTSKNQFSLKLNSVTAADTAVYYCASLTAYWGQGSLVTVSSGDPAESKYG PPCPPCPELPTQ GTFSNVSTNVSYQETTTPSTLGSTSLHPVSQHGNEATTNITETTVKFTSTSVITSV YGNTNSSVQSQTSVISTVFTTPA NVSTPETTLKPSLSPKDPKFVWLVWGGVLACYSLLVTVAFIIFW VRSKRSRLLHSDYMNMTPRRPGPTRKHYQPYAP PRDFAAYRSRVKFSRSADAPAYQQGQNQLYN ELN LG RREEYDVLDKRRG RDPE MGG KPRRKN PQEG LYN ELQKDKMAEAYSE IG M KGE RRRGKG H DGLYQGLSTATKDTYDALHMQALPPR (SEQ ID NO 47, CD30-(5F11)-tCD34) or a sequence with at least 80% sequence identity to SEQ ID NO 47.

In preferred embodiments, the chimeric antigen receptor (CAR) polypeptide described herein comprises or consists of a nucleotide sequence according to TATTAAATAAAAGAATAAGCAGTATTATTAAGTAGCCCTGCATTTCA GGTTTCCTTGAGTGGCAG GCCAGGCCTGGCCGTGAACGTTCACTGAAATCATGGCCTCTTGGCCAAGATTGATA GCTTGTG CCTGTCCCTGAGTCCCAGTCCATCACGAGCAGCTGGTTTCTAAGATGCTATTTCCCGTATAAAG CATG AGACCGTGACTTGCCAGCCCCACAGAGCCCCGCCCTTGTCCATCACTGGCATCTGGACT CCAGCCTGGGTTGG GGCAAAGAGGGAAATGAGATCATGTCCTAACCCTGATCCTCTTGTCCCA CAGATATCCAGAACCCTGACCCTGCC GTGTACCAGCTGAGAGACTCTAAATCCAGTGACAAGT CTGTCTGCCTATTCACCGGCAGCGGCGCCACCAACTT CAGCCTGCTGAAGCAGGCCGGCGAC GTGGAAGAGAACCCCGGGCCCATGGAGTTTGGCCTGAGCTGGCTGTT TCTGGTGGCCATTCTG AAGGGCGTGCAGTGCGACATCCAGATGACCCAGTCTCCAACCTCACTGTCTGCATCTG TAGGA GACAGAGTCACCATCACTTGTCGGGCGAGTCAGGGTATTAGCAGCTGGTTAACCTGGTATCAG CAGAAA CCAGAGAAAGCCCCTAAGTCCCTGATCTATGCTGCATCCAGTTTGCAAAGTGGGGTC CCATCAAGGTTCAGCGG CAGTGGATCTGGGACAGATTTCACTCTCACCATCAGCAGCCTGCAG CCTGAAGATTTTGCAACTTATTACTGCCA ACAGTATGATAGTTACCCTATCACCTTCGGCCAAGG GACACGACTGGAGATTAAAAGCGGAGGCGGAGGAAGTG GTGGCGGAGGTTCTGGCGGCGGA GGATCTCAGGTGCAGCTACAGCAGTGGGGCGCAGGACTGTTGAAGCCTT CGGAGACCCTGTC CCTCACCTGCGCTGTCTATGGTGGGTCCTTCAGTGCTTACTACTGGAGCTGGATCCGCCAG CC CCCAGGGAAGGGGCTGGAGTGGATTGGGGACATCAATCATGGTGGAGGCACCAACTACAACC CGTCCCTC AAGAGTCGAGTCACCATATCAGTAGACACGTCCAAGAACCAGTTCTCCCTGAAGCT GAACTCTGTAACCGCCGC GGACACGGCTGTGTATTACTGTGCGAGCCTAACTGCCTACTGGGG CCAGGGAAGCCTGGTCACCGTCTCCTCA GGGGATCCCGCCGAGTCTAAATATGGCCCACCTTG CCCACCGTGCCCAGAGTTACCTACCCAGGGAACATTTTC AAATGTTTCTACAATGTATCCTAC CAAGAAACTACAACACCTAGTACCCTTGGAAGTACCAGCCTGCACCCTGTG TCTCAACATGGCA ATGAGGCCACAACAAACATCACAGAAACGACAGTCAAATTCACATCTACCTCTGTGATAACCT C AGTTTATGGAAACACAAACTCTTCTGTCCAGTCACAGACCTCTGTAATCAGCACAGTGTTCACCA CCCCAGCCA ACGTTTCAACTCCAGAGACAACCTTGAAGCCTAGCCTGTCACCTAAAGATCCCAA ATTTTGGGTGCTGGTGGTG GTTGGTGGAGTCCTGGCTTGCTATAGCTTGCTAGTAACAGTGGC CTTTATTATTTTCTGGGTGAGGAGTAAGAGG AGCAGGCTCCTGCACAGTGACTACATGAACATG ACTCCCCGCCGCCCCGGGCCCACCCGCAAGCATTACCAGC CCTATGCCCCACCACGCGACTT CGCAGCCTATCGCTCCAGAGTGAAGTTCAGCAGGAGCGCAGACGCCCCCGC GTACCAGCAGG GCCAGAACCAGCTCTATAACGAGCTCAATCTAGGACGAAGAGAGGAGTACGATGTTTTGGACA AGAGACGTGGCCGGGACCCTGAGATGGGGGGGAAAGCCGAGAAGGAAGAACCCTCAGGAAGG CCTGTACAATG AACTGCAGAAAGATAAGATGGCGGAGGCCTACAGTGAGATTGGGATGAAAGG CGAGCGCCGGAGGGGCAAGG GGCACGATGGCCTTTACCAGGGTCTCAGTACAGCCACCAAGG ACACCTACGACGCCCTTCACATGCAGGCCCT GCCCCCTCGCTGACTAGAGCTCGCTGATCAGC CTCGACTGTGCCTTCTAGTTGCCAGCCATCTGTTGTTTGCCC CTCCCCCGTGCCTTCCTTGACC CTGGAAGGTGCCACTCCCACTGTCCTTTCCTAATAAAATGAGGAAATTGCATC GCATTGTCTGA GTAGGTGTCATTCTATTCTGGGGGGTGGGGTGGGGCAGGACAGCAAGGGGGAGGATTGGGAA GAGAATAGCAGGCATGCTGGGGAATTTTGATTCTCAAACAAATGTGTCACAAGTAAGGATTCT GATGTGTATATC ACAGACAAAACTGTGCTAGACATGAGGTCTATGGACTTCAAGAGCAACAGTG CTGTGGCCTGGAGCAACAAATC TGACTTTGCATGTGCAAACGCCTTCAACAACAGCATTATTCC AGAAGACACCTTCTTCCCCAGCCCAGGTAAGGG CAGCTTTGGTGCCTTCGCAGGCTGTTTCCT TGCTTCAGGAATGGCCCAGGTTCTGCCCAGAGCTCTGGTCAATGA TGTCTAAAACTCCTCTGATT GGTGGTCTCGGCCTTATCCATTGCCACCAAAACCCTCTTTTTACTAAGAAACAGTG AGCCTTGT TCTGGCAGTCCAGAGAATGACACGGGAAAAAAGCAGATG (SEQ ID NO 48, CD30-(5F11)-tCD34) or a sequence with at least 80% sequence identity to SEQ ID NO 48.

In embodiments, the chimeric antigen receptor (CAR) polypeptide described herein comprises or consists of an amino acid sequence according to MEFGLSWLFLVAILKGVQCRMAVQVLQQSGAELARPGASVKMSCKASGYTFTT YTIHWVRRRPGH DLEWIGYINPSSGCSDYNQNFKGKTTLTADKSSNTAYMQLNSLTSEDSAVYYCARRADYGNYEYT WFAYWGQGTTVTVSSSSGGGGSGGGGSGGGGSVIELTQSPKFMSTSVGDRVNVTYKASQNVGT NVAWFQQKPG QSPKVLIYSASYRYSGVPDRFTGSGSGTDFTLTISNVQSEDLAEYFCQQYHTYPLT FGGGTKLEIKRSDPGDPAESKY GPPCPPCPGQPREPQVYTLPPSRDELTKNQVSLTCLVKGFYPSD IAVEWESNGQPENNYKTTPPVLDSDGSFFLYSK LTVDKSRWQQGNVFSCSVMHEALHNHYTQKSL SLSPGKKDPKFWVLWVGGVLACYSLLVTVAFIIFWVRSKRSRLLH SDYMNMTPRRPGPTRKHYQP YAPPRDFAAYRSRVKFSRSADAPAYQQGQNQLYNELNLGRREEYDVLDKRRGRDP EMGGKPRRK NPQEGLYNELQKDKMAEAYSEIGMKGERRRGKGHDGLYQGLSTATKDTYDALHMQALPPR (SEQ ID NO 49, CD30-(HRS-3)-IgG) or a sequence with at least 80% sequence identity to SEQ ID NO 49.

In embodiments, the chimeric antigen receptor (CAR) polypeptide described herein comprises or consists of a nucleotide sequence according to TATTAAATAAAAGAATAAGCAGTATTATTAAGTAGCCCTGCATTTCAGGTTTCCTT GAGTGGCAG GCCAGGCCTGGCCGTGAACGTTCACTGAAATCATGGCCTCTTGGCCAAGATTGATAGCTTGTG C CTGTCCCTGAGTCCCAGTCCATCACGAGCAGCTGGTTTCTAAGATGCTATTTCCCGTATAAAG CATGAGACCGTG ACTTGCCAGCCCCACAGAGCCCCGCCCTTGTCCATCACTGGCATCTGGACT CCAGCCTGGGTTGGGGCAAAGA GGGAAATGAGATCATGTCCTAACCCTGATCCTCTTGTCCCA CAGATATCCAGAACCCTGACCCTGCCGTGTACCA GCTGAGAGACTCTAAATCCAGTGACAAGT CTGTCTGCCTATTCACCGGCAGCGGCGCCACCAACTTCAGCCTGC TGAAGCAGGCCGGCGAC GTGGAAGAGAACCCCGGGCCCATGGAGTTTGGCCTGAGCTGGCTGTTTCTGGTGG CCATTCTG AAGGGCGTGCAGTGCCGTATGGCCCAGGTTCAGCTGCAGCAATCAGGGGCCGAACTCGCCCG AC

CTGGCGCCTCCGTTAAGATGAGTTGCAAAGCCTCAGGATATACGTTCACGACCTATACTATT CACTGGGGTTCGCC GACGGCCCGGACACGACCTGGAATGGATTGGCTACATTAACCCCTCTTCA GGGTGCTCAGACTATAACCAAAATT TCAAAGGTAAAACCACCCTCACGGCAGATAAGAGTAGCA ATACAGCTTATATGCAATTGAATTCCCTGACTAGCGA AGATTCTGCAGTCTACTACTGCGCCAG GCGTGCAGATTACGGAAACTACGAATACACATGGTTCGCTTACTGGGG CCAAGGGACTACTGT GACCGTCTCTTCCAGTAGCGGAGGCGGAGGAAGTGGTGGCGGAGGTTCTGGCGGCGG AGGA TCTGTGATCGAGTTGACTCAGAGCCCCAAATTCATGAGTACCAGCGTTGGTGATCGTGTTAACG TAACCTA CAAAGCGTCTCAAAATGTGGGAACAAACGTTGCATGGTTTCAGCAGAAGCCCGGCC AGTCCCCAAAGGTCCTG ATCTATAGCGCTAGCTACCGTTACTCCGGTGTTCCCGACCGGTTTAC CGGAAGCGGCAGTGGCACAGATTTCACT CTGACTATCTCCAATGTGCAGAGTGAGGATCTCGC GGAATATTTTTGTCAACAGTATCACACGTACCCCCTGACTT TTGGGGGGGGCACAAACTTGAG ATAAAGCGCTCAGACCCAGGGGATCCCGCCGAGTCTAAATATGGCCCACC TTGCCCACCGTGC CCAGGGCAGCCCCGAGAACCACAGGTGTACACCTGCCCCCATCCCGGGATGAGCTGAC CAA GAACCAGGTCAGCCTGACCTGCCTGGTCAAAGGCTTCTATCCCAGCGACATCGCCGTGGAGTG GGAGAG CAATGGGCAACCGGAGAACAACTACAAGACCACGCCTCCCGTGCTGGACTCCGACG GCTCCTTCTTCCTCTACA GCAAGCTCACCGTGGACAAGAGCAGGTGGCAGCAGGGGAACGTCT TCTCATGCTCCGTGATGCATGAGGCTCT GCACAACCACTACACGCAGAAGAGCCTCTCCCTGT CTCCGGGTAAAAAAGATCCCAAATTTTGGGTGCTGGTGG TGGTTGGTGGAGTCCTGGCTTGCT ATAGCTTGCTAGTAACAGTGGCCTTTATTATTTTCTGGGTGAGGAGTAAGA GGAGCAGGCTCCT GCACAGTGACTACATGAACATGACTCCCCGCCGCCCCGGGCCCACCCGCAAGCATTACCA GC CCTATGCCCCACCACGCGACTTCGCAGCCTATCGCTCCAGAGTGAAGTTCAGCAGGAGCGCAG ACGCCCC CGCGTACCAGCAGGGCCAGAACCAGCTCTATAACGAGCTCAATCTAGGACGAAGA GAGGAGTACGATGTTTTGG ACAAGAGACGTGGCCGGGACCCTGAGATGGGGGGGAAAGCCGAG AAGGAAGAACCCTCAGGAAGGCCTGTACA ATGAACTGCAGAAAGATAAGATGGCGGAGGCCTA CAGTGAGATTGGGATGAAAGGCGAGCGCCGGAGGGGCAA GGGGCACGATGGCCTTTACCAG GGTCTCAGTACAGCCACCAAGGACACCTACGACGCCCTTCACATGCAGGCC CTGCCCCCTCGC TGACTAGAGCTCGCTGATCAGCCTCGACTGTGCCTTCTAGTTGCCAGCCATCTGTTGTTTGC CC CTCCCCCGTGCCTTCCTTGACCCTGGAAGGTGCCACTCCCACTGTCCTTTCCTAATAAAATGAG GAAATTGC ATCGCATTGTCTGAGTAGGTGTCATTCTATTCTGGGGGGTGGGGTGGGGCAGGAC AGCAAGGGGGAGGATTGG GAAGAGAATAGCAGGCATGCTGGGGAATTTTGATTCTCAAACAAA TGTGTCACAAAGTAAGGATTCTGATGTGTAT ATCACAGACAAAACTGTGCTAGACATGAGGTCT ATGGACTTCAAGAGCAACAGTGCTGTGGCCTGGAGCAACAA ATCTGACTTTGCATGTGCAAAC GCCTTCAACAACAGCATTATTCCAGAAGACACCTTCTTCCCCAGCCCAGGTAA GGGCAGCTTTG GTGCCTTCGCAGGCTGTTTCCTTGCTTCAGGAATGGCCAGGTTCTGCCCAGAGCTCTGGTCAA TGATGTCTAAAACTCCTCTGATTGGTGGTCTCGGCCTTATCCATTGCCACCAAAACCCTCTTTTT ACTAAGAAACA GTGAGCCTTGTTCTGGCAGTCCAGAGAATGACACGGGAAAAAAGCAGATG (SEQ ID NO 50, CD30-(HRS-3)-IgG) or a sequence with at least 80% sequence identity to SEQ ID NO 50.

*CD30-(2H9 V<sub>L</sub> V<sub>H</sub>)-CAR*

*CD30-(2H9 V$_L$ V$_H$)-CAR*

In preferred embodiments, the chimeric antigen receptor (CAR) polypeptide described herein comprises or consists of an amino acid sequence according to MEFGLSWLFLVAILKGVQCEIVLTQSPATLSLSPGERATLSCRASQ SVSSNLAWYQQKPGQAPRLLI YDASNRATGIPARLSGSGSGTDFTLTISSLEPEDFAVYYCQQRSNWPWTFGQGTKV EIKSGGGGS GGGGSGGGGSQVQLQQWGAGLLKPSETLSLTCAVYGGSFSGYYWSWIRQPPGKGLEWIGEINHS G STKYTPSLKSRVTISVDTSKHQFSLKLSSVTAADTAVYYCARETVYYFDLWGRGTLVTVSSGDPAE SKYGPPCPPCPG QPREPQVYTLPPSRDELTKNQVSLTCLVKGFYPSDIAVEWESNGQPENNYKTT PPVLDSDGSFFLYSKLTVDKSRWQ QGNVFSCSVMHEALHNHYTQKSLSLSPGKKDPKFVWLVWG GVLACYSLLVTVAFIIFWVRSKRSRLLHSDYMNMTP RRPGPTRKHYQPYAPPRDFAAYRSRVKFSR SADAPAYQQGQNQLYNELNLGRREEYDVLDKRRGRDPEMGGKPRR KNPQEGLYNELQKDKMAE AYSEIGMKGERRRGKGHDGLYQGLSTATKDTYDALHMQALPPR (SEQ ID NO 51, *CD30-(2H9V<sub>L</sub> V<sub>H</sub>)* or a sequence with at least 80% sequence identity to SEQ ID NO 51.

In preferred embodiments, the chimeric antigen receptor (CAR) polypeptide described herein comprises or consists of a nucleotide sequence according to TATTAAATAAAAGAATAAGCAGTATTATTAAGTAGCCCTGCATTTCA GGTTTCCTTGAGTGGCAG GCCAGGCCTGGCCGTGAACGTTCACTGAAATCATGGCCTCTTGGCCAAGATTGATA GCTTGTG CCTGTCCCTGAGTCCCAGTCCATCACGAGCAGCTGGTTTCTAAGATGCTATTTCCCGTATAAAG CATG AGACCGTGACTTGCCAGCCCCACAGAGCCCCGCCCTTGTCCATCACTGGCATCTGGACT CCAGCCTGGGTTGG GGCAAAGAGGGAAATGAGATCATGTCCTAACCCTGATCCTCTTGTCCCA CAGATATCCAGAACCCTGACCCTGCC GTGTACCAGCTGAGAGACTCTAAATCCAGTGACAAGT CTGTCTGCCTATTCACCGGCAGCGGCGCCACCAACTT CAGCCTGCTGAAGCAGGCCGGCGAC GTGGAAGAGAACCCCGGGCCCATGGAGTTTGGCCTGAGCTGGCTGTT TCTGGTGGCCATTCTG AAGGGCGTGCAGTGCGAGATTGTGCTGACACAGTCTCCTGCTACATTAAGTCTGTCTC CGGGT GAACGCGCCACCCTGTCCTGTCGTGCATCCCAATCAGTGAGTAGCAACCTGGCATGGTACCAG CAGAA ACCCGGGCAAGCGCCAAGATTGCTCATCTACGATGCTAGCAACCGGGCCACAGGAATA CCAGCCCGACTTAGC GGCTCTGGCAGCGGGACCGACTTCACTCTCACTATCTCATCCCTAGAG CCCGAGGACTTCGCCGTCTATTATTG CCAGCAGAGGTCGAATTGGCCTTGGACCTTTGGCCAG GGAACGAAGGTTGAAATTAAGAGCGGAGGCGGAGGA

AGTGGTGGCGGAGGTTCTGGCGGCG GAGGATCTCAGGTTCAGTTGCAGCAATGGGGTGCCGGGGCTGTTGAAG CCAAGTGAGACCCTCT CGTTAACCTGCGCTGTCTACGGGGGGAGCTTTTCCGGATATTACTGGAGCTGGATACG CCAGC CGCCTGGAAAAGGCCTGGAGTGGATCGGCGAAATCAACCACTCCGGCTCAACAAAATACACCC CCTCC CTAAAGAGCAGGGTGACAATTTCAGTAGACACTTCTAAGCATCAATTCTCCCTTAAACTG TCTAGCGTGACTGCG GCTGACACTGCAGTTTACTATTGTGCCAGAGAAACGGTGTATTATTTCG ATCTGTGGGGACGGGGTACCCTCGTG ACAGTCTCTAGTGGGGATCCCGCCGAGTCTAAATATG GCCCACCTTGCCCACCGTGCCCAGGGCAGCCCCGAG AACCACAGGTGTACACCCTGCCCCCA TCCCGGGATGAGCTGACCAAGAACCAGGTCAGCCTGACCTGCCTGGT CAAAGGCTTCTATCCC AGCGACATCGCCGTGGAGTGGGAGAGCAATGGGCAACCGGAGAACAACTACAAGACC ACGCC TCCCGTGCTGGACTCCGACGGCTCCTTCTTCCTCTACAGCAAGCTCACCGTGGACAAGAGCAG GTGG CAGCAGGGGAACGTCTTCTCATGCTCCGTGATGCATGAGGCTCTGCACAACCACTACAC GCAGAAGAGCCTCTC CCTGTCTCCGGGTAAAAAGATCCCAAATTTTGGGTGCTGGTGGTGGT TGGTGGAGTCCTGGCTTGCTATAGCTT GCTAGTAACAGTGGCCTTTATTATTTTCTGGGTGAGG AGTAAGAGGAGCAGGCTCCTGCACAGTGACTACATGAA CATGACTCCCCGCCGCCCCGGGCC CACCCGCAAGCATTACCAGCCCTATGCCCCACCACGCGACTTCGCAGCC TATCGCTCCAGAGT GAAGTTCAGCAGGAGCGCAGACGCCCCCGCGTACCAGCAGGGCCAGAACCAGCTCTATA ACG AGCTCAATCTAGGACGAAGAGAGGAGTACGATGTTTTGGACAAGAGACGTGGCCGGGACCCTG AGATGGG GGGAAAGCCGAGAAGGAAGAACCCTCAGGAAGGCCTGTACAATGAACTGCAGAAA GATAAGATGGCGGAGGCC TACAGTGAGATTGGGATGAAAGGCGAGCGCCGGAGGGGCAAGG GGCACGATGGCCTTTACCAGGGTCTCAGTA CAGCCACCAAGGACACCTACGACGCCCTTCACA TGCAGGCCCTGCCCCCTCGCTGACTAGAGCTCGCTGATCA GCCTCGACTGTGCCTTCTAGTTG CCAGCCATCTGTTGTTTGCCCCTCCCCCGTGCCTTCCTTGACCCTGGAAGG TGCCACTCCCAC TGTCCTTTCCTAATAAAATGAGGAAATTGCATCGCATTGTCTGAGTAGGTGTCATTCTATTCTGG GGGGTGGGGTGGGGCAGGACAGCAAGGGGGAGGATTGGGAAGAGAATAGCAGGCATGCTGG GGAATTTTGAT TCTCAAACAAATGTGTCACAAAGTAAGGATTCTGATGTGTATATCACAGACAAA ACTGTGCTAGACATGAGGTCTAT GGACTTCAAGAGCAACAGTGCTGTGGCCTGGAGCAACAAA TCTGACTTTGCATGTGCAAACGCCTTCAACAACA GCATTATTCCAGAAGACACCTTCTTCCCCA GCCCAGGTAAGGGCAGCTTTGGTGCCTTCGCAGGCTGTTTCCTT GCTTCAGGAATGGCCAGGT TCTGCCCAGAGCTCTGGTCAATGATGTCTAAAACTCCTCTGATTGGTGGTCTCGG CCTTATCCA TTGCCACCAAAACCCTCTTTTTACTAAGAAACAGTGAGCCTTGTTCTGGCAGTCCAGAGAATGA CA CGGGAAAAAAGCAGATG (SEQ ID NO 52, CD30-(2H9 $V_L$ $V_H$) or a sequence with at least 80% sequence identity to SEQ ID NO 52.

In preferred embodiments, the chimeric antigen receptor (CAR) polypeptide described herein comprises or consists of a guide RNA (gRNA) sequence for the gene editing according to SEQ ID NO 53 (GAGAAUCAAAAUCGGUGAAU).

In embodiments, the various signaling domains may be exchanged in multiple configurations, providing a CAR with flexibility with respect to its design without losing advantageous binding properties.

Due to the variants (by adding alternative components) employed as the linker, spacer, transmembrane, and intracellular domains, it becomes apparent that the various components may be exchanged as required by the skilled person. The CD30 binding properties may be maintained, thereby maintaining the desired biological effects.

Pilot studies were performed testing the CD3O-CAR-T cells using different target cells: a previously described Hodgkin Lymphoma (HL) immortalized cell line infected with EBV (L-591), EBV-transformed lymphoblastoid cell lines (LCLs), immortalized human T cell leukemia chronically infected with HIV (ACH2). As demonstrated in the examples below, the VL-VH sequences of a humanized monoclonal antibody targeting CD30 could be successfully inserted via CRISPR/Cas gene editing into the TRAC locus, resulting in highly viable CD30-CAR-T cells. It was further shown that the CD30-CAR-T cells recognized and killed CD30-positive cells infected with EBV or with HIV *in vitro*. Thus, the CD30-CAR-T cell of the present invention advantageously enables the elimination of the reservoir of cells infected with herpesviruses or retroviruses.

**Table 1.** Preferred amino acid and nucleotide sequences of the present invention:

| SEQ ID No. | Sequence | Description |
|---|---|---|
| 1 | TYTIH | H-CDR1 (CD30-HRS-3) |
| 2 | YINPSSGCSDYNQNFKG | H-CDR2 (CD30-HRS-3) |
| 3 | RADYGNYEYTWFAY | H-CDR3 (CD30-HRS-3) |

(continued)

| SEQ ID No. | Sequence | Description |
|---|---|---|
| 4 | KASQNVGTNVA | L-CDR1 (CD30-HRS-3) |
| 5 | SASYRYS | L-CDR2 (CD30-HRS-3) |
| 6 | QQYHTYPLT | L-CDR3 (CD30-HRS-3) |
| 7 | RMAQVQLQQSGAELARPGASVKMSCKASGYTFTTYTIHWVRRRPGHDLEWI GYINPSSGCSDYNQNFKGKTTLTADKSSNTAYMQLNSLTSEDSAVYYCARR ADYGNYEYTWFAYWGQGTTVTVSSS | VH (CD30-HRS-3) antibody |
| 8 | VIELTQSPKFMSTSVGDRVNVTYKASQNVGTNVAWFQQKPGQSPKVLIYSA SYRYSGVPDRFTGSGSGTDFTLTISNVQSEDLAEYFCQQYHTYPLTFGGGT KLEIKRSDP | VL (CD30-HRS-3) antibody |
| 9 | AYYWS | H-CDR1 (CD30-5F11) |
| 10 | DINHGGGTNYNPSLKS | H-CDR2 (CD30-5F11) |
| 11 | LTAY | H-CDR3 (CD30-5F11) |
| 12 | RASQGISSWLT | L-CDR1 (CD30-5F11) |
| 13 | AASSLQS | L-CDR2 (CD30-5F11) |
| 14 | QQYDSYPIT | L-CDR3 (CD30-5F11) |
| 15 | QVQLQQWGAGLLKPSETLSLTCAVYGGSFSAYYWSWIRQPPGKGLEWIGDI NHGGGTNYNPSLKSRVTISVDTSKNQFSLKLNSVTAADTAVYYCASLTAYW GQGSLVTVSS | VH (CD30-5F11) antibody |
| 16 | DIQMTQSPTSLSASVGDRVTITCRASQGISSWLTWYQQKPEKAPKSLIYAA SSLQSGVPSRFSGSGSGTDFTLTISSLQPEDFATYYCQQYDSYPITFGQGT RLEIK | VL (CD30-5F11) antibody |
| 17 | GYYWS | H-CDR1 (CD30-2H9) |
| 18 | EINHSGSTKYTPSLKS | H-CDR2 (CD30-2H9) |
| 19 | ETVYYFDL | H-CDR3 (CD30-2H9) |
| 20 | RASQSVSSNLA | L-CDR1 (CD30-2H9) |
| 21 | DASNRAT | L-CDR2 (CD30-2H9) |
| 22 | QQRSNWPWT | L-CDR3 (CD30-2H9) |
| 23 | QVQLQQWGAGLLKPSETLSLTCAVYGGSFSGYYWSWIRQPPGKGLEWIGEI NHSGSTKYTPSLKSRVTISVDTSKHQFSLKLSSVTAADTAVYYCARETVYY FDLWGRGTLVTVSS | VH (CD30-2H9) antibody |
| 24 | EIVLTQSPATLSLSPGERATLSCRASQSVSSNLAWYQQKPGQAPRLLIYDA SNRATGIPARLSGSGSGTDFTLTISSLEPEDFAVYYCQQRSNWPWTFGQGT KVEIK | VL (CD30-2H9) antibody |

(continued)

| SEQ ID No. | Sequence | Description |
|---|---|---|
| 25 | TATTAAATAAAAGAATAAGCAGTATTATTAAGTAGCCCTGCATTTCAGGTT TCCTTGAGTGGCAGGCCAGGCCTGGCCGTGAACGTTCACTGAAATCATGGC CTCTTGGCCAAGATTGATAGCTTGTGCCTGTCCCTGAGTCCCAGTCCATCA CGAGCAGCTGGTTTCTAAGATGCTATTTCCCGTATAAAGCATGAGACCGTG ACTTGCCAGCCCCACAGAGCCCCGCCCTTGTCCATCACTGGCATCTGGACT CCAGCCTGGGTTGGGGCAAAGAGGGAAATGAGATCATGTCCTAACCCTGAT CCTCTTGTCCCACAGATATCCAGAACCCTGACCCTGCCGTGTACCAGCTGA GAGACTCTAAATCCAGTGACAAGTCTGTCTGCCTATTCACC | TRAC homology arm left for CRISPR homologous recombination 1 |
| 26 | ATTTTGATTCTCAAACAAATGTGTCACAAAGTAAGGATTCTGATGTGTATA TCACAGACAAAACTGTGCTAGACATGAGGTCTATGGACTTCAAGAGCAACA GTGCTGTGGCCTGGAGCAACAAATCTGACTTTGCATGTGCAAACGCCTTCA ACAACAGCATTATTCCAGAAGACACCTTCTTCCCCAGCCCAGGTAAGGGCA GCTTTGGTGCCTTCGCAGGCTGTTTCCTTGCTTCAGGAATGGCCAGGTTCT GCCCAGAGCTCTGGTCAATGATGTCTAAAACTCCTCTGATTGGTGGTCTCG GCCTTATCCATTGCCACCAAAACCCTCTTTTTACTAAGAAACAGTGAGCCT TGTTCTGGCAGTCCAGAGAATGACACGGGAAAAAAGCAGATG | TRAC homology arm right for CRISPR homologous recombination 2 |
| 27 | GSTSGSGKPGSGEGSTKG | Whitlow |
| 28 | SGGGGSGGGGSGGGGS | Gly-Ser linker |
| 29 | GDPA | Linker 1 |
| 30 | KDPK | Linker 2 |
| 31 | PAEPKSPDKTHTCPPCPAPPVAGPSVFLFPPKPKDTLMIARTPEVTCVVVD VSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTC LVKGFYPSDIAVEWESNGQPENNYKTTPPVLDSDGSFFLYSKLTVDKSRWQ QGNVFSCSVMHEALHNHYTQKSLSLSPGKKDPK | IgG1 spacer |
| 32 | PAEPKSPDKTHTCPPCPAPPVAGPSVFLFPPKPKDTLMIARTPEVTCVVVD VSHEDPEVKFNWYVDGVEVHNAKTKPREEQYNSTYRVVSVLTVLHQDWLNG KEYKCKVSNKALPAPIEKTISKAKGQPREPQVYTLPPSRDELTKNQVSLTC LVKGFYPSDIAVEWESNGQPENNYKTTPPVLDSDGSFFLYSKLTVDKSRWQ QGNVFSCSVMHEALHNHYTQKSLSSLSPGKK | IgG1Δ spacer |
| 33 | GQPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKSLS LSPGK | IgG1 (CH3) spacer |
| 34 | ESKYGPPCPPCPAPEFEGGPSVFLFPPKPKDTLMISRTPEVTCVVVDVSQE DPEVQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGNV FSCSVMHEALHNHYTQKSLSLSLGK | IgG4 (Hi-CH2-CH3) |

(continued)

| SEQ ID No. | Sequence | Description |
|---|---|---|
| 35 | ESKYGPPCPPCPGQPREPQVYTLPPSQEEMTKNQVSLTCLVKGFYPSDIAV EWESNGQPENNYKTTPPVLDSDGSFFLYSRLTVDKSRWQEGNVFSCSVMHE ALHNHYTQKSLSLSLGK | IgG4 (Hi-CH3) spacer |
| 36 | ESKYGPPCPPCP | IgG4 (Hi) spacer |
| 37 | ELPTQGTFSNVSTNVSYQETTTPSTLGSTSLHPVSQHGNEATTNITETTVK FTSTSVITSVYGNTNSSVQSQTSVISTVFTTPANVSTPETTLKPSLSP | tCD34 spacer |
| 38 | FWVLVVVGGVLACYSLLVTVAFIIFWVRS | CD28 TM domain |
| 39 | IYIWAPLAGTCGVLLLSLVITLYC | CD8α domain |
| 40 | KRSRLLHSDYMNMTPRRPGPTRKHYQPYAPPRDFAAYRS | CD28 co-stimulatory domain |
| 41 | KRGRKKLLYIFKQPFMRPVQTTQEEDGCSCRFPEEEEGGCEL | 4-1BB co-stimulatory domain |
| 42 | RVKFSRSADAPAYQQGQNQLYNELNLGRREEYDVLDKRRGRDPEMGGKPRR KNPQEGLYNELQKDKMAEAYSEIGMKGERRRGKGHDGLYQGLSTATKDTYD ALHMQALPPR | CD3zeta signaling do-main |
| 43 | GSGATNFSLLKQAGDVEENPGP | P2A element for protein translation of down-stream cistron |
| 44 | CTAGAGCTCGCTGATCAGCCTCGACTGTGCCTTCTAGTTGCCAGCCATCTG TTGTTTGCCCCTCCCCCGTGCCTTCCTTGACCCTGGAAGGTGCCACTCCCA CTGTCCTTTCCTAATAAAATGAGGAAATTGCATCGCATTGTCTGAGTAGGT GTCATTCTATTCTGGGGGGTGGGGTGGGGCAGGACAGCAAGGGGGAGGATT GGGAAGAGAATAGCAGGCATGCTGGGGA | Sequence with Poly A signal |
| 45 | MEFGLSWLFLVAILKGVQCDIQMTQSPTSLSASVGDRVTITCRASQGISSW LTWYQQKPEKAPKSLIYAASSLQSGVPSRFSGSGSGTDFTLTISSLQPEDF ATYYCQQYDSYPITFGQGTRLEIKSGGGGSGGGGSGGGGSQVQLQQWGAGL LKPSETLSLTCAVYGGSFSAYYWSWIRQPPGKGLEWIGDINHGGGTNYNPS LKSRVTISVDTSKNQFSLKLNSVTAADTAVYYCASLTAYWGQGSLVTVSSG DPAESKYGPPCPPCPGQPREPQVYTLPPSRDELTKNQVSLTCLVKGFYPSD IAVEWESNGQPENNYKTTPPVLDSDGSFFLYSKLTVDKSRWQQGNVFSCSV MHEALHNHYTQKSLSLSPGKKDPKFWVLVVVGGVLACYSLLVTVAFIIFWV RSKRSRLLHSDYMNMTPRRPGPTRKHYQPYAPPRDFAAYRSRVKFSRSADA PAYQQGQNQLYNELNLGRREEYDVLDKRRGRDPEMGGKPRRKNPQEGLYNE LQKDKMAEAYSEIGMKGERRRGKGHDGLYQGLSTATKDTYDALHMQALPPR | Preferred CAR (5F11-CD30-IgG) AA seq |

(continued)

| SEQ ID No. | Sequence | Description |
|---|---|---|
| 46 | TATTAAATAAAAGAATAAGCAGTATTATTAAGTAGCCCTGCATTTCAGGTT<br>TCCTTGAGTGGCAGGCCAGGCCTGGCCGTGAACGTTCACTGAAATCATGGC<br>CTCTTGGCCAAGATTGATAGCTTGTGCCTGTCCCTGAGTCCCAGTCCATCA<br>CGAGCAGCTGGTTTCTAAGATGCTATTTCCCGTATAAAGCATGAGACCGTG<br>ACTTGCCAGCCCCACAGAGCCCCGCCCTTGTCCATCACTGGCATCTGGACT<br>CCAGCCTGGGTTGGGGCAAAGAGGGAAATGAGATCATGTCCTAACCCTGAT<br>CCTCTTGTCCCACAGATATCCAGAACCCTGACCCTGCCGTGTACCAGCTGA<br>GAGACTCTAAATCCAGTGACAAGTCTGTCTGCCTATTCACCGGCAGCGGCG<br>CCACCAACTTCAGCCTGCTGAAGCAGGCCGGCGACGTGGAAGAGAACCCCG<br>GGCCCATGGAGTTTGGCCTGAGCTGGCTGTTTCTGGTGGCCATTCTGAAGG<br>GCGTGCAGTGCGACATCCAGATGACCCAGTCTCCAACCTCACTGTCTGCAT<br>CTGTAGGAGACAGAGTCACCATCACTTGTCGGGCGAGTCAGGGTATTAGCA<br>GCTGGTTAACCTGGTATCAGCAGAAACCAGAGAAAGCCCCTAAGTCCCTGA<br>TCTATGCTGCATCCAGTTTGCAAAGTGGGGTCCCATCAAGGTTCAGCGGCA<br>GTGGATCTGGGACAGATTTCACTCTCACCATCAGCAGCCTGCAGCCTGAAG<br>ATTTTGCAACTTATTACTGCCAACAGTATGATAGTTACCCTATCACCTTCG<br>GCCAAGGGACACGACTGGAGATTAAAAGCGGAGGCGGAGGAAGTGGTGGCG<br>GAGGTTCTGGCGGCGGAGGATCTCAGGTGCAGCTACAGCAGTGGGGCGCAG<br>GACTGTTGAAGCCTTCGGAGACCCTGTCCCTCACCTGCGCTGTCTATGGTG | Preferred CAR (5F11-CD30-IgG) nucleotide seq |

(continued)

| SEQ ID No. | Sequence | Description |
|---|---|---|
| | GGTCCTTCAGTGCTTACTACTGGAGCTGGATCCGCCAGCCCCCAGGGAAGG GGCTGGAGTGGATTGGGGACATCAATCATGGTGGAGGCACCAACTACAACC CGTCCCTCAAGAGTCGAGTCACCATATCAGTAGACACGTCCAAGAACCAGT TCTCCCTGAAGCTGAACTCTGTAACCGCCGCGGACACGGCTGTGTATTACT GTGCGAGCCTAACTGCCTACTGGGGCCAGGGAAGCCTGGTCACCGTCTCCT CAGGGGATCCCGCCGAGTCTAAATATGGCCCACCTTGCCCACCGTGCCCAG GGCAGCCCCGAGAACCACAGGTGTACACCCTGCCCCCATCCCGGGATGAGC TGACCAAGAACCAGGTCAGCCTGACCTGCCTGGTCAAAGGCTTCTATCCCA GCGACATCGCCGTGGAGTGGGAGAGCAATGGGCAACCGGAGAACAACTACA AGACCACGCCTCCCGTGCTGGACTCCGACGGCTCCTTCTTCCTCTACAGCA AGCTCACCGTGGACAAGAGCAGGTGGCAGCAGGGGAACGTCTTCTCATGCT CCGTGATGCATGAGGCTCTGCACAACCACTACACGCAGAAGAGCCTCTCCC TGTCTCCGGGTAAAAAAGATCCCAAATTTTGGGTGCTGGTGGTGGTTGGTG GAGTCCTGGCTTGCTATAGCTTGCTAGTAACAGTGGCCTTTATTATTTTCT GGGTGAGGAGTAAGAGGAGCAGGCTCCTGCACAGTGACTACATGAACATGA CTCCCCGCCGCCCCGGGCCCACCCGCAAGCATTACCAGCCCTATGCCCCAC CACGCGACTTCGCAGCCTATCGCTCCAGAGTGAAGTTCAGCAGGAGCGCAG ACGCCCCCGCGTACCAGCAGGGCCAGAACCAGCTCTATAACGAGCTCAATC TAGGACGAAGAGAGGAGTACGATGTTTTGGACAAGAGACGTGGCCGGGACC CTGAGATGGGGGGGAAAGCCGAGAAGGAAGAACCCTCAGGAAGGCCTGTACA ATGAACTGCAGAAAGATAAGATGGCGGAGGCCTACAGTGAGATTGGGATGA AAGGCGAGCGCCGGAGGGGCAAGGGGCACGATGGCCTTTACCAGGGTCTCA GTACAGCCACCAAGGACACCTACGACGCCCTTCACATGCAGGCCCTGCCCC CTCGCTGACTAGAGCTCGCTGATCAGCCTCGACTGTGCCTTCTAGTTGCCA GCCATCTGTTGTTTGCCCCTCCCCCGTGCCTTCCTTGACCCTGGAAGGTGC CACTCCCACTGTCCTTTCCTAATAAAATGAGGAAATTGCATCGCATTGTCT GAGTAGGTGTCATTCTATTCTGGGGGGTGGGGTGGGGCAGGACAGCAAGGG GGAGGATTGGGAAGAGAATAGCAGGCATGCTGGGGAATTTTGATTCTCAAA CAAATGTGTCACAAAGTAAGGATTCTGATGTGTATATCACAGACAAAACTG TGCTAGACATGAGGTCTATGGACTTCAAGAGCAACAGTGCTGTGGCCTGGA GCAACAAATCTGACTTTGCATGTGCAAACGCCTTCAACAACAGCATTATTC CAGAAGACACCTTCTTCCCCAGCCCAGGTAAGGGCAGCTTTGGTGCCTTCG CAGGCTGTTTCCTTGCTTCAGGAATGGCCAGGTTCTGCCCAGAGCTCTGGT CAATGATGTCTAAAACTCCTCTGATTGGTGGTCTCGGCCTTATCCATTGCC ACCAAAACCCTCTTTTTACTAAGAAACAGTGAGCCTTGTTCTGGCAGTCCA GAGAATGACACGGGAAAAAAGCAGATG | |

(continued)

| SEQ ID No. | Sequence | Description |
|---|---|---|
| 47 | MEFGLSWLFLVAILKGVQCDIQMTQSPTSLSASVGDRVTITCRASQGISSW LTWYQQKPEKAPKSLIYAASSLQSGVPSRFSGSGSGTDFTLTISSLQPEDF ATYYCQQYDSYPITFGQGTRLEIKSGGGGSGGGGSGGGGSQVQLQQWGAGL LKPSETLSLTCAVYGGSFSAYYWSWIRQPPGKGLEWIGDINHGGGTNYNPS LKSRVTISVDTSKNQFSLKLNSVTAADTAVYYCASLTAYWGQGSLVTVSSG DPAESKYGPPCPPCPELPTQGTFSNVSTNVSYQETTTPSTLGSTSLHPVSQ HGNEATTNITETTVKFTSTSVITSVYGNTNSSVQSQTSVISTVFTTPANVS TPETTLKPSLSPKDPKFWVLVVVGGVLACYSLLVTVAFIIFWVRSKRSRLL HSDYMNMTPRRPGPTRKHYQPYAPPRDFAAYRSRVKFSRSADAPAYQQGQN QLYNELNLGRREEYDVLDKRRGRDPEMGGKPRRKNPQEGLYNELQKDKMAE AYSEIGMKGERRGKGHDGLYQGLSTATKDTYDALHMQALPPR | Preferred CAR (5F11-CD30-tCD34) AA seq |

| SEQ ID No. | Sequence | Description |
|---|---|---|
| 48 | TATTAAATAAAAGAATAAGCAGTATTATTAAGTAGCCCTGCATTTCAGGTT<br>TCCTTGAGTGGCAGGCCAGGCCTGGCCGTGAACGTTCACTGAAATCATGGC<br>CTCTTGGCCAAGATTGATAGCTTGTGCCTGTCCCTGAGTCCCAGTCCATCA<br>CGAGCAGCTGGTTTCTAAGATGCTATTTCCCGTATAAAGCATGAGACCGTG<br>ACTTGCCAGCCCCACAGAGCCCCGCCCTTGTCCATCACTGGCATCTGGACT<br>CCAGCCTGGGTTGGGGCAAAGAGGGAAATGAGATCATGTCCTAACCCTGAT<br>CCTCTTGTCCCACAGATATCCAGAACCCTGACCCTGCCGTGTACCAGCTGA<br>GAGACTCTAAATCCAGTGACAAGTCTGTCTGCCTATTCACCGGCAGCGGCG<br>CCACCAACTTCAGCCTGCTGAAGCAGGCCGGCGACGTGGAAGAGAACCCCG<br>GGCCCATGGAGTTTGGCCTGAGCTGGCTGTTTCTGGTGGCCATTCTGAAGG<br>GCGTGCAGTGCGACATCCAGATGACCCAGTCTCCAACCTCACTGTCTGCAT<br>CTGTAGGAGACAGAGTCACCATCACTTGTCGGGCGAGTCAGGGTATTAGCA<br>GCTGGTTAACCTGGTATCAGCAGAAACCAGAGAAAGCCCCTAAGTCCCTGA<br>TCTATGCTGCATCCAGTTTGCAAAGTGGGGTCCCATCAAGGTTCAGCGGCA<br>GTGGATCTGGGACAGATTTCACTCTCACCATCAGCAGCCTGCAGCCTGAAG<br>ATTTTGCAACTTATTACTGCCAACAGTATGATAGTTACCCTATCACCTTCG<br>GCCAAGGGACACGACTGGAGATTAAAAGCGGAGGCGGAGGAAGTGGTGGCG<br>GAGGTTCTGGCGGCGGAGGATCTCAGGTGCAGCTACAGCAGTGGGGCGCAG<br>GACTGTTGAAGCCTTCGGAGACCCTGTCCCTCACCTGCGCTGTCTATGGTG<br>GGTCCTTCAGTGCTTACTACTGGAGCTGGATCCGCCAGCCCCCAGGGAAGG<br>GGCTGGAGTGGATTGGGGACATCAATCATGGTGGAGGCACCAACTACAACC<br>CGTCCCTCAAGAGTCGAGTCACCATATCAGTAGACACGTCCAAGAACCAGT<br>TCTCCCTGAAGCTGAACTCTGTAACCGCCGCGGACACGGCTGTGTATTACT<br>GTGCGAGCCTAACTGCCTACTGGGGCCAGGGAAGCCTGGTCACCGTCTCCT<br>CAGGGGATCCCGCCGAGTCTAAATATGGCCCACCTTGCCCACCGTGCCCAG<br>AGTTACCTACCCAGGGAACATTTTCAAATGTTTCTACAAATGTATCCTACC<br>AAGAAACTACAACACCTAGTACCCTTGGAAGTACCAGCCTGCACCCTGTGT<br>CTCAACATGGCAATGAGGCCACAACAAACATCACAGAAACGACAGTCAAAT<br>TCACATCTACCTCTGTGATAACCTCAGTTTATGGAAACACAAACTCTTCTG<br>TCCAGTCACAGACCTCTGTAATCAGCACAGTGTTCACCACCCCAGCCAACG<br>TTTCAACTCCAGAGACAACCTTGAAGCCTAGCCTGTCACCTAAAGATCCCA<br>AATTTTGGGTGCTGGTGGTGGTTGGTGGAGTCCTGGCTTGCTATAGCTTGC<br>TAGTAACAGTGGCCTTTATTATTTTCTGGGTGAGGAGTAAGAGGAGCAGGC<br>TCCTGCACAGTGACTACATGAACATGACTCCCCGCCGCCCCGGGCCCACCC<br>GCAAGCATTACCAGCCCTATGCCCCACCACGCGACTTCGCAGCCTATCGCT<br>CCAGAGTGAAGTTCAGCAGGAGCGCAGACGCCCCCGCGTACCAGCAGGGCC<br>AGAACCAGCTCTATAACGAGCTCAATCTAGGACGAAGAGAGGAGTACGATG<br>TTTTGGACAAGAGACGTGGCCGGGACCCTGAGATGGGGGGAAAGCCGAGAA<br>GGAAGAACCCTCAGGAAGGCCTGTACAATGAACTGCAGAAAGATAAGATGG<br>CGGAGGCCTACAGTGAGATTGGGATGAAAGGCGAGCGCCGGAGGGGCAAGG<br>GGCACGATGGCCTTTACCAGGGTCTCAGTACAGCCACCAAGGACACCTACG | Preferred CAR (5F11-CD30-tCD34) nucleotide seq |

(continued)

| SEQ ID No. | Sequence | Description |
|---|---|---|
| | ACGCCCTTCACATGCAGGCCCTGCCCCCTCGCTGACTAGAGCTCGCTGATC AGCCTCGACTGTGCCTTCTAGTTGCCAGCCATCTGTTGTTTGCCCCTCCCC CGTGCCTTCCTTGACCCTGGAAGGTGCCACTCCCACTGTCCTTTCCTAATA AAATGAGGAAATTGCATCGCATTGTCTGAGTAGGTGTCATTCTATTCTGGG GGGTGGGGTGGGGCAGGACAGCAAGGGGGAGGATTGGGAAGAGAATAGCAG GCATGCTGGGGAATTTTGATTCTCAAACAAATGTGTCACAAAGTAAGGATT CTGATGTGTATATCACAGACAAAACTGTGCTAGACATGAGGTCTATGGACT TCAAGAGCAACAGTGCTGTGGCCTGGAGCAACAAATCTGACTTTGCATGTG CAAACGCCTTCAACAACAGCATTATTCCAGAAGACACCTTCTTCCCCAGCC CAGGTAAGGGCAGCTTTGGTGCCTTCGCAGGCTGTTTCCTTGCTTCAGGAA TGGCCAGGTTCTGCCCAGAGCTCTGGTCAATGATGTCTAAAACTCCTCTGA TTGGTGGTCTCGGCCTTATCCATTGCCACCAAAACCCTCTTTTTACTAAGA AACAGTGAGCCTTGTTCTGGCAGTCCAGAGAATGACACGGGAAAAAAGCAG ATG | |
| 49 | MEFGLSWLFLVAILKGVQCRMAQVQLQQSGAELARPGASVKMSCKASGYTF TTYTIHWVRRRPGHDLEWIGYINPSSGCSDYNQNFKGKTTLTADKSSNTAY MQLNSLTSEDSAVYYCARRADYGNYEYTWFAYWGQGTTVTVSSSSGGGGSG GGGSGGGGSVIELTQSPKFMSTSVGDRVNVTYKASQNVGTNVAWFQQKPGQ SPKVLIYSASYRYSGVPDRFTGSGSGTDFTLTISNVQSEDLAEYFCQQYHT YPLTFGGGTKLEIKRSDPGDPAESKYGPPCPPCPGQPREPQVYTLPPSRDE LTKNQVSLTCLVKGFYPSDIAVEWESNGQPENNYKTTPPVLDSDGSFFLYS KLTVDKSRWQQGNVFSCSVMHEALHNHYTQKSLSLSPGKKDPKFWVLVVVG GVLACYSLLVTVAFIIFWVRSKRSRLLHSDYMNMTPRRPGPTRKHYQPYAP PRDFAAYRSRVKFSRSADAPAYQQGQNQLYNELNLGRREEYDVLDKRRGRD PEMGGKPRRKNPQEGLYNELQKDKMAEAYSEIGMKGERRRGKGHDGLYQGL STATKDTYDALHMQALPPR | Preferred CAR (HRS3-CD30-IgG) AA seq |

(continued)

| SEQ ID No. | Sequence | Description |
|---|---|---|
| 50 | TATTAAATAAAAGAATAAGCAGTATTATTAAGTAGCCCTGCATTTCAGGTT<br>TCCTTGAGTGGCAGGCCAGGCCTGGCCGTGAACGTTCACTGAAATCATGGC<br>CTCTTGGCCAAGATTGATAGCTTGTGCCTGTCCCTGAGTCCCAGTCCATCA<br>CGAGCAGCTGGTTTCTAAGATGCTATTTCCCGTATAAAGCATGAGACCGTG<br>ACTTGCCAGCCCCACAGAGCCCCGCCCTTGTCCATCACTGGCATCTGGACT<br>CCAGCCTGGGTTGGGGCAAAGAGGGAAATGAGATCATGTCCTAACCCTGAT<br>CCTCTTGTCCCACAGATATCCAGAACCCTGACCCTGCCGTGTACCAGCTGA<br>GAGACTCTAAATCCAGTGACAAGTCTGTCTGCCTATTCACCGGCAGCGGCG<br>CCACCAACTTCAGCCTGCTGAAGCAGGCCGGCGACGTGGAAGAGAACCCCG<br>GGCCCATGGAGTTTGGCCTGAGCTGGCTGTTTCTGGTGGCCATTCTGAAGG<br>GCGTGCAGTGCCGTATGGCCCAGGTTCAGCTGCAGCAATCAGGGGCCGAAC<br>TCGCCCGACCTGGCGCCTCCGTTAAGATGAGTTGCAAAGCCTCAGGATATA<br>CGTTCACGACCTATACTATTCACTGGGTTCGCCGACGGCCCGGACACGACC<br>TGGAATGGATTGGCTACATTAACCCCTCTTCAGGGTGCTCAGACTATAACC<br>AAAATTTCAAAGGTAAAACCACCCTCACGGCAGATAAGAGTAGCAATACAG<br>CTTATATGCAATTGAATTCCCTGACTAGCGAAGATTCTGCAGTCTACTACT<br>GCGCCAGGCGTGCAGATTACGGAAACTACGAATACACATGGTTCGCTTACT<br>GGGGCCAAGGGACTACTGTGACCGTCTCTTCCAGTAGCGGAGGCGGAGGAA | Preferred CAR (HRS3-CD30-IgG) nucleotide seq |

(continued)

| SEQ ID No. | Sequence | Description |
|---|---|---|
|  | GTGGTGGCGGAGGTTCTGGCGGCGGAGGATCTGTGATCGAGTTGACTCAGA GCCCCAAATTCATGAGTACCAGCGTTGGTGATCGTGTTAACGTAACCTACA AAGCGTCTCAAAATGTGGGAACAAACGTTGCATGGTTTCAGCAGAAGCCCG GCCAGTCCCCAAAGGTCCTGATCTATAGCGCTAGCTACCGTTACTCCGGTG TTCCCGACCGGTTTACCGGAAGCGGCAGTGGCACAGATTTCACTCTGACTA TCTCCAATGTGCAGAGTGAGGATCTCGCGGAATATTTTTGTCAACAGTATC ACACGTACCCCCTGACTTTTGGGGGGGGCACAAAACTTGAGATAAAGCGCT CAGACCCAGGGGATCCCGCCGAGTCTAAATATGGCCCACCTTGCCCACCGT GCCCAGGGCAGCCCCGAGAACCACAGGTGTACACCCTGCCCCCATCCCGGG ATGAGCTGACCAAGAACCAGGTCAGCCTGACCTGCCTGGTCAAAGGCTTCT ATCCCAGCGACATCGCCGTGGAGTGGGAGAGCAATGGGCAACCGGAGAACA ACTACAAGACCACGCCTCCCGTGCTGGACTCCGACGGCTCCTTCTTCCTCT ACAGCAAGCTCACCGTGGACAAGAGCAGGTGGCAGCAGGGGAACGTCTTCT CATGCTCCGTGATGCATGAGGCTCTGCACAACCACTACACGCAGAAGAGCC TCTCCCTGTCTCCGGGTAAAAAAGATCCCAAATTTTGGGTGCTGGTGGTGG TTGGTGGAGTCCTGGCTTGCTATAGCTTGCTAGTAACAGTGGCCTTTATTA TTTTCTGGGTGAGGAGTAAGAGGAGCAGGCTCCTGCACAGTGACTACATGA ACATGACTCCCCGCCGCCCCGGGCCCACCCGCAAGCATTACCAGCCCTATG CCCCACCACGCGACTTCGCAGCCTATCGCTCCAGAGTGAAGTTCAGCAGGA GCGCAGACGCCCCCGCGTACCAGCAGGGCCAGAACCAGCTCTATAACGAGC TCAATCTAGGACGAAGAGAGGAGTACGATGTTTTGGACAAGAGACGTGGCC GGGACCCTGAGATGGGGGGGAAAGCCGAGAAGGAAGAACCCTCAGGAAGGCC TGTACAATGAACTGCAGAAAGATAAGATGGCGGAGGCCTACAGTGAGATTG GGATGAAAGGCGAGCGCCGGAGGGGCAAGGGGCACGATGGCCTTTACCAGG GTCTCAGTACAGCCACCAAGGACACCTACGACGCCCTTCACATGCAGGCCC TGCCCCCTCGCTGACTAGAGCTCGCTGATCAGCCTCGACTGTGCCTTCTAG TTGCCAGCCATCTGTTGTTTGCCCCTCCCCCGTGCCTTCCTTGACCCTGGA AGGTGCCACTCCCACTGTCCTTTCCTAATAAAATGAGGAAATTGCATCGCA TTGTCTGAGTAGGTGTCATTCTATTCTGGGGGGTGGGGTGGGGCAGGACAG CAAGGGGGAGGATTGGGAAGAGAATAGCAGGCATGCTGGGGAATTTTGATT CTCAAACAAATGTGTCACAAAGTAAGGATTCTGATGTGTATATCACAGACA AAACTGTGCTAGACATGAGGTCTATGGACTTCAAGAGCAACAGTGCTGTGG CCTGGAGCAACAAATCTGACTTTGCATGTGCAAACGCCTTCAACAACAGCA TTATTCCAGAAGACACCTTCTTCCCCAGCCCAGGTAAGGGCAGCTTTGGTG CCTTCGCAGGCTGTTTCCTTGCTTCAGGAATGGCCAGGTTCTGCCCAGAGC TCTGGTCAATGATGTCTAAAACTCCTCTGATTGGTGGTCTCGGCCTTATCC ATTGCCACCAAAACCCTCTTTTTACTAAGAAACAGTGAGCCTTGTTCTGGC AGTCCAGAGAATGACACGGGAAAAAAGCAGATG |  |

(continued)

| SEQ ID No. | Sequence | Description |
|---|---|---|
| 51 | MEFGLSWLFLVAILKGVQCEIVLTQSPATLSLSPGERATLSCRASQSVSSN LAWYQQKPGQAPRLLIYDASNRATGIPARLSGSGSGTDFTLTISSLEPEDF AVYYCQQRSNWPWTFGQGTKVEIKSGGGGSGGGGSGGGGSQVQLQQWGAGL LKPSETLSLTCAVYGGSFSGYYWSWIRQPPGKGLEWIGEINHSGSTKYTPS LKSRVTISVDTSKHQFSLKLSSVTAADTAVYYCARETVYYFDLWGRGTLVT VSSGDPAESKYGPPCPPCPGQPREPQVYTLPPSRDELTKNQVSLTCLVKGF YPSDIAVEWESNGQPENNYKTTPPVLDSDGSFFLYSKLTVDKSRWQQGNVF SCSVMHEALHNHYTQKSLSLSPGKKDPKFWVLVVVGGVLACYSLLVTVAFI IFWVRSKRSRLLHSDYMNMTPRRPGPTRKHYQPYAPPRDFAAYRSRVKFSR SADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQA LPPR | Preferred CAR (CD30-(2H9 $V_L$ $V_H$)) AA sequence |

(continued)

| SEQ ID No. | Sequence | Description |
|---|---|---|
| 52 | TATTAAATAAAAGAATAAGCAGTATTATTAAGTAGCCCTGCATTTCAGGTT TCCTTGAGTGGCAGGCCAGGCCTGGCCGTGAACGTTCACTGAAATCATGGC CTCTTGGCCAAGATTGATAGCTTGTGCCTGTCCCTGAGTCCCAGTCCATCA CGAGCAGCTGGTTTCTAAGATGCTATTTCCCGTATAAAGCATGAGACCGTG ACTTGCCAGCCCCACAGAGCCCCGCCCTTGTCCATCACTGGCATCTGGACT CCAGCCTGGGTTGGGGCAAAGAGGGAAATGAGATCATGTCCTAACCCTGAT CCTCTTGTCCCACAGATATCCAGAACCCTGACCCTGCCGTGTACCAGCTGA GAGACTCTAAATCCAGTGACAAGTCTGTCTGCCTATTCACCGGCAGCGGCG CCACCAACTTCAGCCTGCTGAAGCAGGCCGGCGACGTGGAAGAGAACCCCG GGCCCATGGAGTTTGGCCTGAGCTGGCTGTTTCTGGTGGCCATTCTGAAGG GCGTGCAGTGCGAGATTGTGCTGACACAGTCTCCTGCTACATTAAGTCTGT CTCCGGGTGAACGCGCCACCCTGTCCTGTCGTGCATCCCAATCAGTGAGTA GCAACCTGGCATGGTACCAGCAGAAACCCGGGCAAGCGCCAAGATTGCTCA TCTACGATGCTAGCAACCGGGCCACAGGAATACCAGCCCGACTTAGCGGCT CTGGCAGCGGGACCGACTTCACTCTCACTATCTCATCCCTAGAGCCCGAGG ACTTCGCCGTCTATTATTGCCAGCAGAGGTCGAATTGGCCTTGGACCTTTG GCCAGGGAACGAAGGTTGAAATTAAGAGCGGAGGCGGAGGAAGTGGTGGCG GAGGTTCTGGCGGCGGAGGATCTCAGGTTCAGTTGCAGCAATGGGGTGCCG GGCTGTTGAAGCCAAGTGAGACCCTCTCGTTAACCTGCGCTGTCTACGGGG GGAGCTTTTCCGGATATTACTGGAGCTGGATACGCCAGCCGCCTGGAAAAG GCCTGGAGTGGATCGGCGAAATCAACCACTCCGGCTCAACAAAATACACCC CCTCCCTAAAGAGCAGGGTGACAATTTCAGTAGACACTTCTAAGCATCAAT TCTCCCTTAAACTGTCTAGCGTGACTGCGGCTGACACTGCAGTTTACTATT GTGCCAGAGAAACGGTGTATTATTTCGATCTGTGGGGACGGGGTACCCTCG TGACAGTCTCTAGTGGGGATCCCGCCGAGTCTAAATATGGCCCACCTTGCC CACCGTGCCCAGGGCAGCCCCGAGAACCACAGGTGTACACCCTGCCCCCAT CCCGGGATGAGCTGACCAAGAACCAGGTCAGCCTGACCTGCCTGGTCAAAG GCTTCTATCCCAGCGACATCGCCGTGGAGTGGGAGAGCAATGGGCAACCGG AGAACAACTACAAGACCACGCCTCCCGTGCTGGACTCCGACGGCTCCTTCT TCCTCTACAGCAAGCTCACCGTGGACAAGAGCAGGTGGCAGCAGGGGAACG TCTTCTCATGCTCCGTGATGCATGAGGCTCTGCACAACCACTACACGCAGA AGAGCCTCTCCCTGTCTCCGGGTAAAAAGATCCCAAATTTTGGGTGCTGG TGGTGGTTGGTGGAGTCCTGGCTTGCTATAGCTTGCTAGTAACAGTGGCCT TTATTATTTTCTGGGTGAGGAGTAAGAGGAGCAGGCTCCTGCACAGTGACT ACATGAACATGACTCCCCGCCGCCCCGGGCCCACCCGCAAGCATTACCAGC CCTATGCCCCACCACGCGACTTCGCAGCCTATCGCTCCAGAGTGAAGTTCA GCAGGAGCGCAGACGCCCCCGCGTACCAGCAGGGCCAGAACCAGCTCTATA ACGAGCTCAATCTAGGACGAAGAGAGGAGTACGATGTTTTGGACAAGAGAC | Preferred CAR CAR (CD30-(2H9 $V_L$ $V_H$)) nucleotide seq |

(continued)

| SEQ ID No. | Sequence | Description |
|---|---|---|
| | GTGGCCGGGACCCTGAGATGGGGGGAAAGCCGAGAAGGAAGAACCCTCAGG AAGGCCTGTACAATGAACTGCAGAAAGATAAGATGGCGGAGGCCTACAGTG AGATTGGGATGAAAGGCGAGCGCCGGAGGGGCAAGGGGCACGATGGCCTTT ACCAGGGTCTCAGTACAGCCACCAAGGACACCTACGACGCCCTTCACATGC AGGCCCTGCCCCCTCGCTGACTAGAGCTCGCTGATCAGCCTCGACTGTGCC TTCTAGTTGCCAGCCATCTGTTGTTTGCCCCTCCCCCGTGCCTTCCTTGAC CCTGGAAGGTGCCACTCCCACTGTCCTTTCCTAATAAAATGAGGAAATTGC ATCGCATTGTCTGAGTAGGTGTCATTCTATTCTGGGGGGTGGGGTGGGGCA GGACAGCAAGGGGGAGGATTGGGAAGAGAATAGCAGGCATGCTGGGGAATT TTGATTCTCAAACAAATGTGTCACAAAGTAAGGATTCTGATGTGTATATCA CAGACAAAACTGTGCTAGACATGAGGTCTATGGACTTCAAGAGCAACAGTG CTGTGGCCTGGAGCAACAAATCTGACTTTGCATGTGCAAACGCCTTCAACA ACAGCATTATTCCAGAAGACACCTTCTTCCCCAGCCCAGGTAAGGGCAGCT TTGGTGCCTTCGCAGGCTGTTTCCTTGCTTCAGGAATGGCCAGGTTCTGCC CAGAGCTCTGGTCAATGATGTCTAAAACTCCTCTGATTGGTGGTCTCGGCC TTATCCATTGCCACCAAAACCCTCTTTTTACTAAGAAACAGTGAGCCTTGT TCTGGCAGTCCAGAGAATGACACGGGAAAAAAGCAGATG | |
| 53 | GAGAAUCAAAAUCGGUGAAU | gRNA |

*Further aspects and embodiments of the invention*

In embodiments, the cytotoxic cell is an immune effector cell, preferably a lymphocyte, more preferably a T cell or natural killer (NK) cell.

In embodiments, the cytotoxic cell is a lymphocyte. In embodiments, the cytotoxic cell is a T cell. In embodiments, the cytotoxic cell is an NK cell.

In one embodiment, the cytotoxic cell is an allogeneic immune effector cell with respect to a patient. In one embodiment, the cytotoxic cell is an allogeneic lymphocyte with respect to a patient. In one embodiment, the cytotoxic cell is an allogeneic T cell with respect to a patient. In one embodiment, the cytotoxic cell is an allogeneic NK cell with respect to a patient.

In one embodiment, the cytotoxic cell is an autologous immune effector cell with respect to a patient. In one embodiment, the cytotoxic cell is an autologous lymphocyte with respect to a patient. In one embodiment, the cytotoxic cell is an autologous T cell with respect to a patient. In one embodiment, the cytotoxic cell is an autologous NK cell with respect to a patient.

In some embodiments, cells can be obtained from a subject different from the intended patient (allogeneic cells). As used herein, a cell is "allogeneic" with respect to a subject if it or one of its progenitor cells is derived from another subject of the same species. As used herein, a cell is "autologous" with respect to a subject if it or its progenitor cells originate from the same subject.

"Off-the-shelf' CD30-CAR cell products, as described in the present invention, lacking expression of the TCR, the human leukocyte antigens or co-expression cytokines, can be produced from cells of healthy donors in stock. These "off-the-shelf' CD30-CAR cell products are indicated in the case of lytic or latent infections and are applicable to a broad range of virus infections and their pathologies.

The means provided by the invention enable reducing the cost of individual doses of CAR-T cell products and failures in the production of CAR-T cells.

The most serious risk associated with the use of allogeneic T-cell products is an often-fatal acute graft-versus-host disease (GvHD), in which the donor's (CAR-modified) T-cells recognize and attack the patient's tissue as "foreign" through their endogenous TCR. Due to the beneficial properties of the cells of the invention that lack the TCR expression, these risks are reduced compared to known approaches.

In embodiments, a nucleic acid construct comprising a sequence encoding a CAR-T that binds CD30 (CD30-CAR) is integrated into the genome of said cytotoxic cell, and said sequence encoding the CD30-CAR is expressed from an endogenous promoter.

Using an endogenous promoter for CAR expression leads to a more physiological pattern of CAR production, mimicking how a T cell normally regulates its own genes. Furthermore, a CD30-CAR controlled by an endogenous promoter may exhibit more consistent and regulated CAR expression. This could potentially mitigate exhaustion in the cytotoxic cell comprising the CD30-CAR.

In embodiments, the CD30-CAR construct is integrated into another genomic locus encoding a component of the TCR.

In embodiments, a nucleic acid construct comprising a sequence encoding a CD30-CAR is integrated into an endogenous T-cell receptor locus, preferably a TCR alpha (TRAC) locus, in the genome of said cytotoxic cell.

In embodiments, the CD30-CAR construct is integrated into a T cell receptor (TCR) alpha (TRAC) locus in the genome of said cytotoxic cell. In embodiments, the CD30-CAR as provided by the invention can be expressed under the control of the endogenous TRAC promoter.

In embodiments, the sequence of the gRNA for the genetic knock-in into the TRAC locus is GAGAAUCAAAAUCGGUGAAU (SEQ ID NO. 53).

One advantage of integrating the CD30-CAR construct into a genomic locus encoding a component of the TCR receptor, preferably the TRAC locus, of the cytotoxic cell, is that endogenous TCRs are disrupted and no endogenous TCRs are presented on the cytotoxic cell comprising the CD30-CAR. This disruption of endogenous TCR expression provides a method for safely implementing immunotherapies with allogeneic CD30-CAR constructs.

In embodiments, the CD30-CAR construct is a CD3 zeta-deficient chimeric antigen receptor (CAR) fragment (transgene) that is then integrated into an endogenous CD3 zeta/CD247 gene for generating a gene fusion and therefore a functional CAR comprising an exogenous CAR fragment fused with an endogenous CD3 zeta domain. The technology employing integrating CD3 zeta deficient CAR constructs into endogenous CD3 zeta genes is described in detail in WO2022/136551.

In embodiments, the CD30 CAR construct comprises three nucleic acid sequence regions, wherein a first nucleic acid sequence region encodes a CD30 chimeric antigen receptor (CAR) fragment without a sequence encoding a functional intracellular domain of a CD3 zeta protein, a second nucleic acid sequence region comprises a targeting sequence configured for integrating the construct at an endogenous CD3 zeta gene of a human cell, and a third nucleic acid sequence region encoding and or comprising a protein separation site upstream of the first sequence region.

In embodiments, the targeting sequence is configured for integrating the construct in-frame with an endogenous CD3 zeta encoding sequence, preferably into an exon of an endogenous CD3 zeta gene. In embodiments, the protein separation site of the third nucleic acid sequence encodes a first self-cleavage peptide, protease cleavage site or an internal ribosomal entry site (IRES).

In embodiments, the target site of the CD3 zeta gene, into which the first sequence region is integrated, is an exon and/or an intron, preferably exon 2, and is positioned upstream of an endogenous CD3 zeta sequence encoding an intracellular domain or fragment thereof, said intracellular domain or fragment thereof comprising one or more immunoreceptor tyrosine-based activation motifs (ITAMs).

In further aspects, the invention relates to a CD30-CAR polypeptide as an in-frame fusion protein comprising the exogenous CAR fragment and an endogenous CD3 zeta intracellular domain. The invention further relates to a genetically modified human cell expressing the CD30 CAR fragment, without a sequence encoding a functional intracellular domain of a CD3 zeta protein, integrated in-frame into the endogenous CD3 zeta/CD247 gene, to produce a fusion CAR comprising an exogenous CAR fragment with an endogenous CD3 zeta chain.

In embodiments, the CD30-CAR as provided by the invention can be expressed under the control of the endogenous CD3 zeta promoter.

In embodiments, the cytotoxic cell is genetically modified by integrating a nucleic acid construct comprising a sequence encoding a CD30-CAR into the genome of said cytotoxic cell by gene editing, preferably by CRISPR/Cas gene editing, more preferably without a viral vector.

In preferred embodiments, the cytotoxic cell is genetically modified by CRISPR/Cas gene editing. In preferred embodiments, the cytotoxic cell is genetically modified without a viral vector.

Allogeneic T-cell products have a high risk of leading to the often-fatal acute graft-versus-host (GvHD) reaction, in which the donor's CAR-modified T-cells triggered by their endogenous TCR recognize and attack the patient's tissue as "foreign".

Using CRIPSR/Cas gene editing for the genetic modification of the cytotoxic cell of the present invention provides unparalleled precision, offering the ability to precisely integrate the nucleic acid construct comprising a sequence encoding the CD30-CAR into the genome of said cytotoxic cell and specifically switch off genes for the TCR of the donor's CAR-modified T cells. Thus, the present invention provides a strategy and method that avoids adverse TCR expression, thus reducing side effects like GvHD.

Another advantage of the present invention is that a cytotoxic cell of the present invention can be genetically edited without using a viral vector. Viral vectors, while effective, carry the risk of insertional mutagenesis. Thus, a non-viral method offers - as suggested by the present invention - more precise control over the insertion of the CAR construct.

The advantageous aspects of the present invention, therefore, allow for a universal off-the-shelf cell therapy, especially for patients at high risk of EBV, HCMV, HIV and HTLV reactivation, infectious mononucleosis, PTLD, EBV-related, HIV-related or HTLV-related malignancies.

Consequently, the cytotoxic cell according to the present invention not only enhances the safety of immunotherapy but also streamlines the process, making it more efficient and cost-effective.

As demonstrated in the examples, the VL-VH sequence of the human monoclonal antibody could be inserted via CRISPR/Cas gene editing into the TRAC, resulting in highly viable CD30-CAR-T cells.

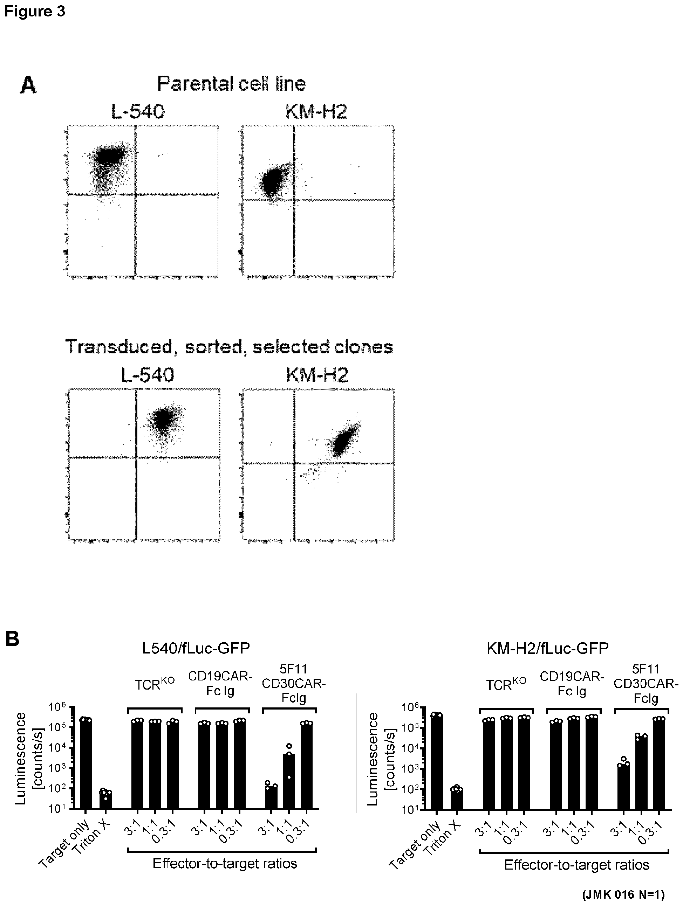

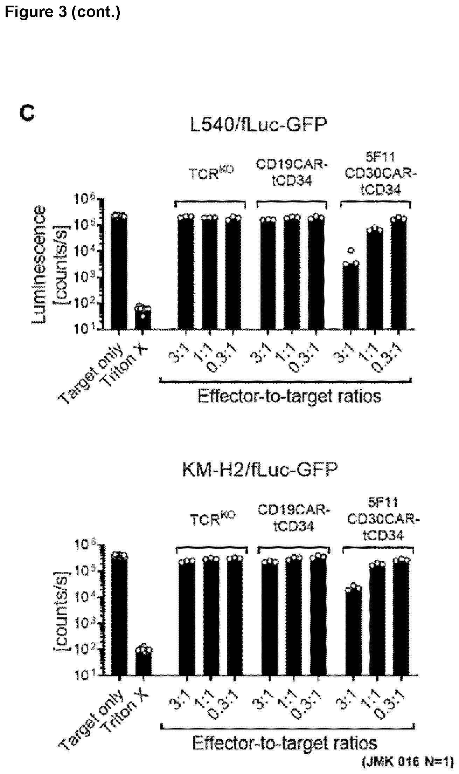

By way of example, figure 3 illustrates the successful knock-in of CD30-CAR. 5F11-CD30-CAR exhibited the most effective knock-ins using non-viral gene-editing techniques compared to controls. Moreover, CD30-CARs continuously expand without fratricide effects. Lastly, CD30-CAR-T cells were shown to kill EBV-infected lymphoblastic cell lines (LCLs), even at low effector-to-target ratios.

In embodiments, the CD30-CAR-expressing cell expands without fratricide effects.

In embodiments, the cytotoxic cell is generated by gene editing and lacks expression of a functional endogenous T cell receptor.

In embodiments, the cytotoxic cell is generated by gene editing and lacks expression of one or more human leukocyte antigens.

In embodiments, the cytotoxic cell is generated by gene editing and lacks expression of CD30.

In embodiments, the cytotoxic cell can be administered in an allogeneic setting.

In embodiments, the cytotoxic cell does not cause graft-versus-host disease.

In embodiments, the cytotoxic cell is administered in a pharmaceutical composition with one or more pharmaceutically acceptable excipients.

The various aspects of the invention are unified by, benefit from, are based on, and/or are linked by the structural and/or functional features, including functional properties and beneficial technical effects, of the genetically modified cytotoxic cell described herein. Any features disclosed in any further aspects of the invention, such as the medical use thereof or the pharmaceutical composition, are also considered disclosed in the context of the genetically modified cytotoxic cell and vice versa.

## DETAILED DESCRIPTION OF THE INVENTION

The present invention relates to a genetically modified cytotoxic cell comprising a chimeric antigen receptor (CAR) that binds CD30, for use in the treatment of an infection, in which infected cells express CD30. In embodiments, in infected target cells, expression and/or presentation of CD30 is upregulated over uninfected cells.

As used herein, "upregulation of CD30" refers to an increase in the expression, production, cell surface presentation or activity of CD30 in a cell or tissue compared to healthy cells or tissues. The "upregulation of CD30 in an infected cell" may also refer to an increase in the expression, production, cell surface presentation or activity of CD30 in a cell compared to uninfected cells. The reference or control value varies depending on the cell, context, and disease. However, a skilled person in the art is able to determine if CD30 is upregulated without undue effort. Means to determine the upregulation of a gene or protein, such as CD30, are known in the art and may include but are not limited to RT-PCR, Northern Blot, Western Blot, immunohistochemistry, mass spectrometry, RNA-sequencing, in-situ hybridization, etc.

### *CD30-CAR construct*

CD30, also known as tumor necrosis factor (TNF) receptor superfamily 8 (TNFRSF8), mediates various cellular responses through complex molecular mechanisms. Upon binding with its CD30L (CD153) ligand, CD30 triggers signal transduction pathways involving TNFR-associated factor (TRAF) proteins. TRAF2, TRAF1, and TRAF5 are known to interact with CD30, initiating downstream signaling cascades that culminate in the activation of transcription factors, including nuclear factor kappa B (NF-κB). The activation of NF-κB plays a pivotal role in regulating gene expression related to cell proliferation, survival, and apoptosis. CD30 stimulation can activate both the canonical and non-canonical NF-κB pathways, leading to the nuclear translocation of NF-κB dimers and subsequent transcriptional activity. Additionally, CD30 signaling pathways intersect with mitogen-activated protein kinase (MAPK)/extracellular signal-regulated kinase (ERK) pathways, further modulating cellular responses. The pleiotropic effects of CD30 stimulation, such as proliferation, survival, cytokine secretion, and cell death, depend on cell type and differentiation state. It is expressed on activated T and B lymphocytes and is notably present in certain hematopoietic malignancies such as anaplastic large cell lymphoma (ALCL) and Hodgkin lymphoma (HL). CD30 upregulation has been associated with anti-apoptotic mechanisms, contributing to lymphomagenesis and promoting cell survival. CD30 is upregulated in some EBV-positive lymphomas and on polyclonal B cells infected with EBV. This mechanism is mediated by the EBV protein LMP1, but its role in promoting EBV tumorigenesis is not fully understood. Further, CD30 expression on CD4$^+$ T cells significantly increases before HIV-1 rebound. CD30 is also expressed in a subset of adult T cell leukemia/lymphoma caused by HTLV-1 infection.

According to the present invention, a "chimeric antigen receptor (CAR)" polypeptide comprises an extracellular antigen-binding domain, comprising an antibody or antibody fragment that binds a target antigen, a transmembrane domain, and an intracellular domain. CARs are typically described as comprising an extracellular ectodomain (antigen-binding domain) derived from an antibody and an endodomain comprising signaling modules derived from T-cell signaling proteins.

In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8$\alpha$ or CD28. In the first generation of CARs, the signaling domain consists of the zeta chain of the TCR complex. The term "generation" refers to the structure of the intracellular signaling domains. Second-generation CARs are equipped with a single costimulatory domain originating from CD28 or 4-1BB. Third-generation CARs already include two costimulatory domains, e.g., CD28, 4-1BB, ICOS or OX40, CD3 zeta. The present invention preferably relates to a second or third-generation CAR, although the antigen-binding fragments described herein may be employed in any given CAR format.

As used herein, the term "chimeric" describes being composed of parts of different proteins or DNAs from different origins.

The methods of the present invention comprise the administration of cells designed to express a CAR. In some embodiments, the ligand or extracellular ligand binding domain is selected so that the cell expressing the CAR is targeted to a CD30-expressing cell.

CARs contemplated herein comprise an extracellular domain (also referred to as a binding domain or antigen-binding domain) that binds to a CD30-expressing cell, preferably with a chronic infection, a transmembrane domain, and an intracellular domain or intracellular signaling domain. Engagement of the antigen-binding domain of the CAR with the target on the surface of a target cell results in clustering of the CAR and delivers an activation stimulus to the CAR-containing cell. The main characteristic of CARs is their ability to redirect immune effector cell specificity, thereby triggering the proliferation of antigen-specific effector T cells, cytokine production (such as IFN-y), and production of molecules that can mediate death of the target cells expressing the surface antigen in a major histocompatibility complex (MHC) independent manner.

In various embodiments, a CAR comprises an extracellular binding domain that comprises a (optionally humanized) CD30-specific binding domain; a transmembrane domain; one or more intracellular signaling domains. In particular embodiments, a CAR comprises an extracellular binding domain that comprises a (optionally humanized) anti-CD30 antigen binding fragment thereof; one or more spacer domains; a transmembrane domain; one or more intracellular signaling domains. In embodiments, the scFvs derived from the 5F11 and 2H9 monoclonal antibodies are humanized.

The "extracellular antigen-binding domain" or "extracellular binding domain" are used interchangeably and provide a CAR with the ability to bind specifically to the target antigen of interest. The binding domain may be derived either from a natural, synthetic, semi-synthetic, or recombinant source. Preferred are scFv domains.

"Specific binding" is to be understood by a person skilled in the art, whereby the skilled person is aware of various experimental procedures that can be used to test binding and binding specificity. Methods for determining equilibrium association or equilibrium dissociation constants are known in the art. Cross-reaction or background binding may be inevitable in many protein-protein interactions; this is not to detract from the "specificity" of the binding between CAR and epitope. "Specific binding" describes the binding of an anti-herpes virus antigen-antibody or antigen-binding fragment thereof (or a CAR comprising the same) to said herpes virus antigen at greater binding affinity than background binding. The term "directed against" is also applicable when considering the term "specificity" in understanding the interaction between antibodies and epitopes.

An "antigen (Ag)" refers to a compound, composition, or substance that can stimulate the production of antibodies or a T cell response in an animal. In particular embodiments, the target antigen is an epitope of a CD30 polypeptide. An "epitope" refers to the region of an antigen to which a binding agent binds. Epitopes can be formed both from contiguous amino acids or noncontiguous amino acids juxtaposed by tertiary folding of a protein. Any suitable antigen can be selected depending on the disease to be treated.

*Antibodies and antibody fragments:*

The CAR comprises an extracellular antigen-binding domain, comprising an antibody or antibody fragment that binds a target polypeptide as described herein. Antibodies or antibody fragments of the invention therefore include, but are not limited to, polyclonal, monoclonal, bispecific, human, humanized, or chimeric antibodies, single chain fragments (scFv), single variable fragments (ssFv), single domain antibodies (such as VHH fragments from nanobodies), Fab fragments, F(ab')2 fragments, fragments produced by a Fab expression library, anti-idiotypic antibodies and epitope-binding fragments or combinations thereof of any of the above, provided that they retain similar binding properties of the CAR described herein, preferably comprising the corresponding CDRs, or VH and VL regions as described herein. Also, mini-antibodies and multivalent antibodies such as diabodies, triabodies, tetravalent antibodies, and peptabodies can be used in a method of the invention. The immunoglobulin molecules of the invention can be of any class (i.e., IgG, IgE, IgM, IgD, and IgA) or subclass of immunoglobulin molecules. Thus, the term antibody, as used herein, also includes antibodies and antibody fragments comprised by the CAR of the invention, either produced by the modification of whole antibodies or synthesized de novo using recombinant DNA methodologies.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 110 or more amino acids, primarily responsible for antigen recognition. The terms "variable light chain" and "variable heavy chain" refer to these variable regions of the light and heavy chains, respectively. Optionally, the antibody, or the immunological portion of the antibody, can be chemically conjugated to, or expressed as, a fusion protein with other proteins.

"Single-chain Fv" or "scFv" antibody fragments comprise the VH and VL domains of an antibody, wherein these domains are present in a single polypeptide chain and either orientation (e.g., VL-VH or VH-VL). Generally, the scFv polypeptide further comprises a polypeptide linker between the VH and VL domains, which enables the scFv to form the desired structure for antigen binding.

In preferred embodiments, a CAR contemplated herein comprises an antigen-specific binding domain that is a scFv and may be a murine, human, or humanized scFv. Single-chain antibodies may be cloned from the V region genes of a hybridoma specific for a desired target. In particular embodiments, the antigen-specific binding domain is a (humanized) scFv that binds CD30.

In a preferred embodiment, the CAR construct comprises a human monoclonal antibody 5F11 or fragment thereof. In further embodiments, the CAR construct comprises a human monoclonal antibody HRS3 or fragments thereof.

The CARs of the invention are intended to bind against mammalian, in particular human, protein targets. The use of protein names may correspond to either mouse or human versions of a protein. Affinities of binding domain polypeptides and CAR proteins according to the present disclosure can be readily determined using conventional techniques, e.g., by competitive ELISA (enzyme-linked immunosorbent assay), or by binding association, or displacement assays using labeled ligands, or using a surface-plasmon resonance device such as the Biacore.

Humanized antibodies comprising one or more CDRs of the invention or one or more CDRs derived from said antibodies can be made using any methods known in the art. For example, four general steps may be used to humanize a monoclonal antibody. These are: (1) determining the nucleotide and predicted amino acid sequence of the starting antibody light and heavy variable domains (2) designing the humanized antibody, i.e., deciding which antibody framework region to use during the humanizing process (3) the actual humanizing methodologies/techniques and (4) the transfection and expression of the humanized antibody. See, for example, U.S. Pat. Nos. 4,816,567; 5,807,715; 5,866,692; 6,331,415; 5,530,101; 5,693,761; 5,693,762; 5,585,089; 6,180,370; 5,225,539; 6,548,640.

The term humanized antibody means that at least a portion of the framework regions, and optionally a portion of CDR regions or other regions involved in binding, of immunoglobulin is derived from or adjusted to human immunoglobulin sequences. The humanized, chimeric, or partially humanized versions of the mouse monoclonal antibodies can, for example, be made using recombinant DNA technology, departing from the mouse and/or human genomic DNA sequences coding for H and L chains or from cDNA clones coding for H and L chains. Humanized forms of mouse antibodies can be generated by linking the CDR regions of non-human antibodies to human constant regions by recombinant DNA techniques (Queen et al., 1989; WO 90/07861). Alternatively, the monoclonal antibodies used in the method of the invention may be human monoclonal antibodies. Human antibodies can be obtained, for example, using phage-display methods (WO 91/17271; WO 92/01047).

As used herein, humanized antibodies also refer to forms of non-human (e.g., murine, camel, llama, shark) antibodies that are specific chimeric immunoglobulins, immunoglobulin chains, or fragments thereof (such as Fv, Fab, Fab', F(ab')2 or other antigen-binding subsequences of antibodies) that contain minimal sequence derived from non-human immunoglobulin.

As used herein, human or humanized antibody or antibody fragment means an antibody having an amino acid sequence corresponding to that of an antibody produced by a human and/or has been made using any of the techniques for making human antibodies known in the art or disclosed herein. Human antibodies or fragments thereof can be selected by competitive binding experiments or otherwise to have the same epitope specificity as a particular mouse antibody. The humanized antibodies of the present invention surprisingly share the useful functional properties of the mouse antibodies to a large extent. Human polyclonal antibodies can also be provided in the form of serum from humans immunized with an immunogenic agent. Optionally, such polyclonal antibodies can be concentrated by affinity purification using amyloid fibrillar and/or non-fibrillar polypeptides or fragments thereof as an affinity reagent. Monoclonal antibodies can be obtained from serum according to the technique described in WO 99/60846.

*Variable Regions and CDRs*

A variable region of an antibody refers to the variable region of the antibody light chain or the variable region of the antibody heavy chain, either alone or in combination. The variable regions of the heavy and light chains each consist of four framework regions (FR) connected by three complementarity determining regions (CDRs), also known as hypervariable regions. The CDRs in each chain are held together in close proximity by the FRs and, with the CDRs from the other chain, contribute to the formation of the antigen-binding site of antibodies.

There are a number of techniques available for determining CDRs, such as an approach based on cross-species sequence variability (i.e., Kabat et al., Sequences of Proteins of Immunological Interest, (5th ed., 1991, National Institutes of Health, Bethesda Md.)); and an approach based on crystallographic studies of antigen-antibody complexes (Al-lazikani et al. (1997) J. Molec. Biol. 273:927-948). Alternative approaches include the IMGT international ImMunoGeneTics information system (Marie-Paule Lefranc). The Kabat definition is based on sequence variability and is the most commonly used method. The Chothia definition is based on the location of the structural loop regions, whereas the AbM definition is a compromise between the two used by Oxford Molecular's AbM antibody modeling software (refer to www.bioinf.org.uk : Dr. Andrew C.R. Martin's Group). As used herein, a CDR may refer to CDRs defined by one or more approaches or by a combination of these approaches.

In some embodiments, the invention provides an antibody or fragment thereof incorporated into a CAR, wherein said antibody or fragment thereof comprises at least one CDR, at least two, at least three, or more CDRs that are substantially identical to at least one CDR, at least two, at least three, or more CDRs of the antibody of the invention. Other embodiments include antibodies that have at least two, three, four, five, or six CDR(s) that are substantially identical to at least two, three, four, five, or six CDRs of the antibodies of the invention or derived from the antibodies of the invention. In some embodiments, at least one, two, three, four, five, or six CDR(s) are at least about 70%, 75%, 85%, 86%, 87%, 88%, 89%, 90%, 95%, 96%, 97%, 98%, or 99% identical to at least one, two or three CDRs of the antibody of the invention. It is understood that, for purposes of this invention, binding specificity and/or overall activity is generally retained, although the extent of activity may vary compared to said antibody (may be greater or lesser).

*Additional components of the CAR*

In certain embodiments, the CARs contemplated herein may comprise linker residues between the various domains, added for appropriate spacing and conformation of the molecule, for example, a linker comprising an amino acid sequence that connects the VH and VL domains and provides a spacer function compatible with interaction of the two sub-binding domains so that the resulting polypeptide retains a specific binding affinity to the same target molecule as an antibody that comprises the same light and heavy chain variable regions. CARs contemplated herein may comprise one, two, three, four, or five or more linkers. In particular embodiments, the length of a linker is about 1 to about 25 amino acids, about 5 to about 20 amino acids, or about 10 to about 20 amino acids, or any intervening length of amino acids.

Illustrative examples of linkers include glycine polymers; glycine-serine polymers; glycine-alanine polymers; alanine-serine polymers; and other flexible linkers known in the art, such as the Whitlow linker. Glycine and glycine-serine polymers are relatively unstructured and, therefore, may be able to serve as a neutral tether between domains of fusion proteins such as the CARs described herein.

In particular embodiments, the binding domain of the CAR is followed by one or more "spacers" or "spacer polypeptides," which refers to the region that moves the antigen binding domain away from the effector cell surface to enable proper cell/cell contact, antigen binding, and activation. In certain embodiments, a spacer domain is a portion of an immunoglobulin, including, but not limited to, one or more heavy chain constant regions, e.g., CH2 and CH3. The spacer domain can include the amino acid sequence of a naturally occurring immunoglobulin hinge region or an altered immunoglobulin hinge region. In one embodiment, the CAR contemplated herein comprises a spacer domain derived from IgG4, IgG1 (CH3), and/or CD34.

The binding domain of the CAR may, in some embodiments, be followed by one or more "hinge domains," which play a role in positioning the antigen binding domain away from the effector cell surface to enable proper cell/cell contact, antigen binding, and activation. A CAR may comprise one or more hinge domains between the binding and transmembrane domains (TM). The hinge domain may be derived from a natural, synthetic, semi-synthetic, or recombinant source.

The hinge domain can include the amino acid sequence of a naturally occurring immunoglobulin hinge region or an altered immunoglobulin hinge region. Illustrative hinge domains suitable for use in the CARs described herein include the hinge region derived from the extracellular regions of type 1 membrane proteins such as CD8 alpha, CD4, CD28, PD1, CD 152, and CD7, which may be wild-type hinge regions from these molecules or may be altered.

The " transmembrane domain" is the portion of the CAR that fuses the extracellular binding portion and intracellular signaling domain and anchors the CAR to the plasma membrane of the immune effector cell. The TM domain may be derived either from a natural, synthetic, semi-synthetic, or recombinant source. The TM domain may be derived from the alpha, beta, or zeta chain of the T-cell receptor, CD3ε, CD3ζ, CD4, CD5, CD8 alpha, CD9, CD 16, CD22, CD27, CD28, CD33, CD37, CD45, CD64, CD80, CD86, CD 134, CD 137, CD 152, CD 154, and PD1. In one embodiment, the CARs contemplated herein comprise a TM domain derived from CD28.

In particular embodiments, CARs contemplated herein comprise an intracellular signaling domain. An "intracellular signaling domain" refers to the part of a CAR that participates in transducing the message of effective CAR binding to a target polypeptide into the interior of the immune effector cell to elicit effector cell function, e.g., activation, cytokine production, proliferation, and cytotoxic activity, including the release of cytotoxic factors to the CAR-bound target cell, or other cellular responses elicited with antigen binding to the extracellular CAR domain. The term "effector function" refers to a specialized function of an immune effector cell. The effector function of the T cell, for example, may be cytolytic activity or help or activity, including the secretion of a cytokine. Thus, the term "intracellular signaling domain" refers to the portion of a protein that transduces the effector function signal and directs the cell to perform a specialized function.

CARs contemplated herein comprise one or more co-stimulatory signaling domains to enhance the efficacy, expansion, and/or memory formation of T cells expressing CAR receptors. As used herein, the term "co-stimulatory signaling domain" refers to an intracellular signaling domain of a co-stimulatory molecule. Co-stimulatory molecules are cell surface molecules other than antigen receptors or Fc receptors that provide a second signal required for efficient activation and function of T lymphocytes upon binding to antigen. The co-stimulatory molecule is a cell surface molecule other than an antigen receptor or its ligand that contributes to an efficient immune response. Co-stimulatory molecules include MHC class I molecules, BTLA and Toll ligand receptors, OX40, CD27, CD28, CDS, ICAM-1, LFA-1 (CD11a / CD18), ICOS (CD278) and 4-1BB (CD137). Including, but not limited to, further examples of such costimulatory molecules are CDS, ICAM-1, GITR, BAFFR, HVEM (LIGHTTR), SLAMF7, NKp80 (KLRF1), NKp44, NKp30, NKp46, CD160, CD19, CD4, CD8 alpha, CD8 beta, IL2Rbeta, IL2Rgamma, IL7Ralpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE / RANKL, DNAM1 (CD 26), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG / Cbp, CD19a and ligands that specifically bind to CD83,

A co-stimulatory intracellular signaling domain can be the intracellular portion of a co-stimulatory molecule. In one embodiment, the CAR comprises an intracellular domain, which comprises a co-stimulatory domain and a signaling (activation) domain. The CAR construct may therefore include an intracellular signaling domain (CD3 zeta) of the native T cell receptor complex and one or more co-stimulatory domains that provide a second signal to stimulate full T cell activation. Co-stimulatory domains are thought to increase CAR-T cell cytokine production and facilitate T cell replication and T cell persistence. Co-stimulatory domains have also been shown to potentially prevent CAR-T cell exhaustion, increase T cell anti-tumor activity, and enhance survival of CAR-T cells in patients.

In some non-limiting embodiments, an intracellular domain of a CAR may additionally comprise at least one co-stimulating signal domain. Such a co-stimulatory signal domain can result in increased activation of a T cell.

*Genetically modified cells and immune cells*

The present invention contemplates, in particular embodiments, cells genetically modified to express the CARs contemplated herein, for use in the treatment chronic infections in which infected cells express CD30. As used herein, the term "genetically engineered" or "genetically modified" refers to the addition of extra genetic material in the form of DNA or RNA into the total genetic material in a cell. The terms, "genetically modified cells," "modified cells," and, "redirected cells," are used interchangeably.

An "immune cell" or "immune effector cell" is any cell of the immune system that has one or more effector functions (e.g., cytotoxic cell killing activity, secretion of cytokines, induction of ADCC and/or CDC).

In embodiments, the cytotoxic cell is an immune effector cell, preferably a lymphocyte, more preferably a T cell or natural killer (NK) cell.

In embodiments, the invention refers to a genetically modified T cell comprising a chimeric antigen receptor (CAR) that binds CD30 for use in the treatment of chronic (viral) infections in which infected cells express CD30. In embodiments, the invention refers to a genetically modified NK cell comprising a chimeric antigen receptor (CAR) that binds CD30 for use in the treatment of chronic (viral) infections in which infected cells express CD30.

In embodiments, the invention refers to a genetically modified T cell comprising a chimeric antigen receptor (CAR) that binds CD30 for use in the treatment of acute (viral) infections in which infected cells express CD30. In embodiments, the invention refers to a genetically modified NK cell comprising a chimeric antigen receptor (CAR) that binds CD30 for use in the treatment of acute (viral) infections in which infected cells express CD30.

Immune effector cells of the invention can be autologous/autogeneic ("self') or non-autologous ("non-self," e.g., allogeneic, syngeneic, or xenogeneic). "Autologous," as used herein, refers to cells from the same subject and represents a preferred embodiment of the invention. "Allogeneic," as used herein, refers to cells of the same species that differ genetically from the cell in comparison. "Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic.

Illustrative immune effector cells used with the CARs contemplated herein include T lymphocytes. The terms "T cell" or "T lymphocyte" are art-recognized and are intended to include thymocytes, immature T lymphocytes, mature T lymphocytes, resting T lymphocytes, cytokine-induced killer cells (CIK cells), or activated T lymphocytes. Cytokine-induced killer (CIK) cells are typically CD3- and CD56-positive, non-major histocompatibility complex (MHC)-restricted, natural killer (NK)-like T lymphocytes. A T cell can be a T helper (Th; $CD4^+$ T cell) cell, for example, a T helper 1 (Th1) or a T helper 2 (Th2) cell. The T cell can be a cytotoxic T cell (CTL; $CD8^+$ T cell), CD4+CD8+ T cell, CD4 CD8 T cell, or any other subset of T cells. Other illustrative populations of T cells suitable for use in particular embodiments include naive T cells and memory T cells.

For example, when reintroduced back to patients after autologous cell transplantation, the T cells modified with the CAR of the invention as described herein may recognize and kill tumor cells.

As would be understood by the skilled person, other cells may also be used as immune effector cells with the CARs described herein. In particular, immune effector cells include NK cells, NKT cells, neutrophils, and macrophages. Immune effector cells also include progenitors of effector cells, wherein such progenitor cells can be induced to differentiate into immune effector cells *in vivo* or *in vitro.*

The present invention provides methods for making the immune effector cells that express the CAR contemplated herein. In one embodiment, the method comprises transfecting or transducing immune effector cells isolated from an individual such that the immune effector cells express one or more CARs as described herein. In certain embodiments, the immune effector cells are isolated from an individual and genetically modified without further manipulation *in vitro.* Such cells can then be directly re-administered into the individual. In further embodiments, the immune effector cells are first activated and stimulated to proliferate *in vitro* prior to being genetically modified to express a CAR. In this regard, the immune effector cells may be cultured before and/or after being genetically modified (i.e., transduced or transfected to express a CAR contemplated herein).

In particular embodiments, prior to *in vitro* manipulation or genetic modification of the immune effector cells described herein, the source of cells is obtained from a subject. In particular embodiments, the CAR-modified immune effector cells comprise T cells. T cells can be obtained from a number of sources including, but not limited to, peripheral blood mononuclear cells, bone marrow, lymph nodes tissue, cord blood, thymus tissue, tissue from a site of infection, ascites, pleural effusion, spleen tissue, and tumors. In certain embodiments, T cells can be obtained from a unit of blood collected from a subject using any number of techniques known to the skilled person, such as sedimentation, e.g., FICOLL™ separation, antibody-conjugated bead-based methods such as MACS™ separation (Miltenyi Biotec). In one embodiment, cells from the circulating blood of an individual are obtained by apheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing. The cells can be washed with PBS or with another suitable solution that lacks calcium, magnesium, and most if not all, other divalent cations. As would be appreciated by those of ordinary skill in the art, a washing step may be accomplished by methods known to those in the art, such as by using a semiautomated flow through a centrifuge - for example, the Cobe 2991 cell processor, the Baxter CytoMate, or the like. After washing, the cells may be resuspended in a variety of biocompatible buffers or other saline solutions with or without buffer. In certain embodiments, the undesirable components of the apheresis sample may be removed in the cell directly resuspended culture media.

In certain embodiments, T cells are isolated from peripheral blood mononuclear cells (PBMCs) by lysing the red blood cells and depleting the monocytes, for example, by centrifugation through a PERCOLL™ gradient. A specific subpopulation of T cells can be further isolated by positive or negative selection techniques. One method for use herein is cell sorting and/or selection via negative magnetic immunoadherence or flow cytometry that uses a cocktail of monoclonal antibodies directed to cell surface markers present on the cells negatively selected.

PBMC may be directly genetically modified to express CARs using methods contemplated herein. In certain embodiments, after isolation of PBMC, T lymphocytes are further isolated, and in certain embodiments, both cytotoxic and helper T lymphocytes can be sorted into naive, memory, and effector T cell subpopulations either before or after genetic modification and/or expansion. $CD8^+$ cells can be obtained by using standard methods. In some embodiments, $CD8^+$ cells are further sorted into naive, central memory, and effector cells by identifying cell surface antigens that are associated with each of those types of CD8$^+$ cells.

The immune effector cells, such as T cells, can be genetically modified following isolation using known methods, or the immune effector cells can be activated and expanded (or differentiated in the case of progenitors) *in vitro* prior to being genetically modified. In a particular embodiment, the immune effector cells, such as T cells, are genetically modified with the chimeric antigen receptors contemplated herein (e.g., transduced with a viral vector comprising a nucleic acid encoding a CAR) and then are activated and expanded *in vitro.* In various embodiments, T cells can be activated and expanded before or after genetic modification to express a CAR, using methods as described, for example, in U.S. Patents 6,352,694; 6,534,055; 6,905,680; 6,692,964; 5,858,358; 6,887,466; 6,905,681 ; 7, 144,575; 7,067,318; 7, 172,869; 7,232,566; 7, 175,843; 5,883,223; 6,905,874; 6,797,514; 6,867,041; and U.S. Patent Application Publication No. 20060121005.

The "T-cell receptor alpha constant (TRAC)" locus, encoded by the TRA gene, contributes the alpha chain to the T-cell receptor (TCR) protein, which is crucial for recognizing foreign antigens presented by major histocompatibility complex (MHC) molecules on antigen-presenting cells (APC). The TCR alpha locus undergoes rearrangement during T cell development, synthesizing a diverse repertoire of T cell receptors capable of recognizing a wide range of antigens. Upon binding to peptide-MHC complexes, the TCR-CD3 complex initiates intracellular signaling cascades, leading to T-cell activation and subsequent immune responses against pathogens or tumor cells. The interaction between TCRs and peptide-MHC complexes shapes the structural and functional avidity of T cells, contributing to the overall adaptive immune response.

To address the limitations of CARs, CARs can be precisely integrated into the T-cell receptor alpha constant (TRAC) locus using CRISPR/Cas technology. This approach results in uniform CAR expression, reduced tonic signaling, decreased T-cell exhaustion, and increased efficacy, offering a safer and more potent therapeutic product. Additionally, targeting the TRAC locus enables generating potential universal CAR products.

The term "bystander inflammatory cells" refers to immune cells activated independently of their specific antigen recognition and play crucial roles in various disease contexts, including infection, autoimmunity, and cancer. These cells, often T lymphocytes, can become activated through mechanisms other than traditional antigen presentation via the T cell receptor (TCR). For instance, pro-inflammatory cytokines, such as interleukins (IL) and interferons (IFN), can trigger the activation of bystander T cells, particularly CD4$^+$ and CD8$^+$ T cell subsets. This activation occurs even without direct antigen stimulation, leading to rapid secretion of effector cytokines like IFN-$\gamma$ and IL-17. Notably, bystander activation of CD4$^+$ T cells has been implicated in infection clearance and autoimmune pathogenesis, highlighting the complex interplay between innate and adaptive immunity. Additionally, bystander-activated T cells or B cells have been observed in viral infections or cancer or auto-immunity, where they contribute to immune responses and may influence disease outcomes.

In embodiments, the cytotoxic cell induces cytotoxic activity against human CD30-expressing infected cells and/or bystander inflammatory cells. In embodiments, the cytotoxic cell induces cytotoxic activity against CD30-expressing bystander T or B lymphocytes.

In embodiments, the cytotoxic cell targets a reservoir comprising CD30-expressing T cells.

"B cells" or "B lymphocytes" constitute a subset of white blood cells within the lymphocyte category, operating in the humoral immunity sector of the adaptive immune system. These cells play a crucial role by producing antibody molecules that can be secreted or embedded in the plasma membrane, serving as B-cell receptors. Upon activation by an antigen, B cells proliferate and transform into antibody-secreting effector cells called plasmablasts or plasma cells. B cells act as professional antigen-presenting cells, presenting antigens and secreting cytokines. In mammals, B cells mature in the bone marrow. Unlike T cells and natural killer cells, B cells express B cell receptors (BCRs) on their membrane, facilitating the binding to foreign antigens and initiating an antibody response. Development starts from hematopoietic stem cells in the bone marrow, progressing through various stages with specific gene expressions and rearrangements. B cells undergo positive and negative selections during development to ensure proper maturation, involving BCRs in both processes. Positive selection relies on antigen-independent signaling through pre-BCR and BCR, while negative selection involves the binding of self-antigens to BCRs, leading to various outcomes such as clonal deletion, receptor editing, anergy, or ignorance. After development, immature B cells migrate to the spleen as transitional B cells, transitioning from T1 to T2. These cells further differentiate into follicular (FO) B cells or marginal zone (MZ) B cells based on signals received through BCRs and other receptors. B cell activation occurs in secondary lymphoid organs like the spleen and lymph nodes. T cell-dependent activation involves the interaction of B cells with T cells, resulting in a prolonged but high-affinity antibody response. T cell-independent activation, on the other hand, is faster but produces antibodies with lower affinity. Activated B cells undergo differentiation into plasmablasts during the extrafollicular response or form germinal centers for extensive proliferation, immunoglobulin class switching, and affinity maturation, generating long-lived plasma cells and memory B cells. B cell types include plasmablasts, plasma cells, lymphoplasmacytoid cells, memory B cells, B-2 cells (FO and MZ B cells), B-1 cells, and regulatory B (Breg) cells, each with specific functions and locations within the body. B cell-related pathologies include autoimmune diseases resulting from abnormal B cell recognition of self-antigens and malignant transformations leading to cancers such as chronic lymphocytic leukemia, lymphomas, and multiple myeloma. Abnormal B cells may also cause conditions like diffuse large B-cell lymphomas.

In embodiments, the cytotoxic cell induces cytotoxic activity against CD30-expressing bystander B lymphocytes.

In embodiments, the cytotoxic cell targets a reservoir comprising CD30-expressing B cells.

"Myeloid cells," including granulocytes and monocytes, originate from hematopoietic stem cells in the bone marrow and constitute a major component of the peripheral blood leukocytes. Upon pathogen invasion, myeloid cells are swiftly recruited to local tissues via chemokine receptors, where they perform essential roles in innate immunity by engaging in phagocytosis and secreting inflammatory cytokines. Monocytes differentiate into tissue-specific macrophages, such as Kupffer cells in the liver and alveolar macrophages in the lungs, while granulocytes serve as the first line of defense against infections. Myeloid neoplasms, often associated with genetic alterations, can lead to conditions like chronic or acute myelogenous leukemia. Myeloid tissue, resembling or originating from the bone marrow, may also be present in organs like the liver and spleen, leading to extramedullary hematopoiesis.

In embodiments, the cytotoxic cell targets a reservoir comprising CD30-expressing myeloid cells.

"Natural Killer (NK) cells," also known as large granular lymphocytes (LGL), are a vital component of the innate immune system, comprising 5-20% of circulating lymphocytes in humans. Functionally analogous to cytotoxic T cells in adaptive immunity, NK cells respond rapidly to virus-infected cells and tumors, acting approximately three days after infection. Unlike most immune cells, NK cells do not require antibodies or major histocompatibility complex (MHC) for target recognition, allowing for a swift immune response. Identified by the presence of CD56 and absence of CD3, NK cells differentiate from $CD127^+$ common innate lymphoid progenitors and mature in various tissues before entering circulation. $CD56^{bright}$ NK cells, predominant in bone marrow and lymphoid tissues, exhibit immunoregulatory roles, while CD56dim NK cells in peripheral blood excel in cell killing. There are various sources from which NK cells can be derived, e.g., peripheral blood mononuclear cells, cord blood, immortalized cell lines, hematopoietic stem and progenitor cells (HSPCs), and induced pluripotent stem cells (iPSCs). All sources can provide clinically meaningful cell doses, are amenable to CAR receptor engineering, and have transitioned into in-human studies. NK cells express distinct receptors, including inhibitory receptors recognizing self-MHC I and activating receptors (Raftery MJ, Ann Rev of Cancer Biol, 2023). Activating molecules may include the natural cytotoxicity receptors, NKG2D, some CD94/NKG2 complexes, and CD16 (FcγIII). Activating molecules recognize partner ligands on cells except for CD16, a low-affinity Fc receptor responsible for antibody-dependent cellular cytotoxicity (ADCC). Inhibitory molecules may include the killer cell immunoglobulin-like receptor (KIR) family of molecules, some CD94/NKG2 complexes, and leukocyte Ig-like receptors (LIRs). These receptors govern NK cell activity, adjusting to environmental cues. NK cells contribute to both innate and adaptive immunity, demonstrating antigen-specific immunological memory.

In embodiments, the cytotoxic cell is a natural killer (NK) cell.

*Nucleic acids*

As used herein, the terms "polynucleotide" or "nucleic acid molecule" refer to any nucleic acid molecule, for example, DNA or RNA, such as messenger RNA (mRNA), RNA, genomic RNA (gRNA), plus-strand RNA (RNA(+)), minus-strand RNA (RNA(-)), genomic DNA (gDNA), complementary DNA (cDNA) or recombinant DNA. Polynucleotides include single and double-stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100% sequence identity to any of the reference sequences described herein, typically where the variant maintains at least one biological activity of the reference sequence. In various illustrative embodiments, the present invention contemplates, in part, polynucleotides comprising expression vectors, viral vectors, transfer plasmids and compositions, and cells comprising the same.

In various illustrative embodiments, the present invention contemplates, in part, polynucleotides comprising expression vectors, viral vectors, transfer plasmids and compositions, and cells comprising the same. Polynucleotides can be prepared, manipulated and/or expressed using any of a variety of well-established techniques known and available in the art. In order to express a desired polypeptide, a nucleotide sequence encoding the polypeptide can be inserted into an appropriate vector. Examples of vectors are plasmids, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI-derived artificial chromosome (PAC), bacteriophages such as lambda phage or Ml 3 phage, and animal viruses. Examples of categories of animal viruses useful as vectors include, without limitation, retrovirus (including lentivirus), adenovirus, adeno-associated virus, herpesvirus {e.g., herpes simplex virus), poxvirus, baculovirus, papillomavirus, and papovavirus (e.g., SV40). Examples of expression vectors are pCIneo vectors (Promega) for expression in mammalian cells; pLenti4/V5-DEST™, pLenti6/V5-DEST™, and pLenti6.2/V5-GW/IacZ (Invitrogen) for lentivirus-mediated gene transfer and expression in mammalian cells. In particular embodiments, the coding sequences of the chimeric proteins disclosed herein can be ligated into such expression vectors for the expression of the chimeric protein in mammalian cells. The "control elements" or "regulatory sequences" present in an expression vector are those non-translated regions of the vector - origin of replication, selection cassettes, promoters, enhancers, translation initiation signals (Shine Dalgarno sequence or Kozak sequence) introns, a polyadenylation sequence, 5' and 3' untranslated regions - which interact with host cellular proteins to carry out transcription and translation. Such elements may vary in their strength and specificity. Depending on the vector system and host utilized, any number of suitable transcription and translation elements, including ubiquitous promoters and inducible promoters, may be used.

*Gene editing*

As used herein, the terms "gene-edited" and "genetically modified" may be used interchangeably. A gene-editing technique can be used to interfere with the endogenous expression of any expressed structure on a genome, preferably the TCR complex component, CD5, CD7, CD52, MHC, and/or a molecule involved in a control point gene. "Endogenous," as used herein, is particularly used to define a gene expression that originates from within a system such as a host, organism, tissue, or cell, whereas "exogenous" particularly defines a genetic sequence introduced from externally into a cell by one or more genetic, biochemical or other methods.

Gene editing is a type of genetic engineering in which DNA is inserted, deleted, or replaced in the genome of a living organism by manipulated nucleases, or "molecular Scissors." These nucleases generate site-specific double-strand breaks (DSBs) at desired sites in the genome. The induced double-strand breaks are repaired by non-homologous end compound (HEJ) or homologous recombination (HR), resulting in targeted mutations ("edits"). Five families of manipulated nucleases can be used for gene editing: (a) meganucleases, (b) zinc finger nuclei (ZFNs), (c) transcription activator-like effector-based nucleases (TALENs), (d) Mega-TALENs, and (e) the CRISPR-Cas system.

In certain embodiments, CRISP/Cas is preferably used for gene editing.

CRISPR gene editing, derived from bacterial antiviral defense mechanisms, is a technique in molecular biology that enables precise modification of living organisms' genomes. Using the Cas nuclease complex with a guide RNA (gRNA), specific locations within a cell's genome can be targeted for modification, including gene removal and addition. Notable advancements include the development of variants with reduced off-target effects and the approval of Casgevy, the first CRISPR-based drug, for treating sickle-cell disease and beta thalassemia. CRISPR technology has revolutionized biological research, enabling rapid genome sequencing and editing. Techniques such as CRISPR-induced gene knockouts and CRISPR screens have transformed basic and translational research, providing insights into genetic interactions and biological pathways. Emerging technologies like base editing and prime editing offer precise editing capabilities without introducing double-stranded breaks, expanding the CRISPR toolbox. Challenges remain in improving editing accuracy, precision, and efficiency, as well as developing methods for large gene insertions. Despite these challenges, CRISPR technology holds immense promise for advancing genetic research and therapeutic development.

CRISPR gene editing operates through a mechanism inspired by the natural defense system found in bacteria against viral infections. This system consists of two main components: the Cas protein and a guide RNA (gRNA). The gRNA is designed to complement a specific target sequence within the genome. When the Cas protein and gRNA complex encounter the target DNA sequence, the gRNA guides Cas to bind precisely to that location. Once bound, the Cas protein acts as molecular scissors, cutting both DNA strands at the target site. This creates a double-stranded break (DSB) in the DNA. The cell's natural repair mechanisms then come into play to fix the break. Two main repair pathways are involved: non-homologous end joining (NHEJ) and homology-directed repair (HDR). In the NHEJ pathway, the broken ends of the DNA are rejoined, often resulting in small insertions or deletions at the repair site. This process can introduce mutations, leading to gene knockout or disruption. Alternatively, a repair template containing the desired genetic sequence can be provided in the HDR pathway along with the Cas-gRNA complex. This repair template can be used to introduce specific changes or additions to the DNA sequence at the site of the break, allowing for precise editing of the genome by incorporating new genetic material.

CRISPR/Cas9 technology is increasingly employed in CAR-T cell engineering to address various limitations in current approaches. By disrupting TCR and HLA-I expression, CRISPR/Cas9 facilitates the creation of off-the-shelf universal CAR-T cells, reducing the risk of graft-versus-host disease (GVHD) and graft rejection. Additionally, CRISPR/-Cas9 enables CAR-T cells to resist immunosuppressive cytokines while, e.g., enhancing their anti-tumor potency through the secretion of specific cytokines like IL-15, IL-18, and IL-23. Clinical trials demonstrate the safety and efficacy of CRISPR-engineered CAR-T cells in hematopoietic malignancies and solid tumors.

In embodiments, the cytotoxic cell is genetically modified by CRISPR/Cas gene editing, preferably CRISPR/-Cas9. In embodiments, the VL-VH sequences of the humanized CD30 monoclonal antibody are inserted into the TRAC locus via CRISPR/Cas gene editing.

In embodiments, a guide-RNA (gRNA) sequence targets and/or disrupts the TRAC or CD3 zeta genomic locus.

One or more of these techniques can be used alone or in any combination to disrupt the endogenous expression of a TCR-complex component in the method of the invention. When the gene-editing technique is used to disrupt the expression of a TCR complex component, the gene-editing technology can particularly target one or more of the following: (i) a CD3 zeta locus, (ii) a safe harbor locus, (iii) a TRAC locus; (ii) a locus controlling TCR-beta expression; (iii) a locus controlling TCR gamma expression; (iv) a locus that controls TCR delta expression; (v) a locus that controls the expression of a CD3 chain. CD3 chains are described above. The CD3 chain can be CD3 zeta, CD3 epsilon, CD3 delta, or CD3 gamma. In certain embodiments, genome loci harboring genetic information for expressing at least one TRAC locus are preferably targeted for gene editing.

In some embodiments, a genetically modified cell or animal comprising an inactivated genomic sequence may be termed a "knock-out" or a "conditional knock-out." Similarly, a genetically modified cell or animal comprising an integrated sequence may be termed a "knock-in" or a "conditional knock-in."

The term "promoter" is used for a DNA sequence that initiates and regulates transcription from the DNA downstream of it through the binding of proteins. The transcribed RNA may encode a protein.

Promoters are preferably located near the transcription start sites of genes, upstream of the coding DNA (towards the 5' region of the sense strand).

In some embodiments, it may be preferable to use a cell-type, cell lineage, or tissue-specific expression control sequence to achieve cell-type, lineage, or tissue-specific expression of a desired polynucleotide sequence (e.g., to express a particular nucleic acid encoding a peptide only in a subset of cell types, cell lines or tissues or during certain stages of development).

In embodiments, the nucleic acid sequence encoding the CD30-CAR is expressed from an endogenous promoter.

*Sequence Variants*

Sequence variants of the claimed nucleic acids, proteins, antibodies, antibody fragments, and/or CARs, for example, those defined by % sequence identity, that maintain similar binding properties of the invention are also included in the scope of the invention. Such variants, which show alternative sequences, maintain essentially the same binding properties, such as target specificity, as the specific sequences provided are known as functional analogs or functionally analogous. Sequence identity relates to the percentage of identical nucleotides or amino acids when carrying out a sequence alignment.

The recitation "sequence identity," as used herein, refers to the extent to which sequences are identical on a nucleotide-by-nucleotide basis or an amino acid-by-amino acid basis over a comparison window. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, He, Phe, Tyr, Trp, Lys, Arg, His, Asp, Glu, Asn, Gin, Cys, and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions, and other changes in sequence that fall under the described sequence identity are also encompassed in the invention.

Protein sequence modifications, which may occur through substitutions, are also included within the scope of the invention. Substitutions, as defined herein, are modifications made to the amino acid sequence of the protein, whereby one or more amino acids are replaced with the same number of (different) amino acids, producing a protein that contains a different amino acid sequence than the primary protein. Substitutions may be carried out that preferably do not significantly alter the function of the protein. Like additions, substitutions may be natural or artificial. It is well known in the art that amino acid substitutions may be made without significantly altering the protein's function. This is particularly true when the modification relates to a "conservative" amino acid substitution, substituting one amino acid for another of similar properties. Such "conserved" amino acids can be natural or synthetic, which, because of size, charge, polarity, and conformation, can be substituted without significantly affecting the structure and function of the protein. Frequently, many amino acids may be substituted by conservative amino acids without deleteriously affecting the protein's function.

In general, the non-polar amino acids Gly, Ala, Val, Ile, and Leu; the non-polar aromatic amino acids Phe, Trp, and Tyr; the neutral polar amino acids Ser, Thr, Cys, Gln, Asn, and Met; the positively charged amino acids Lys, Arg and His; the negatively charged amino acids Asp and Glu, represent groups of conservative amino acids. This list is incomplete. For example, it is well known that Ala, Gly, Ser, and sometimes Cys can substitute for each other even though they belong to different groups.

Substitution variants have at least one amino acid residue in the antibody molecule removed and a different residue inserted. The sites of most significant interest for substitutional mutagenesis include the hypervariable regions, but FR alterations are also contemplated. If such substitutions result in a change in biological activity, then more substantial changes, denominated "exemplary substitutions" in the table immediately below, or as further described below in reference to amino acid classes, may be introduced, and the products screened.

**Table 2.** Potential amino acid substitutions:

| Original residue | Preferred conservative substitutions | Examples of exemplary substitutions |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids but include synthetic ones. Commonly used synthetic amino acids are omega amino acids of various chain lengths and cyclohexyl alanine, which are neutral non-polar analogs; citrulline and methionine sulfoxide, which are neutral non-polar analogs; phenyl glycine which is an aromatic neutral analog; cysteic acid which is a negatively charged analog and ornithine which is a positively charged amino acid analog. Like the naturally occurring amino acids, this list is not exhaustive but merely exemplary of the well-known substitutions in the art.

"Codon optimization" involves gene engineering strategies that employ synonymous codon changes to enhance protein production, commonly utilized in the production of protein pharmaceuticals and nucleic acid therapies, including gene therapy, mRNA therapy, and DNA/RNA vaccines. Codon optimization methods may include deep learning, utilizing the concept of "codon boxes" to recode DNA sequences (Hongguang F et al., Sci Rep, 2020).

*Viruses*

As used herein, a "chronic inflammatory virus" refers to a virus that can cause persistent viral infections that maintain long-term inflammation within the host, potentially leading to autoimmune processes. This sustained inflammation, often triggered by viruses such as cytomegalovirus, Epstein-Barr virus, and hepatitis C virus, contributes to the initiation and perpetuation of autoimmune diseases. Chronic inflammation may facilitate the activation of autoreactive T cells, leading to a cross-reactive response against self-antigens mimicked by the viruses. Additionally, persistent viruses and chronic inflammation can synergistically support autoimmunity through mechanisms such as unveiling cryptic self-epitopes, activating dendritic cells, and constantly priming new autoreactive T cells. While chronic infections are crucial in maintaining inflammation, they interact with various environmental and genetic factors to influence health outcomes.

In embodiments, the cytotoxic cell binds to a human cell infected with a chronic inflammatory virus. In embodiments, the cytotoxic cell binds to a human cell infected with a chronic inflammatory virus expressing CD30.

In embodiments, the chronic inflammatory virus is a retrovirus or a herpesvirus. In preferred embodiments, the chronic inflammatory virus is an Epstein-Barr virus (EBV), a human cytomegalovirus (HCMV), a human immunodeficiency virus (HIV), human T-cell lymphotropic virus (HTLV).

In embodiments, the chronic inflammatory virus causes persistent viral infections, preferably a HIV and/or EBV infection. In embodiments, the chronic inflammatory virus leads to an autoimmune disease, preferably rheumatoid arthritis, systemic lupus erythematosus (SLE), multiple sclerosis (scleroderma), Sjörgen's syndrome, Hashimoto's thyroiditis, Wegner's granulomatosis, primary biliary cirrhosis, anti-phospholipid syndrome, uveitis and/or vasculitis; an allergic condition, preferably atopic dermatitis, asthma, and/or allergic rhinitis; and/or a disease associated with inflammation.

As used herein, a "viral reservoir" is an anatomical site in which a virus accumulates and persists. Viral reservoirs encompass one or more interconnected populations or environments where a virus can persist indefinitely and from which it is transmitted to the target population. The target population is the species in which the virus causes disease. Viruses within reservoirs maintain their life cycle without significantly impacting host health, often establishing a commensal relationship. Multi-host organisms can have multiple reservoirs.

In embodiments, the viral reservoir comprises CD30-expressing B cells, T cells, and/or myeloid cells.

There are multiple "herpesvirus" types known to infect humans, those of primary relevance being herpes simplex viruses 1 and 2 (HSV-1 and HSV-2, also known as HHV1 and HHV2), varicella-zoster virus (VZV, also known as HHV-3), Epstein-Barr virus (EBV or HHV-4), human cytomegalovirus (HCMV or HHV-5), human herpesvirus 6A and 6B (HHV-6A and HHV-6B), human herpesvirus 7 (HHV-7), and Kaposi's sarcoma-associated herpesvirus (KSHV, also known as HHV-8). Herpes viruses share a common structure, being composed of relatively large double-stranded, linear DNA genomes encoding 100-200 genes encased within an icosahedral protein cage called the capsid, which is itself wrapped in a protein layer called the tegument, containing both viral proteins and viral mRNAs and a lipid bilayer membrane called the envelope. Infection is initiated when a viral particle contacts a cell with specific receptor molecules on the cell surface.

Following the binding of viral envelope glycoproteins to cell membrane receptors, the virion is internalized and dismantled, allowing viral DNA to migrate to the cell nucleus. Within the nucleus, replication of viral DNA and transcription of viral genes occurs. During symptomatic infection, infected cells transcribe lytic viral genes. In some host cells, a small number of viral genes termed latency-associated transcript (LAT) accumulate instead. In this fashion, the virus can persist indefinitely in the cell (and thus the host). While primary infection is often accompanied by a self-limited period of clinical illness, long-term latency is typically symptom-free. The reactivation of latent viruses has been implicated in a number of diseases. Following activation, transcription of viral genes transitions from LAT to multiple lytic genes; these lead to enhanced replication and virus production. Often, lytic activation leads to cell death. Clinically, lytic activation is often accompanied by the emergence of non-specific symptoms such as low-grade fever, headache, sore throat, malaise, and rash, as well as clinical signs such as swollen or tender lymph nodes and immunological findings.

"Epstein-Barr virus (EBV)" is a human herpesvirus that is carried by almost all of the adult population of the world. Many primary infections occur in childhood and are asymptomatic. Those occurring after the age of about 12- or 13-year-old are more likely to be accompanied by infectious mononucleosis, a self-limiting but temporarily debilitating immunopathology. Regardless of whether or not infection is accompanied by disease, the virus persists. It establishes latency in long-lived memory B cells and reactivates sporadically to be amplified in epithelial cells, shed in saliva for oral transmission to a new host, or returned to B cells to replenish the B-cell reservoir. The vast majority of people suffer no ill effects from the persistence of EBV, but the virus is nevertheless associated, and probably causally associated, with a number of B cell and epithelial cell malignancies, including Burkitt and Hodgkin lymphoma, post-transplant lymphoproliferative disorders and immunoblastic lymphomas of the immunosuppressed, anaplastic nasopharyngeal carcinomas and a subset of gastric carcinomas. It is also linked with autoimmune diseases such as multiple sclerosis (Soldann et al., 2023).

As used herein, "cytomegalovirus (CMV)" refers to a member of the beta subclass of the family of herpesviruses. CMV is a relatively large (containing a 230 kilobase genome), double-stranded DNA virus with host-range specific variants such as MCMV (murine CMV) and HCMV (human CMV). Human cytomegalovirus (HCMV), also known as human betaherpesvirus 5 (HHV-5), is a member of the herpesvirus family and the Betaherpesvirinae subfamily. It is a double-stranded DNA virus that is widespread globally, with a seroprevalence of 60-90% in adults. While HCMV infection is often asymptomatic in healthy individuals, it poses significant risks to immunocompromised individuals and can lead to severe complications in newborns, such as congenital neurological disease. HCMV exhibits broad cell tropism, infecting various cell types, and can establish latency in myeloid cells, resulting in lifelong infection with intermittent reactivation. Transmission is primarily through bodily fluids, and infection can occur via close contact, sexual intercourse, organ transplantation, or blood transfusion.

"Retroviruses," members of the Retroviridae family, are enveloped viruses with a diameter of approximately 100 nm, characterized by a single-stranded RNA genome of about 7-10 kb. They replicate by converting their RNA into a DNA intermediate through the action of the enzyme reverse transcriptase, which then integrates into the host cell's chromosome, forming a provirus. Retroviruses are divided into simple retroviruses and complex retroviruses, with the latter encoding additional accessory proteins. They are known for their association with various diseases, including but not limited to cancers, AIDS, and neurological disorders, and have been identified in a wide range of species.

Retroviruses exhibit a characteristic structure consisting of enveloped particles approximately 100 nm in diameter. These virions contain a single-stranded RNA genome of about 7-10 kb, which is enclosed within a lipid envelope derived from the host plasma membrane during the budding process. The outer envelope is studded with glycoproteins encoded by the env gene, which protect the virus, facilitate entry into host cells, and mediate membrane fusion. The RNA genome, present as a dimer, has specific regions such as R, U5, PBS, and L at the 5' end and PPT, U3, and R at the 3' end, which are essential for replication and transcription. Inside the virion, the RNA is associated with proteins, including group-specific antigen (Gag) proteins, protease (PR), polymerase (Pol), and nucleocapsid (NC) proteins, each fulfilling distinct functions in viral replication and assembly. Retroviruses possess a unique enzyme, reverse transcriptase (RT), which transcribes the viral RNA into DNA during infection, leading to the integration of the viral genome into the host cell chromosome as a provirus. This integrated DNA then serves as a template for viral gene expression and replication. Overall, the structure of retroviruses reflects their intricate machinery for infecting host cells and perpetuating their lifecycle.

In embodiments, the cytotoxic cell is used in the treatment of viral infections caused by a retrovirus, preferably human immunodeficiency virus (HIV) and/or human T-cell lymphotropic virus (HTLV).

*Diseases*

The terms "disorder," "disease," or "medical condition," as used herein, can be used interchangeably.

As used herein, the term "infection" has its ordinary meaning as understood by a skilled person. An infection is typically understood as the invasion of a subject (host), for example their tissues or cells, by a pathogen. The infection may also be considered to include pathogen multiplication, and the reaction of host tissues or cells to the infectious agent. Infections may lead to infectious diseases, also known as transmissible or communicable diseases, which are illnesses resulting from an infection. Infections can be caused by a wide range of pathogens, most prominently bacteria and viruses, but also including fungi or parasites. In embodiments, the infection to be treated comprises, or is associated with, infected cells of the host (patient, subject) expressing CD30 on the cell surface, thus enabling targeting by the CD30 CARs of the present invention. A skilled person is capable of determining which infections belong to this group, for example, by simple assessment of CD30 expression in infected cells.

In embodiments, the infection is a bacterial infection. Non-limiting examples of a bacterial infection, common in human subjects, are Mycobacterium tuberculosis, Staphylococcus aureus, Escherichia coli, Clostridium botulinum, and Salmonella species.

As used herein, a tuberculosis infection has its ordinary meaning as understood by a skilled person. Mycobacterium tuberculosis (M. tb), also known as Koch's bacillus, is a species of pathogenic bacteria in the family Mycobacteriaceae and is the causative agent of tuberculosis. M. tuberculosis belongs to a genetically related group of Mycobacterium species called Mycobacterium tuberculosis complex, including, without limitation, M. tuberculosis, M. africanum, M. canettii, M. bovis, M. caprae, M. microti, M. pinnipedii, M. mungi, and M. orygis. Humans are the only known reservoirs of M. tuberculosis, and major spread occurs through air droplets originating from a person who has the disease. In embodiments, the tuberculosis infection to be treated comprises, or is associated with, infected cells of the host (patient, subject) expressing CD30 on the cell surface, thus enabling targeting by the CD30 CARs of the present invention. A skilled person is capable of determining which tuberculosis infections belong to this group, for example, by simple assessment of CD30 expression in infected cells.

The terms "chronic infection," "chronic disease," or "chronic medical impairment" are used interchangeably and are characterized by their persistent or long-lasting effects, typically lasting over three months. Chronic conditions often impact multiple areas of the body, exhibit incomplete responsiveness to treatment, and endure over an extended duration. Periods of remission and relapse, where the condition temporarily improves or reoccurs, are common in chronic diseases. These conditions are frequently linked to non-communicable diseases, which have non-infectious causes and encompass a range of health states, including syndromes, physical impairments, disabilities, and diseases.

"Chronic viral infections" exhibit diverse strategies for persistence within the host. These strategies include continuous replication, latency and reactivation, and integration into the host genome, each presenting unique challenges to the immune system. Continuous replication, exemplified by viruses like HIV, HBV, and HCV, generates large numbers of viral particles daily, leading to ongoing antigenic stimulation of lymphocytes and potential tissue damage. Latent infections, such as those caused by HIV and EBV, establish a quiescent state, evading immune detection and clearance until reactivation occurs. Reactivation events, common in herpesviruses, stimulate the immune system but may not always result in productive infection. Additionally, mammalian genomes harbor endogenous retroviral elements (ERVs) transmitted vertically, which can influence immune responses through encoding antigens or superantigens.

"Autoimmune diseases" result from an abnormal immune system response, wherein it mistakenly attacks healthy parts of the body, perceiving them as foreign invaders. With over 80 recognized types, autoimmune diseases encompass various conditions affecting various body parts. Symptoms, ranging from fatigue to joint pain and skin rashes, can vary and fluctuate in intensity. These diseases, often affecting blood vessels, connective tissues, and glands, can pose diagnostic challenges due to their variable presentations. Treatment primarily aims to manage symptoms and suppress immune activity, typically involving medications like NSAIDs and immunosuppressants. Genetics, environmental factors, and viral infections are implicated in their development, with mechanisms including molecular mimicry and bystander activation.

Autoimmune diseases can also arise due to viral infections, adding another layer of complexity to their etiology. Certain viruses, such as herpesviruses, have been linked to the development of multiple autoimmune disorders, including multiple sclerosis (MS), systemic lupus erythematosus (SLE), and rheumatoid arthritis (RA). Mechanisms by which viruses induce autoimmunity include molecular mimicry, wherein viral antigens resemble self-antigens, leading to cross-reactive immune responses. Additionally, bystander activation occurs when a robust antiviral immune response creates a pro-inflammatory environment, triggering autoreactive T cells. Epitope spreading is another mechanism wherein viral infections induce the release of self-antigens, activating previously non-responsive autoreactive cells. Furthermore, viruses like Epstein-Barr virus (EBV) can immortalize autoreactive effector cells, exacerbating autoimmune responses.

In embodiments, the cytotoxic cell is used in the treatment of autoimmune diseases.

"Allergies," or allergic conditions, result from an overreaction of the immune system to normally harmless environmental substances. These conditions encompass a range of disorders, such as hay fever, food allergies, atopic dermatitis, allergic asthma, and anaphylaxis. Symptoms vary widely but can include red eyes, itching, sneezing, coughing, shortness of breath, or swelling. Common allergens include pollen, certain foods, metals, insect stings, and medications. Both genetic and environmental factors influence allergy development. The underlying mechanism involves immunoglobulin E (IgE) antibodies binding to allergens, triggering the release of inflammatory chemicals like histamine from mast cells or basophils. Diagnosis typically relies on medical history, with further testing occasionally necessary. Treatments range from avoidance of allergens to medications such as antihistamines and steroids. In severe cases, injectable adrenaline may be required. Allergen immunotherapy is effective for some allergies, gradually desensitizing individuals to specific allergens.

CD30 has been implicated in allergic diseases such as atopic dermatitis (AD) and asthma. Elevated levels of soluble CD30 (sCD30) have been observed in AD patients, particularly during acute phases, indicating its regulatory role in Th2-mediated inflammation. Similarly, increased sCD30 levels in asthma are associated with allergen-specific Th2 cytokine production in atopic asthmatics. CD30 expression on CD4+ T cells correlates with serum IgE levels in allergic patients, suggesting its involvement in the IgE response. Elevated sCD30 levels have also been reported in allergic rhinoconjunctivitis and food allergy, indicating a potential role for CD30 in these conditions.

In embodiments, the cytotoxic cell is used in the treatment of allergies.

"Inflammation" is a biological response triggered by harmful stimuli, including pathogens, damaged cells, or irritants. It is a protective mechanism involving immune cells, blood vessels, and molecular mediators to eliminate the initial cause of cell injury, clear damaged cells and tissues, and initiate tissue repair. Acute inflammation is the immediate response to injury, characterized by increased blood flow and migration of immune cells to the affected area, while chronic inflammation persists for months or years, involving different cell types and often leading to tissue destruction. The process is regulated by a complex interplay of cellular and biochemical factors, with inflammation being essential for efficient immunity and tissue healing but also having negative effects when dysregulated. Inflammation is distinct from infection but is often associated with it. Diseases associated with chronic inflammation are multiple and include but are not limited to cardiovascular diseases (CVD), diabetes, malignancy, auto-immune disease, chronic hepatic and renal disease, etc.

In embodiments, the cytotoxic cell is used in the treatment of diseases associated with inflammation.

"Precancer" refers to morphologically distinguishable proliferative lesions associated with an increased risk of developing cancer. These lesions undergo a premalignant phase where oncogenic genetic and epigenetic alterations accumulate before becoming malignant. The pathophysiology of precancerous lesions mirrors that of cancer, with varying durations of the premalignant phase depending on cancer type and individual factors. Evasion of the immune system is observed in premalignant lesions, and the nature of the immune response may influence progression or regression to normal tissue. Clinically, precancerous conditions encompass abnormal tissues prone to cancer development, such as colon polyps, monoclonal gammopathy of undetermined significance, and cervical dysplasia. Pathologically, precancerous tissue ranges from benign neoplasias to dysplasia and carcinoma in situ, with varying degrees of invasion and progression to invasive cancer. Precancerous conditions affect multiple organ systems and may include hereditary genetic conditions predisposing individuals to cancer.

Precancerous cells exhibit distinct features from non-cancerous cells, which may include abnormal growth, abnormal development, changes in appearance, and/or changes in DNA, and have the potential to turn into cancer cells.

The skilled person in the art is able to distinguish precancerous cells from normal non-cancerous cells.

In embodiments, the cytotoxic cell targets a pre-cancerous human CD30-expressing cell.

*Compositions and Formulations*

The compositions contemplated herein may comprise one or more polypeptides, polynucleotides, vectors comprising said polynucleotides, genetically modified immune effector cells, etc., as contemplated herein. Compositions include but are not limited to pharmaceutical compositions.

A "pharmaceutical composition" refers to a composition formulated in pharmaceutically acceptable or physiologically acceptable solutions for administration to a cell or an animal, either alone or in combination with one or more other therapy modalities. It will also be understood that, if desired, the compositions of the invention may be administered in combination with other agents as well, such as e.g. cytokines, growth factors, hormones, small molecules, chemotherapeutics, pro-drugs, drugs, antibodies, or other various pharmaceutically-active agents. There is virtually no limit to other components that may also be included in the compositions, provided that the additional agents do not adversely affect the ability of the composition to deliver the intended therapy.

The phrase "pharmaceutically acceptable" is employed herein to refer to those compounds, materials, compositions, and/or dosage forms that are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals. Exemplary pharmaceutically acceptable carriers include, but are not limited to, sugars, such as lactose, glucose, and sucrose; starches, such as corn starch and potato starch; cellulose and its derivatives, such as sodium carboxymethyl cellulose, ethyl cellulose, and cellulose acetate; tragacanth; malt; gelatin; talc; cocoa butter, waxes, animal and vegetable fats, paraffin, silicones, bentonites, silicic acid, zinc oxide; oils, such as peanut oil, cottonseed oil, safflower oil, sesame oil, olive oil, corn oil, and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and any other compatible substances employed in pharmaceutical formulations.

In particular embodiments, compositions of the present invention comprise an amount of CAR-expressing immune effector cells contemplated herein. As used herein, the term "amount" refers to "an amount effective" or "an effective amount" of a genetically modified therapeutic cell, e.g., T cell, to achieve a beneficial or desired prophylactic or therapeutic result, including clinical results.

A "prophylactically effective amount" refers to the amount of a genetically modified therapeutic cell that effectively achieves the desired prophylactic result. Typically, but not necessarily, since a prophylactic dose is used in subjects prior to or at an earlier stage of disease, the prophylactically effective amount is less than the therapeutically effective amount. The term "prophylactic" does not necessarily refer to a complete prohibition or prevention of a particular medical disorder. The term prophylactic also refers to the reduction of risk of a certain medical disorder occurring or worsening in its symptoms.

A "therapeutically effective amount" of a genetically modified therapeutic cell may vary according to factors such as the disease state, age, sex, and weight of the individual, and the ability of the stem and progenitor cells to elicit a desired response in the individual. A therapeutically effective amount is also one in which the therapeutically beneficial effects outweigh any toxic or detrimental effects of the virus or transduced therapeutic cells. The term "therapeutically effective amount" includes an amount that is effective to "treat" a subject (e.g., a patient). When a therapeutic amount is indicated, the precise amount of the compositions of the present invention to be administered can be determined by a physician considering individual differences in age, weight, tumor size, extent of infection or metastasis, and patient condition (subject).

It can generally be stated that a pharmaceutical composition comprising the immune cells (T cells) described herein may be administered at a dosage of $10^2$ to $10^{10}$ cells/kg body weight, preferably $10^5$ to $10^6$ cells/kg body weight, including all integer values within those ranges. The number of cells will depend upon the ultimate use for which the composition is intended, as will the type of cells included therein. For uses provided herein, the cells are generally in a volume of a liter or less, 500 mLs or less, or even 250 mLs or 100 mLs or less. Hence, the density of the desired cells is typically greater than 106 cells/ml and generally greater than 107 cells/ml, generally 108 cells/ml or greater. The clinically relevant number of immune cells can be apportioned into multiple infusions that cumulatively equal or exceed $10^5$, $10^6$, $10^7$, $10^8$, $10^9$, $10^{10}$, $10^{11}$, or $10^{12}$ cells. In some aspects of the present invention, particularly since all the infused cells will be redirected to a particular target antigen, lower numbers of cells may be administered. CAR-expressing cell compositions may be administered multiple times at dosages within these ranges. The cells may be allogeneic, syngeneic, xenogeneic, or autologous to the patient undergoing therapy.

Generally, compositions comprising the cells activated and expanded, as described herein, may be utilized in treating and preventing diseases that arise in immunocompromised individuals. The CAR-modified T cells of the present invention may be administered either alone or as a pharmaceutical composition in combination with carriers, diluents, excipients, and/or with other components such as IL-2 or other cytokines or cell populations. In particular embodiments, pharmaceutical compositions contemplated herein comprise an amount of genetically modified T cells in combination with one or more pharmaceutically or physiologically acceptable carriers, diluents, or excipients.

Pharmaceutical compositions of the present invention comprising a CAR-expressing immune effector cell population, such as T cells, may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for parenteral administration, e.g., intravascular (intravenous or intraarterial), intraperitoneal, or intramuscular administration.

The liquid pharmaceutical compositions, whether they be solutions, suspensions, or other like forms, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methylparaben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes, or multiple dose vials made of glass or plastic. An injectable pharmaceutical composition is preferably sterile.

In a particular embodiment, compositions contemplated herein comprise an effective amount of CAR-expressing immune effector cells, alone or combined with one or more therapeutic

agents. Thus, the CAR-expressing immune effector cell compositions may be administered alone or in combination with other known treatments, such as other immunotherapies, etc. The compositions may also be administered in combination with antibiotics. Such therapeutic agents may be accepted in the art as a standard treatment for a particular disease state as described herein, such as a particular cancer. Exemplary therapeutic agents contemplated include cytokines, growth factors, steroids, NSAIDs, DMARDs, anti-inflammatories, chemotherapeutics, radiotherapeutics, therapeutic antibodies, or other active and ancillary agents.

*Therapeutic Methods*

As used herein, the terms "individual" and "subject" are often used interchangeably and refer to any animal that exhibits a symptom of a disease, disorder, or condition that can be treated with the gene therapy vectors, cell-based therapeutics, and methods disclosed elsewhere herein. In preferred embodiments, a subject includes any animal that exhibits symptoms of a disease, disorder, or condition of the hematopoietic system, e.g., an autoimmune disease, that can be treated with the cell-based therapeutics and methods disclosed herein. Suitable subjects include laboratory animals (such as mice, rats, rabbits, or guinea pigs), farm animals, and domestic animals or pets (such as cats or dogs). Non-human primates and, preferably, human patients are included.

As used herein, "treatment" or "treating" includes any beneficial or desirable effect on the symptoms or pathology of a disease or pathological condition and may include even minimal reductions in one or more measurable markers of the disease or condition being treated. Treatment can optionally involve either the reduction or amelioration of symptoms of the disease or condition or the delaying of the progression of the disease or condition. "Treatment" does not necessarily indicate complete eradication or cure of the disease, condition, or associated symptoms.

As used herein, "prevent" and similar words such as "prevented," "preventing," or "prophylactic," etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms, and/or burden of a disease or condition prior to the onset or recurrence of the disease or condition.

The quantity and frequency of administration will be determined by such factors as the condition of the patient, and the type and severity of the patient's disease, although appropriate dosages may be determined by clinical trials.

The administration of the compositions contemplated herein may be carried out in any convenient manner, including by aerosol inhalation, injection, ingestion, transfusion, implantation, or transplantation. In a preferred embodiment, compositions are administered parenterally. The phrases "parenteral administration" and "administered parenterally," as used herein, refer to modes of administration other than enteral and topical administration, usually by injection, and includes, without limitation, intravascular, intravenous, intramuscular, intraarterial, intrathecal, intracapsular, intraorbital, intratumoral, intracardiac, intradermal, intraperitoneal, transtracheal, subcutaneous, subcuticular, intraarticular, subcapsular, subarachnoid, intraspinal and intrasternal injection and infusion. In one embodiment, the compositions contemplated herein are administered to a subject by direct injection into a tumor, lymph node, or site of infection.

*General remarks*

All words and terms used herein shall have the same meaning commonly given to them by the person skilled in the art unless the context indicates a different meaning. All terms used in the singular shall include the plural of that term and vice versa.

It will be understood that particular embodiments described herein are shown by way of illustration and not as limitations of the invention. The principal features of this invention can be employed in various embodiments without departing from the scope of the invention. Those skilled in the art will recognize or be able to ascertain, using most routine study, numerous equivalents to the specific procedures described herein. Such equivalents are considered to be within the scope of this invention and are covered by the claims. All publications and patent applications mentioned in the specification indicate the skill level of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The term "or" in the claims is used to mean "and/or" unless explicitly indicated to refer to alternatives only or the alternatives are mutually exclusive. However, the disclosure supports a definition of only alternatives and "and/or." Throughout this application, where relevant, the term "about" indicates that a value includes the inherent variation of error for the device, the method employed to determine the value or the variation among the study subjects.

**FIGURES**

The invention is demonstrated by way of example in the following figures. The figures are to provide a further description of potentially preferred embodiments that enhance the support of one or more non-limiting embodiments of the invention.

*Brief description of the figures:*

**Figure 1:** Constructs and methods for production of CD30CAR-T cells via CAR knock-in in the TRAC locus and upregulation of C30CAR expression during *in vitro* expansion

**Figure 2:** Frequencies of CD30CAR[+]-T cells engineered with scFvs from humanized 5F11 or 2H9 mAbs increase upon in vitro culture, show a naive phenotype and do not overexpress check-point receptors.

**Figure 3:** 5F11-CD30CAR-Fclg-T[pos]CD19[neg] Hodgkin lymphoma cells while CD19CAR-T cells do not show allograft reactivity.

**Figure 4:** HRS3-CD30CAR, 5F11-CD30CAR and 2H9-CD30CAR-T cells kill CD30[pos] cells infected with EBV

**Figure 5:** 5F11-CD30CAR-Fclg-T cells kill ACH2 CD30[pos] cells infected with HIV

**Figure 6:** 5F11-CD30CAR-tCD34-T cells maintain cytotoxic function after repetitive antigen exposure with CD30[pos] EBV[pos] L591 cell targets.

*Detailed description of the figures:*

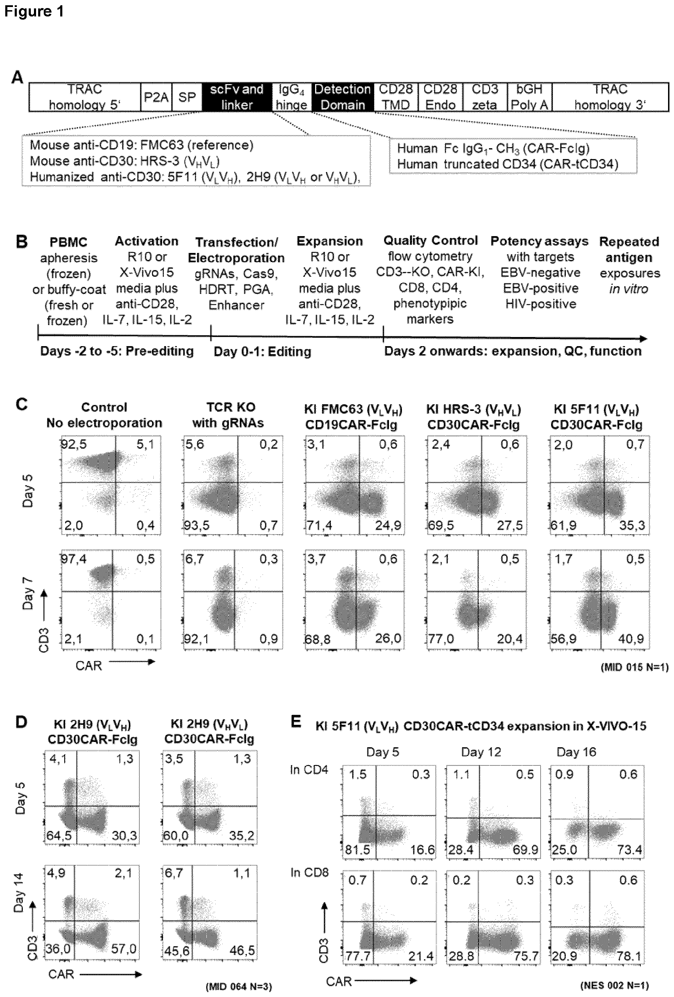

**Figure 1: Constructs and methods for production of CD30CAR-T cells via CAR knock-in in the TRAC locus and upregulation of C30CAR expression during *in vitro* expansion.** A: Schematic representation of exemplary constructs. Homology-directed DNA repair template (HDRT) containing a T cell receptor alpha constant 5' homology arm (5' *TRAC* homology), a P2A element for translational initiation, a signal peptide for driving CAR expression to the cell membrane (SP), a single-chain variable fragment (scFv) for targeting the CAR against CD19 (FMC63 mouse mAb) or CD30 (mouse mAb: VH-VL HRS-3; humanized mAbs: VL-VH 5F11, VL-VH 2H9 or VH-VL 2H9), a glycine-serine linker (GS), a hinge domain (IgG4), a detection domain (IgG1-CH3 / FCIG or truncated CD34/tCD34), a transmembrane domain (CD28 TMD), a endocytoplasmatic domain (CD28 Endo), a signaling domain (CD3 zeta), a RNA polyadenylation signal sequence (bovine growth hormone/ bGH PolyA), and a T cell receptor alpha constant 5' homology arm (3' *TRAC* homology). B: Schematic representation of the gene editing methods. Peripheral blood mononuclear cells (PBMCs) isolated after gradient centrifugation from apheresis products or blood were pre-cultured for 1-3 days in media (RPMI 10% fetal calf serum/R10 orX-VIVO-15) with cytokines (IL-7 plus IL-15 or IL-2) and activated for 2 days (anti-CD28, IL-7 plus IL-15). On day 0, cells were transfected (electroporation) with Cas9 protein, gRNAs, HDRTs in the presence of adjuvants, on day 1 washed and re-seeded. Cells were expanded in media with cytokines and, at different time points, collected for TCR knock-out and CAR knock-in analyses via flow cytometry. After expansion, cells were tested for cytotoxic potency *in vitro.* C: Exemplary data showing that expression of CD30CAR engineered with humanized VL-VH derived from the mAb 5F11 and containing the Fclg detection domain increases upon time (in R10 medium plus IL-7 and IL-15). Flow cytometric analyses showing CD3/ TCR expression on y-axis and CAR detection on x-axis. Control cells not electroporated, cells transfected with Cas9, gRNAs and adjuvants (but no HDRTs) were used as references. Analyses performed at days 5 and 7 after editing show >95% CD3 knock-out and that expression of CAR maintained stable or decreased for FMC3-CD19KICAR-T and HRS3-CD30KICAR-T cells respectively. CAR expression increased from day 5 to day 7 for 5F11-CD30KICAR-T cells. Data obtained from a single PBMC donor, experiment MID 015. D: Exemplary data showing that expression of CD30CAR engineered with humanized VL-VH or VH-VL derived from the 2H9 mAb and containing the Fclg detection domain increases upon time (in R10 medium plus IL-7 and IL-15). Flow cytometric analyses showing CD3/TCR expression on y-axis and CAR detection on x-axis. Analyses performed at days 5 and 14 after editing show >95% CD3 knock-out and that expression of CARs increased from day 5 to day 14. Representative data from three PBMC donors, experiment MID 064. E: Exemplary data showing that expression of CD30CAR engineered with humanized VL-VH derived from the mAb 5F11 and containing the tCD34 detection domain increases upon time (in serum free X-VIVO-15 medium plus IL-7 and IL-15). Flow cytometric analyses showing gated CD4$^+$ or CD8$^+$ T cell populations and CD3/TCR expression on y-axis and CAR detection on x-axis. Analyses performed after editing show that expression of CD30CAR increased continuously from days 5 and 12 to day 16. Data obtained from a single PBMC donor, experiment NES 002.

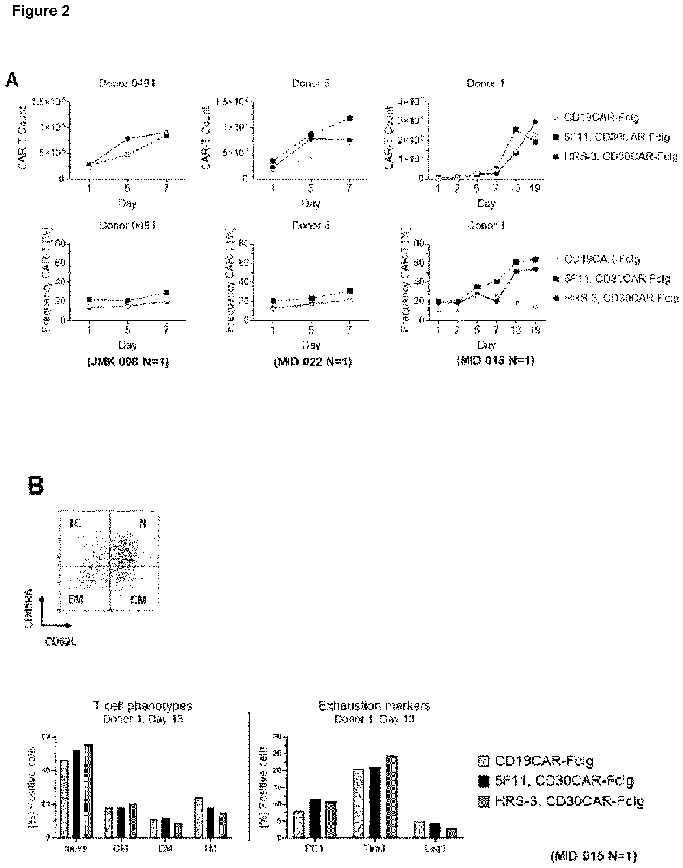

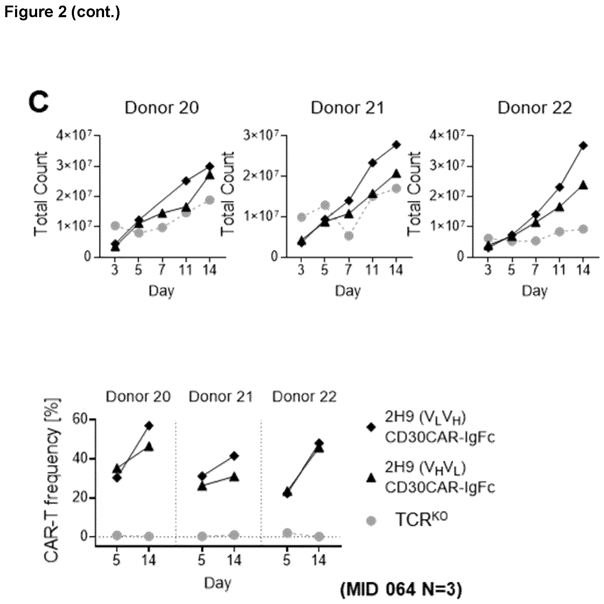

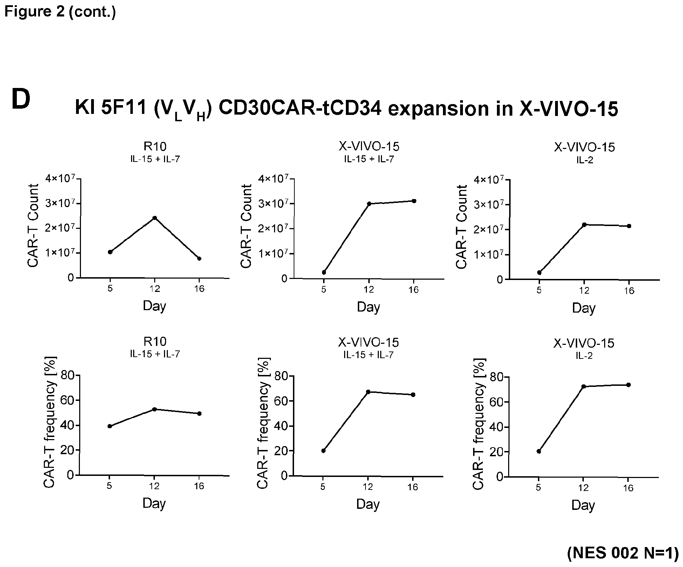

**Figure 2: Frequencies of CD30CAR$^+$-T cells engineered with scFvs from humanized 5F11 or 2H9 mAbs increase upon in vitro culture, show a naive phenotype and do not overexpress check-point receptors.** A: Edited CD30CAR-Fclg-T cells with different scFvs (5F11, HRS-3) generated with PBMCs of 3 different donors (0481, 5 and 1) show continuous *in vitro* expansion and no signs of fratricide effects. Upper panels show absolute viable CAR$^+$ T cell counts and lower panels show frequencies of CAR$^+$ cells in whole cell population. Cells were expanded up to 19 days. Edited CD19CAR-T cells were included as reference. Data obtained from three PBMC donors, experiments JMK 008, MIF 002 and MID 015. B: Edited 5F11-CD30CAR-Fclg-T cells show naive and non-exhausted phenotype after *in vitro* expansion for 13 days. Panel on the left shows the flow cytometry gating using CD62L (x-axis) and CD45RA (y-axis) as markers for identification of naive (N), central memory (CM), effector memory (EM), terminal effector (TE) cells. Bar graph in the middle showing quantified frequencies of cells with each phenotype within different CAR-T cell types. Bar graph on the right side showing quantified frequencies of cells stained for detection of check-point receptors (PD1, TIM3 and LAG3). Data obtained from one PBMC donor, experiment MID 015. C: Edited 2H9-CD30CAR-Fclg-T cells continuously expand *in vitro.* CD30CAR-T cells generated with PBMCs of 3 different donors (20, 21, 22) were compared. PBMCs electroporated with RNP but no HDRT are shown as references. Graphs on the left show the absolute CAR$^+$ T cell count. Graphs on the right show the CAR$^+$ frequencies within the total cells. Data obtained from three PBMC donors, experiment MID 064. D: Edited 5F11-CD30CAR-tCD34-T cells continuously expand *in vitro* in serum-free X-VIVO-15 medium. CD30CAR-tCD34 T cells were cultured after electroporation in different media (R10 +IL-7 + IL-15 / X-Vivo-15 + IL-7 + IL-15 / X-VIVO-15 + IL-2) for 16 days. Upper graphs show the absolute CAR$^+$ T cell count. Lower graphs show the CAR$^+$ frequencies within the total cells. Data obtained from one PBMC donor, experiment NES 002).

**Figure 3: 5F11-CD30CAR-Fclg-T and 5F11-CD30CAR-tCD34-T cells kill** CD30$^{pos}$CD19$^{neg}$ **Hodgkin lymphoma cells while CD19CAR-T cells do not show allograft reactivity.** A: CD30$^{pos}$CD19$^{neg}$ Hodgkin lymphoma cell lines (L-540 and KM-H2) used for the studies. Parental cell lines were transduced with a lentiviral vector co-expressing firefly-luciferase and GFP. After sorting the GFP$^{pos}$ populations, GFP$^{pos}$ clonal cell lines were selected. Flow cytometry plots on the left show the parental lines and on the right the gene-marked lines regarding expression of GFP (X-axis) and CD30 (Y-axis). B: Dose-dependent killing of CD30$^+$ targets by 5F11-CD30CAR-Fclg-T cells. L540 and KM-H2 target cells (10$^4$) clonal cell lines were used for bioluminescence-based cytotoxic assays. Targets were co-cultured with T cells (TCR$^{KO}$, CD19CAR-Fclg$^{KI}$ or 5F11CD30CAR-Fclg$^{KI}$) for 72 h at Effector:Target ratios of 3:1, 1:1, 0,3:1 in 96-well U-bottom plates. After incubation, luciferin substrate was added and pellets were used for bioluminescence detection (counts/s). Non-treated target only and Triton-X treated target cells were used as references for the assay. C: Dose-dependent killing of CD30$^+$ targets by 5F11-CD30CAR-tCD34-T cells. L540 and KM-H2 target cells (10$^4$) clonal cell lines were used for bioluminescence-based cytotoxic assays. Targets were co-cultured with T cells (TCR$^{KO}$, CD19CAR-tCD34$^{KI}$ or 5F11CD30CAR-tCD34$^{KI}$) for 72 h at Effector:Target ratios of 3:1, 1:1, 0,3:1 in 96-well U-bottom plates. After incubation, luciferin substrate was added and pellets were used for bioluminescence detection (counts/s). Non-treated target only and Triton-X treated target cells were used as references for the assay.

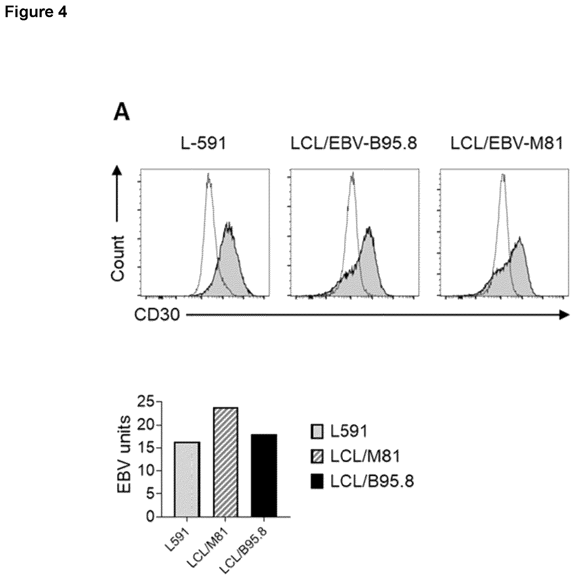

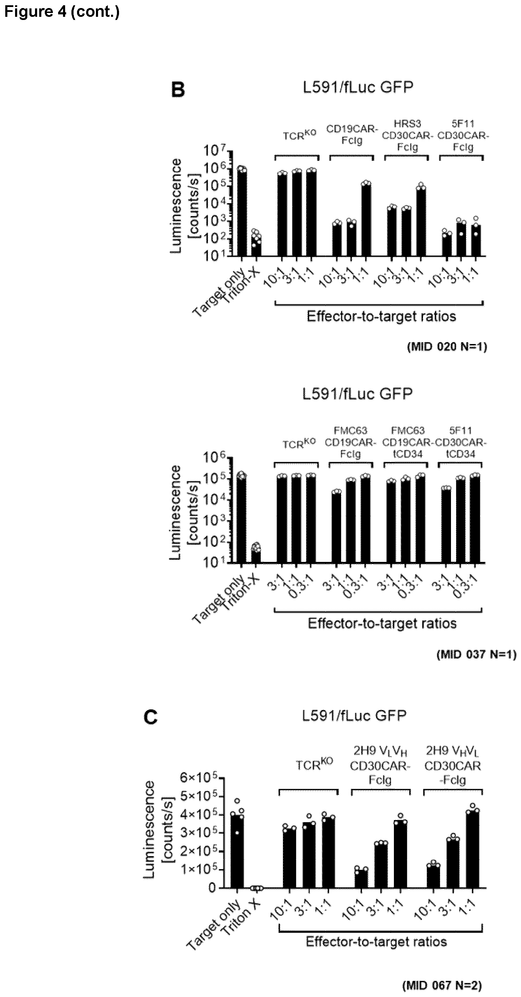

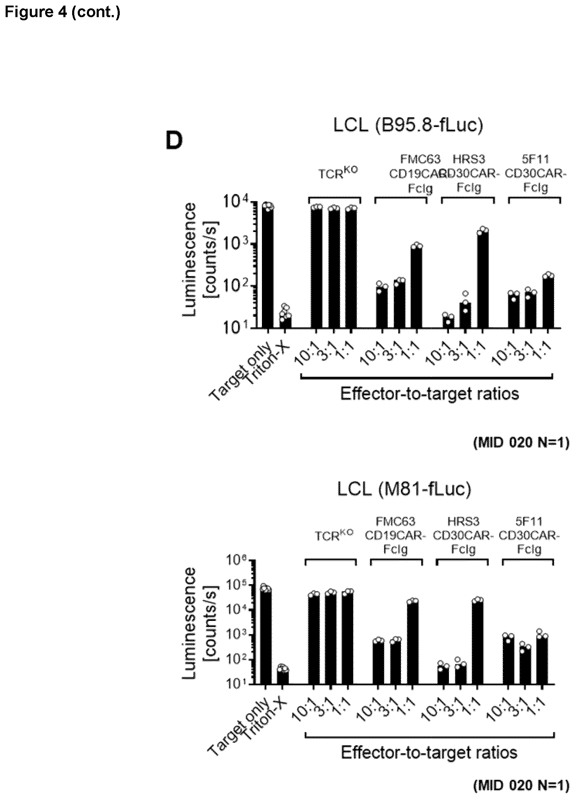

**Figure 4: HRS3-CD30CAR, 5F11-CD30CAR and 2H9-CD30CAR-T cells kill CD30$^{pos}$ cells infected with EBV.** A: Detection of EBV in cell types and CD30 expression. The CD30$^{pos}$CD19$^{pos}$ L591 lymphoma cell line and lymphoblastoid cell lines (LCLs) generated after infection of primary B cells with B95.8/fLuc-GFP or M81/fLuc-GFP EBV strains. Left panels: Flow cytometric analysis of L-591, LCL/B95.8 and LCL/M81 cells showing CD30 expression (staining with specific antibody is shown in grey and isotype-control in unfilled graph). Graph on the right: DNA was extracted from cell lines and EBV viral copies were detected via quantitative PCR. B: Dose-dependent killing of L591 EBV$^+$ targets by HRS3-CD30CAR-T and 5F11-CD30CAR-T cells. L591 cells transduced with a lentiviral vector co-expressing firefly-luciferase (10$^4$ cells) were co-cultured with T cells at different E:T ratios (10:1, 3:1, 1:1 or 3:1, 1:1,0,3:1) in 96-well U-bottom plates for 72 h. TCR$^{KO}$, CD19CAR-Fclg, CD19CAR-tCD34 T cells were used as references and HRS3-CD30CAR-Fclg, 5F11-CD30CAR-Fclg, 5F11-CD30CAR-tCD34 effectors were compared. After incubation, luciferin substrate was added and pellets were used for bioluminescence detection (counts/s). Non-treated target only and Triton-X treated target cells were used as references for the assay. D: Dose-dependent killing of L591 EBV$^+$ targets by 2H9(V$_H$-V$^L$)-CD30CAR-T and 2H9(V$_H$-V$^L$)-CD30CAR-T cells. L591/fluc-GFP cells (10$^4$ cells) were co-cultured with T cells (10:1, 3:1, 1:1) in 96-well U-bottom plates for 72 h. TCR$^{KO}$ T cells were used as references and 2H9(V$_H$-V$^L$)-CD30CAR-T and 2H9(V$_H$-V$_L$)-CD30CAR-T cells were compared. After incubation, luciferin substrate was added and pellets were used for bioluminescence detection (counts/s). Non-treated target only and Triton-X treated target cells were used as references for the assay. C: Superior killing of LCL EBV$^+$ targets by 5F11-CD30CAR-Fclg-T cells at E:T ratio of 1:1. LCLs co-expressing firefly-luciferase (10$^4$ cells) were co-cultured with T cells 10:1, 3:1, 1:1) in 96-well U-bottom plates for 72 h. CD19CAR-Fclg, HRS3-CD30CAR-Fclg and 5F11-CD30CAR-Fclg effectors were compared. After incubation, luciferin substrate was added and pellets were used for bioluminescence detection (counts/s). Non-treated target only and Triton-X treated target cells were used as references for the assay.

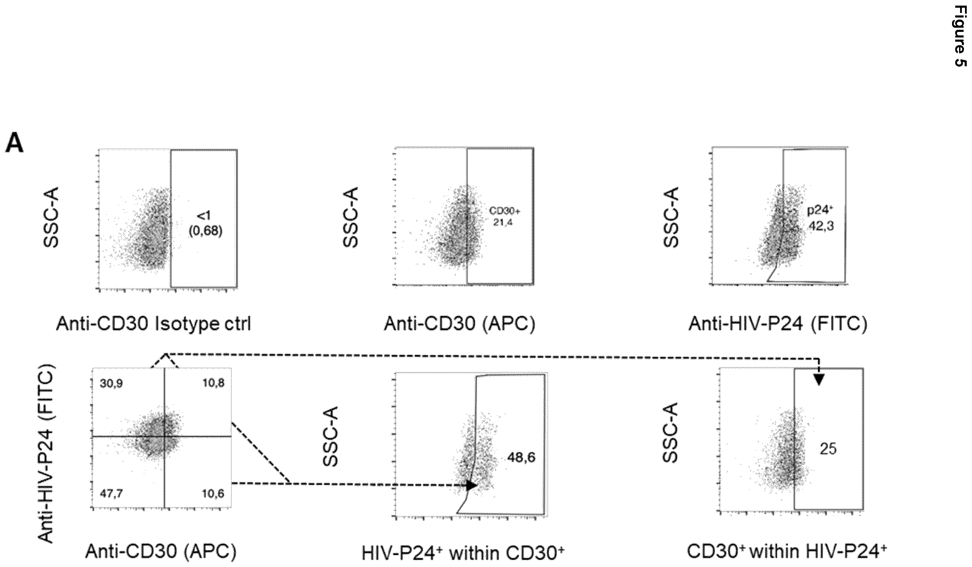

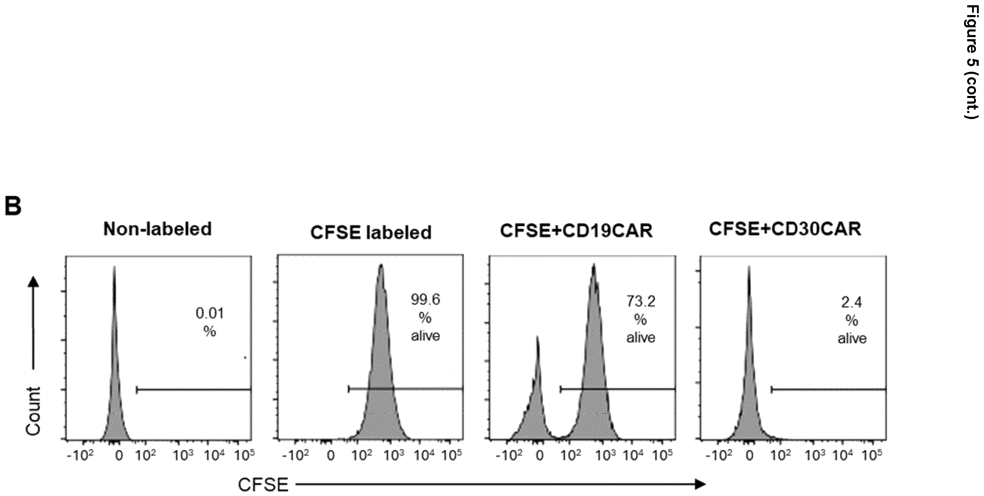

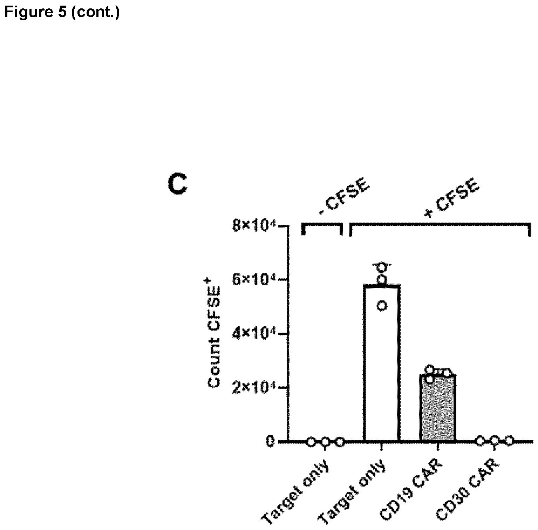

**Figure 5: 5F11-CD30CAR-Fclg-T cells kill ACH2 CD30$^{pos}$ cells infected with HIV.** A: Detection of HIV-p24 and CD30 expression in ACH2 cells. Flow cytometry analyses showing staining of ACH2 cells with an isotype control antibody (upper left panel), with an anti-CD30 antibody (upper middle panel) or with an anti-HIV-p24 antibody (upper right panel). The lower panels show that both HIV-p24 and CD30 can be detected in sub-populations of cells. B: Raw data depicting 1×10$^5$ CFSE-labeled ACH2 target cells were co-cultured with CD19CAR-Fclg or with 5F11-CD30CAR-Fclg-T cells at effector to target ratio of 1:1 for 72 h. Non-labeled ACH-2 cells or ACH-2 cells in the absence of effectors were cultivated in parallel Depletion of CFSE-positive events was determined via flow cytometry, indicating cytotoxicity. C: Quantified CFSE-events measured for each ACH2 cell culture condition. Data indicate efficient killing by 5F11-CD30CAR-Fclg-T cells at effector to target ratio of 1:1 for 72 h.

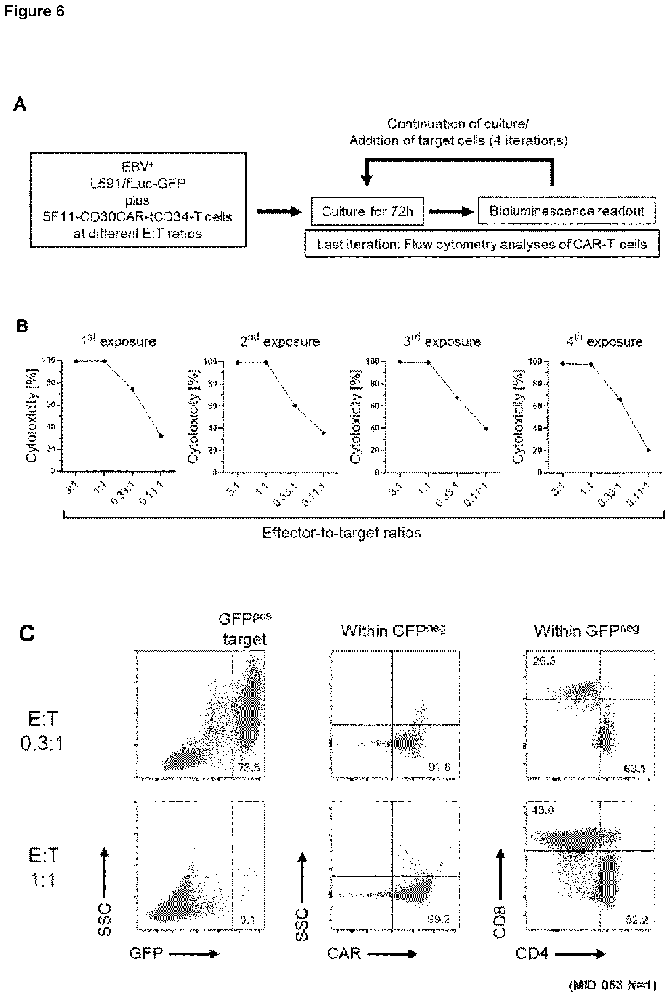

**Figure 6: 5F11-CD30CAR-tCD34-T cells maintain cytotoxic function after repetitive antigen exposure with CD30$^{pos}$ EBV$^{pos}$ L591 cell targets.** A: Scheme of repeated antigen exposure assays. 2×10$^5$ CAR-T cells were incubated with L591/fLuc-GFP target cells at different E:T ratios for 72 h. The process was repeated for a total of four iterations. After each iteration, the depletion of target cells was determined by detection of bioluminescence and the cytotoxicity was calculated. The co-culture was either continued with remaining target cells or target cells were replenished. Bioluminescence was measured 72 h after each exposure for 4 iterations. At the last iteration, effectors were analyzed by flow cytometry. B: 5F11-CD30CAR-tCD34-T cells maintain killing capacity after antigen re-exposure. Cytotoxicity determined after each iteration of coculture of CAR-T cells and L591/fLuc-GFP target cells at different E:T ratios. C: Persistent killing after antigen re-exposure is associated with enrichment of CAR$^+$ T cells. Flow cytometric analyses performed after the last iteration. Left panels: GFP$^{pos}$ target cells were excluded for analyses of CAR-T cells. Middle panels: Identification of CAR expression within GFP$^{neg}$ subpolulation, showing >95% CAR$^+$ cells. Right panels: Relative frequencies of CD4$^{pos}$ and CD8 $^{pos}$ cells within CAR$^{pos}$ population for iterations with E:T of 0.3:1 and 1:1.

**EXAMPLES**

The invention is demonstrated by way of the examples disclosed below. The examples provide technical support for and a more detailed description of potentially preferred, non-limiting embodiments of the invention.

More detailed information on the materials and methods can be found in a previous publication describing the generation and characterization of the reference CAR construct TCR$^{KO}$CD19(FMC63)CAR$^{KI}$-T cells (Braun et al, Frontiers in Immunology, 24 Mar 2023, 14:1086433).

***Summary of the Examples***

In order to demonstrate the functionality and beneficial properties of the genetically modified cytotoxic cell described herein, the following examples are to be considered:

- Generation of CD30CAR HDRTs using scFv sequences from >3 different mAbs

- Production of gene edited TCR$^{KO}$CD30CAR$^{KI}$-T cells via electroporation of HDRTs, Cas9 and gRNAs

- Assessment of CAR$^+$ T cell frequency by FACS

- Assessment of the TCR$^{KO}$CD30CAR$^{KI}$-T cells phenotype by FACS

- Assessment of CD30 positivity of cells infected with EBV or HIV

- Assessment of TCR$^{KO}$CD30CAR$^{KI}$-T cell cytotoxicity by against CD30$^+$ targets by bioluminescence

- Assessment of TCR$^{KO}$CD30CAR$^{KI}$-T cell cytotoxicity against CD30$^+$ targets by FACS

- Evaluation of the potency of TCR$^{KO}$CD30CAR$^{KI}$-T cells against CD30$^+$ cells infected with EBV in vivo in xenograft or humanized mouse models

- Evaluation of TCR$^{KO}$CD30CAR$^{KI}$-T cells against CD30$^+$ cells infected with HIV in vivo in xenograft or humanized mouse models

- Production of gene edited HLA$^{KO}$TCR$^{KO}$CD30CAR$^{KI}$-T for allogeneic uses

- Statistical analyses comparing the different CAR-T cells generated with different donors.

### Example 1: Design, amplification, and purification of HDRTs

The CD30CAR HDRT sequences are designed to incorporate the HRS3, 5F11 or 2H9 scFv sequences targeting CD30 and other elements of the CAR (Figure 1A). Different constructs are tested in VH-VL or VL-VH orientations. The plasmids containing the HDRT sequences are synthesized (Twist Bioscience) and are amplified from the plasmids by PCR using Herculase II Fusion DNA Polymerase (Agilent) using primers homologous to the extremities of the TRAC homology arms (forward 5'-ataaaagaataagcagtattattaagtagccctgc-3'; reverse 5'-atctgcttttttcccgtgtcattct-3'). For amplification, the samples are placed in a PCR cycler and heated to 95°C for 2 min for initial denaturation; this is followed by 30 cycles of 20 s at 95°C, 20 s at 59°C, and 90 s at 72°C. A final step of extension at 72°C for 3 min is performed. DNA purification is performed with DNA-binding magnetic beads (AMPure XP), 10 min incubation at RT, and the tube is placed on a DynaMag™-2 magnet (Thermo Fisher Scientific) for 10 min at RT. After washing steps, the pellet is air-dried 10 min and resuspended in nuclease-free water. The concentrations of the HDRTs are measured using a Nanophotometer® (Implen). The DNAs are stored at -20°C until use.

### Example 2: Production of CAR-T cells via electroporation

Frozen or fresh PBMC (apheresis product or blood products, obtained according to institutional ethics protocols) are prepared by gradient centrifugation and incubated at routine conditions (incubator 37°C, 5% CO$_2$) in RPMI 1640 (Gibco), supplemented with 10% FCS (Gibco), 1% Penicillin/Streptomycin (Gibco) (heretofore R10 medium). 5 ng/ml recombinant human IL-15 (Miltenyi Biotec) and 5 ng/ml recombinant IL-7 (Miltenyi Biotec) are added for homeostatic activation of the T cells at a concentration of $2\times10^6$ cells/mL. Following an incubation period of 2-5 days, cells are harvested, counted and resuspended at $2\times10^6$ cells/mL in R10. Cells are activated for 48 h with 5 ng/mL IL-15, 5 ng/mL IL-7 and anti-CD3/anti-CD28 activation beads (Transact 1:100 dilution Miltenyi Biotec) and kept in culture for 48h. On the day of electroporation (Day 0), cells are counted and resuspended at $100\times10^6$ cells/mL of serum-free medium (TexMACS, Miltenyi Biotec). For the integration of CAR into the T cells via gene-editing, an RNP mix with a defined ratio of the following components is mixed: (A) crRNA targeting the TRAC locus (IDT), (B) tracrRNA for Cas9 (IDT), (C) Cas9 endonuclease (IDT), (D) PGA. Prior to the addition of Cas9 and PGA, crRNA and tracrRNA are heated for 5 min at 95°C to enable annealing of heteroduplex crRNA : tracrRNA. If a KI of CAR is aimed for, HDRT-bearing homology arms for TRAC encoding for the CAR are added to the RNP mix. 50 μL of previously prepared cell suspension is added to the prepared RNP mix, resuspended, and directly added into the electroporation chamber of a small size R-50x8 cuvette or mid or large size cuvettes (MaxCyte). Electroporation of the cell suspension within the small size or mid-size cuvettes is performed using the electroporation device ATx (MaxCyte) using the program '14-3' (resting T cells), as provided by the manufacturer. Cells are subsequently transferred to a 96-well U-bottom plate for recovery in TexMACS supplemented with V2 enhancer (IDT, final concentration 1 μM) and incubated for 30 min at 37°C, 5% CO2. Following this, the cell suspension is transferred in its entirety to 2.5 mL TexMACS in a 6 well plate and incubated for 2 h at 37°C, 5% CO2. Cells are then given 2.5 mL TexMACS supplemented with IL-15 and IL-17 for a final concentration of 5 ng/mL for each cytokine and incubated at 37°C, 5% CO2.

After 24 h, cells are counted, harvested, and resuspended at $1 \times 10^6$ cells/mL in R10 or in X-VIVO-15 medium (Lonza) and 5 ng/mL IL-15 and IL-7 each. From this day onward, cells are maintained and expanded within this media with additional 5 ng/mL IL-15 and IL-7 each. Viable cell counts and FACS analyses are at several time-points to monitor viability, expansion, frequency of CD3/TCR-KO, frequency of CAR-KI, and frequency of CD8$^+$ and CD4$^+$ cells within CAR$^+$ cells. A scheme of the generation of CAR-T cells is shown in **Figure 1B.**

### Example 3: Assessment of CAR frequency by FACS

Two staining methods are performed sequentially to assess the frequencies of CAR, CD3, CD4, and CD8 positive cells. First, $10^5$ cells are washed with 150 $\mu$L PBS and subsequently blocked with 10 $\mu$g/mL murine IgG for 20 min on ice. Following this, cells are washed with 150 $\mu$L PBS and then stained with anti-CAR antibody diluted in PBS. Depending on the chosen CAR design, fluorescently labeled polyclonal anti-human IgG antibody (against FcIg detection domain) or monoclonal anti-CD34 antibody (against tCD34 detection domain) is used and incubated on the cells for 30 min on ice in the dark. After this first staining, cells are washed with 150 $\mu$L PBS and then incubated with PBS containing a viability dye and fluorescently labeled antibodies detecting human CD3, CD4, and CD8 for 30 min on ice. After washing with 150 $\mu$L PBS, cells are resuspended in 100 $\mu$L FACS buffer, and samples are measured by flow cytometry (for example CytoFLEX device). For gating, viable cells are gated through FSC/SSC, singlets and viability dye, and then CD3$^+$ and CAR$^+$ cells are detected within all cells or within CD4$^+$ or CD8$^+$ cells. Examples of flow cytometry analyses to detect TCR$^{KO}$CD30CAR$^{KI}$-T cells are illustrated in **Figures 1 C-E**. Examples of monitoring the absolute CAR$^+$-T cell count or the frequencies of the CAR$^+$-T cells within all the electroporated cells are show in in **Figures 2A, 2C and 2D.**

### Example 4: Assessment of the CAR-T immunophenotype and expression of check-point receptors by FACS

Two staining methods are performed sequentially for the assessment of the CAR-T cell phenotype. First, $2 \times 10^5$ cells are washed with 150 $\mu$L PBS and subsequently blocked with 10 $\mu$g/mL murine IgG for 20 min on ice. Following this, cells are washed with 150 $\mu$L PBS and then stained with the respective anti-CAR antibody diluted in PBS for 30 min on ice in the dark. After this first staining, cells are washed with 150 $\mu$L PBS and then incubated with PBS containing a viability dye, fluorescently labeled antibodies detecting human CD3, CD4, CD8, CD62L, and CD45RA for 30 min on ice. After washing with 150 $\mu$L PBS, cells are resuspended in 100 $\mu$L FACS buffer, and samples are measured using flow cytometry. Detected CAR-T cell populations are divided, based on the expression of CD62L and CD45RA, into Naive (CD62L$^+$, CD45RA$^+$), CM (CD62L$^+$, CD45RA$^-$), EM (CD62L$^-$, CD45RA$^-$), TE (CD62L$^-$, CD45RA$^+$). The gating strategy is illustrated in Figure 2B. For the detection of the check-point receptors PD1, Tim3 and Lag3, after staining of the CAR, the cells are similarly washed and then incubated with PBS containing a viability dye, fluorescently labeled antibodies detecting human PD1, Tim3 and Lag3 for 30 min on ice.

### Example 5: Assessment of CD30 positivity in cell lines infected with EBV or HIV

To assess CD30 positivity, $10^5$ cells are washed with 150 $\mu$L PBS and incubated with 10 $\mu$g/mL murine IgG for 20 min at 4°C. Subsequently, cells are washed with 150 $\mu$L PBS and incubated either with fluorescently labeled anti-CD30 or corresponding isotype control diluted in PBS for 30 min at 4°C in the dark. Cells are then washed with 150 $\mu$L PBS, resuspended in 100 $\mu$L FACS buffer, and measured via flow cytometry. Analyses of CD30 expression are illustrated in **Figures 3A, 4A, 5A.**

### Example 6: Quantification of EBV copies in cells

DNA isolation of cells to determine the EBV load is performed with a QIAamp DNA Blood Mini Kit (Qiagen) ánd DNA concentration and purity were measured using the Nanodrop instrument. The DNA samples are resuspended and approximately 1,5 $\mu$g was used per reaction. The EBV DNA detection by real-time PCR was performed with the EBV *in vitro* diagnostics PCR kit (GeneProof®) after amplification of a specific conserved DNA sequence of the single-copy gene encoding the nuclear antigen 1 (EBNA1) and measurement of fluorescence emission. An internal standard (IS) was included in the reaction mix as a control for the PCR reaction. The analyses were performed using a LightCycler 2.0/480 instrument. The detection limit of the assay was approximately $1.9 \times 10^5$ EBV units in the sample. Analyses of EBV copies are illustrated in **Figure 4A.**

### Example 7: Assessment of CD30CAR-T cell cytotoxicity by bioluminescence

The assessment of cytotoxicity of CD30CAR-T cells is determined in co-cultures with CD30$^+$ target cells expressing firefly luciferase (fLuc). Target cells are lentivirally transduced to express fLuc and GFP or infected with EBV viral strains expressing fLuc. Target cells are seeded in technical triplicates at $10^4$ cells/well in a 96-well U-bottom plate. Target cells are then incubated at various E:T ratios together with $CD30^+$ CAR-T cells or in the absence thereof in a final volume of R10 and 5 ng/mL of IL-7 and IL-15 for 72 h at 37°C, 5% $CO_2$, in a final volume of 100 $\mu$L. Following this, one triplicate of "target cells only' is given 5 $\mu$L of a 1% solution of Triton X-100 for 15 min to induce cell death (=100% death control). Subsequently, cells are washed twice with 150 $\mu$L PBS before the remaining cells are resuspended in 50 $\mu$L PBS and transferred to a small white area F-bottom 96-well plate. The plate is then placed in a multimode plate reader, suitable for luminescent detection (SPARK, Tecan). A fresh solution of D-Luciferin in PBS with 10 mg/mL is prepared. If applicable, a built-in injector system is used to distribute 50 $\mu$l of prepared D-Luciferin solution to each sample well of the 96-well plate. After 5 minutes of rest, the luminescence is measured to ensure a homogenous luminescence signal. Using the mean luminescence of each triplicate, the cytotoxicity at a given E:T ratio is calculated according to the following formula below. If the calculated cytotoxicity is <0%, it is stated as 0%. Examples are illustrated in **Figures 3B-C, 4B-D**. As demonstrated in Table 3, the cytotoxic cells comprising the 5F11-CD30CAR-Fclg-T cells were able to kill EBV-infected lymphoblastic cell lines, even at low effector-to-target ratio.

$$Cytotoxicity\ [\%] = \left( 1 - \frac{L_{Target+Effector} - L_{Triton-X}}{L_{Target\ only} - L_{Triton-X}} \right) * 100$$

### Example 8: Detection of HIV p24 in cells

The HIV p24 antigen is the most abundant HIV protein and is essential for assembly of the capsid that encases HIV genetic material. Detection of the HIV p24 antigen is used clinically to diagnose HIV infection. To detect the presence of CD30 and p24 on ACH2 cells, the cells were labeled in two consecutive staining steps. First, $10^5$ cells are washed with 150 mL PBS and subsequently blocked with 10 $\mu$g/mL murine IgG for 20 min on ice. Following this, cells are washed with 150 $\mu$L PBS and then stained with APC anti-human CD30 antibody diluted in PBS or with APC Mouse IgG1, $\kappa$ Isotype control antibody for 30 min on ice in the dark. After this first staining, cells are washed with PBS and then fixed in 4% PFA in PBC for 90 minutes (400 $\mu$L PBS with 4% PFA). 2% FBS (Fetal bovine serum) in PBS was added to the samples for washing. In the second wash, 2% FBS/PBS was supplemented with 0,1% Triton X-100 to permeabilize cells. 50 $\mu$L of this buffer was added to cell pellets that were left on ice for 20 minutes. 50 $\mu$L of 2% FBS/PBS containing a 1:100 dilution of the p24 antibody (HIV-1 core antigen-FITC, KC57, Beckman Coulter) was then added to the samples (both control and CD30 stained) and incubated for 30 minutes on ice in the dark. After washing with 150 $\mu$L of 2%FBS/PBS, cells are resuspended in 100 $\mu$L of the same buffer and samples measured using flow cytometry. **Figures 5A.**

### Example 9: Assessment of CD30CAR-T cell cytotoxicity against HIV-infected cells by FACS

ACH2 were labeled with 1,25 $\mu$M CellTraceTM CFSE (ThermoFisher) or left unlabeled. Labeled target cells were then incubated in technical triplicates with $10^5$ cells/well in the presence or absence of CAR-T cells at an E:T ratio of 1:1 in a 96 U-bottom plate for 48 h at 37°C in R10 and 5 ng/mL of IL-7 and IL-15. Subsequently, cells were washed, stained with a viability dye, and fixated with 4% paraformaldehyde prior to measurement via flow cytometry. Each well was measured with a fixed volume to enable comparability in the assessment of the depletion of targets ($CFSE^+$ events). Example is illustrated in **Figures 5B.**

### Example 10: Statistical analyses comparing the different CAR-T cells generated with different donors.

As demonstrated below, the cytotoxic cells comprising the CD30-CAR were able to deplete EBV-infected lymphoblastic cell lines, even at low effector-to-target ratios (Table 3).

**Table 3.** CAR-T cell cytotoxicity at 1:1 effector-to-target ratios

| | CD30-CAR (5F11) vs. CD19-CAR | | | CD30-CAR (5F11) vs. CD30-CAR (HRS-3) | | | CD19-CAR vs. CD30-CAR (HRS-3) | | |
|---|---|---|---|---|---|---|---|---|---|
| Cell line | **L591** | **LCL_2** | **LCL_3** | **L591** | **LCL_2** | **LCL_3** | **L591** | **LCL_2** | **LCL_3** |
| Donor 1 | 0,0366 (*) | 0,5474 (ns) | 0,0008 (***) | 0,3654 (ns) | 0,0008 (***) | 0,0002 (***) | 0,8762 (ns) | 0,0892 (ns) | 0,9978 (ns) |
| Donor 0481 | 0,9277 (ns) | n.t. | n.t. | 0,28 (ns) | n.t. | n.t. | 0,0943 (ns) | n.t. | n.t. |

(continued)

| Cell line | CD30-CAR (5F11) vs. CD19-CAR | | | CD30-CAR (5F11) vs. CD30-CAR (HRS-3) | | | CD19-CAR vs. CD30-CAR (HRS-3) | | |
|---|---|---|---|---|---|---|---|---|---|
| | L591 | LCL_2 | LCL_3 | L591 | LCL_2 | LCL_3 | L591 | LCL_2 | LCL_3 |
| Donor 5 | 0,1419 (ns) | n.t. | n.t. | 0,7928 (ns) | n.t. | n.t. | 0,0206 (*) | n.t. | n.t. |
| Turkey's multiple comparisons test, significance level: 0,05. n.t. = not tested | | | | | | | | | |

### Example 11: Evaluation of the potency of TCR$^{KO}$CD30-CAR$^{KI}$-T cells against primary human CD30$^+$ from HIV patients and patients with autoimmune diseases

The CD30-CAR HDRT sequence with *CD3ζ* homology arms was designed in the Stripecke laboratory, testing several different anti-CD30 antibody sequences. The HDRTs were synthesized by Twist Bioscience, amplified by PCR, and purified with DNA-binding magnetic beads. CRISPR/Cas9 (Alt-R S.p Cas9 Nuclease V3, IDT), crRNA and tracrRNA (IDT, Newark, New Jersey), Poly-L-glutamic acid (PGA) (Sigma Aldrich), and HDRTs were complexed and used to electroporate activated T cells using a MaxCyte ATx™ Flow Electroporation® device. After editing, the cells were expanded for 14 days in the presence of IL-15 and IL-12. At harvest, cells were analyzed for viability (viable cell counts and Annexin-V staining) and identity (CD3-KO; acquired CAR knock-in, expression of CD8, CD4) via flow cytometry using optimized antibody panels. CAR-T cells showed killing effects *in vitro* after co-culture assays with CD30$^{pos}$ lymphoma lines co-expressing fLuc (bioluminescence) or cells labeled with CSFE (flow cytometry). For this project, we use the cryopreserved TCR$^{KO}$CD30-CAR$^{KI}$-T cells and established methods to evaluate the cytotoxicity against primary PBMC samples obtained from HIV patients, HIV/lymphoma patients, and patients with autoimmunity to evaluate if the frequencies of CD30$^+$ cells and expression of CD30 can be reduced.

### Example 12: Evaluation of TCR$^{KO}$CD30-CAR$^{KI}$-T cells against lymphoma development and HIV load in humanized mice infected with both HIV and EBV

Humanized NSG mice (n=36; at 10 weeks after humanization) are obtained commercially from The Jackson Laboratory. 12 weeks after humanization, the mice are infected with HIV and/or EBV and treated with CD30-CAR-T cells on week 15. On week 23, they are sacrificed for analyses to evaluate the EBV load, HIV load, CAR-T persistence, biodistribution, and markers of autoimmunity.

### Example 13: Optimization of the SOPs for the generation of expanded HLA$^{KO}$TCR$^{KO}$CD30-CAR$^{KI}$-T for allogeneic uses

The simultaneous K/O of HLA class I and II is performed by gene disruption of b2M and CIITA according to Glaser, Wagner et al. (Glaser *et al.,* 2023) with modifications. In brief, a catalytically inactivate Cas9 endonuclease (nCas9) fused to an adenine base editor (ABE) is utilized to introduce a base exchange in a splicing acceptor or donor site of the b2M and CIITA loci. The simultaneous use of Cas12a for the KI of CAR into the TRAC locus in addition also eliminates the risk of sgRNA exchange (and thus unwanted DSB), as the sgRNAs of Cas9 and Cas12a are specific for their respective endonuclease. Sequences for gRNAs for b2M and CIITA are shown in Table 1. An *in vitro* assay to demonstrate lower alloreactivity of T cells against CD30-CAR-T cells is performed as described (Glaser *et al.,* 2023) and adapted to our target cells.

**Table 4:** Abbreviations

| Abbreviation | Description |
|---|---|
| CAR | Chimeric antigen receptor |
| CFSE | Carboxyfluoresceinsuccinimidylester |
| crRNA | CRISPR RNA |
| E:T | Effector to target |
| FACS | Fluorescence activated cells sorting/scanning |
| FCS | Fetal calf serum |

(continued)

| Abbreviation | Description |
|---|---|
| fLuc | Firefly luciferase |
| GFP | Green fluorescent protein |
| HDRT | Homology directed repair template |
| IL-15 | Interleukin 15 |
| IL-7 | Interleukin 7 |
| PBMC | Peripheral blood mononuclear cells |
| PBS | Phosphate buffered saline |
| PGA | poly(L-glutamic acid) |
| qPCR | quantitative polymerase chain reaction |
| RNP | Ribonuclear protein |
| TCR | T cell receptor |
| TRAC | TCR alpha constant |
| tracrRNA | transactivating CRISPR RNA |

**Table 5:** Antibodies used for flow cytometry

| Antigen | Dye | Clone | Dilution | Cat. Number | Source |
|---|---|---|---|---|---|
| AffiniPure F(ab')2 Fragment Goat Anti-Human IqG | Alexa Fluor 647 | polyclonal | 1:100 | 109-606-170 | Jackson Immuno Research Laboratories |
| CD45RA | FITC | HI100 | 1:100 | 304106 | Biolegend |
| CD62L | BV605 | DREG56 | 1:200 | 304835 | Biolegend |
| Fixable Viability Dye eFluor™ 450 | eFluor 450 | | 1:1000 | 65-0863-14 | Invitrogen™ |
| Hu CD3 | Alexa Fluor700 | HIT3a | 1:100 | 300324 | Biolegend |
| Hu CD30 | APC | BY88 | 1:100 | 333909 | Biolegend |
| Hu CD30 Isotype | APC | MOPC-21 | 1:100 | 400119 | Biolegend |
| Hu CD34 | PE | QBEND/10.rMAb | 1:100 | 568294 | BD Pharmingen™ |
| Hu CD4 | PerCP | OKT4 | 1:400 | 317432 | Biolegend |
| Hu CD8 | FITC | | 1:200 | | Biolegend |
| Hu CD8a | PE-Cy7 | HIT8a | 1:200 | 300914 | Biolegend |
| LAG-3 | PerCP/Cy5.5 | 11C3C65 | 1:25 | 369312 | Biolegend |
| PD1 | PE | EH12.2H7 | 1:100 | 329906 | Biolegend |
| TIM-3 | APC/Cy7 | F38-2E2 | 1:25 | 345026 | Biolegend |
| HIV-core antigen | FITC | KC57 | 1:100 | 6604665 | Beckman Coulter |

**Table 6:** Cells used for the studies

| Cell line | Cell type | Species | Reference |
|---|---|---|---|
| L-540 | Hodgkin lymphoma | human (Homo sapiens) | DSMZ ACC 72 |
| L-591 | B lymphoblastoid cells | human (Homo sapiens) | DSMZ ACC 602 |
| KM-H2 | Hodgkin lymphoma | human (Homo sapiens) | DSMZ ACC 8 |

(continued)

| Cell line | Cell type | Species | Reference |
|---|---|---|---|
| LCL | lymphoblastoid B lines | human (Homo sapiens) | Not applicable |

**Table 7.** Examples of gRNA sequences for β2M and CIITA knock-out.

| CRISPR guide RNA format | Guide RNA and reference | Target sequence (without PAM) |
|---|---|---|
| crRNA AsCas12a | TRAC 2 (Kath et al. 2022) | GAGUCUCUCAGCUGGUACACGGC |
| sgRNA SpCas9 | β2M (Gaudelli 2020) | CUUACCCCACUUAACUAUCU |
| sgRNA SpCas9 | CIITA (Gaudelli 2020) | ACGAGUCCGAUUCCACUCAC |

**REFERENCES**

1. Patrick J. Hanley et al., CMV-specific T cells generated from naive T cells recognize atypical epitopes and may be protective in vivo. Sci. Transl. Med.7, 285ra63-285ra63 (2015). DOI: 10.1126/scitranslmed.aaa2546

2. van der Weyden, C., Pileri, S., Feldman, A. et al. Understanding CD30 biology and therapeutic targeting: a historical perspective providing insight into future directions. Blood CancerJ. 7, e603 (2017). https://doi.org/10.1038/bcj.2017.85

3. Prator CA, Thanh C, Kumar S, Pan T, Peluso MJ, Bosch R, Jones N, Milush JM, Bakkour S, Stone M, Busch MP, Deeks SG, Hunt PW, Henrich TJ. Circulating CD30+CD4+ T Cells Increase Before Human Immunodeficiency Virus Rebound After Analytical Antiretroviral Treatment Interruption. J Infect Dis. 2020 Mar 16;221(7):1146-1155. doi: 10.1093/infdis/jiz572. PMID: 31677350; PMCID: PMC7325710.

4. Watanabe M, Hatsuse H, Nagao K, Tanaka Y, Watanabe T, Horie R. CD30 stimulation induces multinucleation and chromosomal instability in HTLV-1-infected cell lines. Int J Hematol. 2023 Jul;118(1):75-87. doi: 10.1007/s12185-023-03583-1. Epub 2023 Apr 4. PMID: 37014603.

5. Barbieri A, Dolcino M, Tinazzi E, Rigo A, Argentine G, Patuzzo G, Ottria A, Beri R, Puccetti A, Lunardi C. Characterization of CD30/CD30L(+) Cells in Peripheral Blood and Synovial Fluid of Patients with Rheumatoid Arthritis. J Immunol Res. 2015;2015:729654. doi: 10.1155/2015/729654. Epub 2015 May 19. PMID: 26090498; PMCID: PMC4452350.

6. Soldan, S.S., Lieberman, P.M. Epstein-Barr virus and multiple sclerosis. Nat Rev Microbiol 21, 51-64 (2023). https://doi.org/10.1038/s41579-022-00770-5

7. Bengtsson, A. (2001), The role of CD30 in atopic disease. Allergy, 56: 593-603. https://doi.org/10.1034/j.1398-9995.2001.00137.x

8. Polte T, Behrendt AK, Hansen G. Direct evidence for a critical role of CD30 in the development of allergic asthma. J Allergy Clin Immunol. 2006 Oct;118(4):942-8. doi: 10.1016/j.jaci.2006.07.014. Epub 2006 Sep 6. PMID: 17030250.

9. Dummer W, Rose C, Bröcker EB. Expression of CD30 on T helper cells in the inflammatory infiltrate of acute atopic dermatitis but not of allergic contact dermatitis. Arch Dermatol Res. 1998 Nov;290(11):598-602. doi: 10.1007/s004030050358. PMID: 9860279.

10. Choi S, Pegues MA, Lam N, Geldres C, Vanasse D, Kochenderfer JN. Design and Assessment of Novel Anti-CD30 Chimeric Antigen Receptors with Human Antigen-Recognition Domains. Hum Gene Ther. 2021 Jul;32(13-14):730-743. doi: 10.1089/hum.2020.215. Epub 2021 Feb 22. PMID: 33287637; PMCID: PMC8312022.

11. Guo J, He S, Zhu Y, Yu W, Yang D, Zhao X. Humanized CD30-Targeted Chimeric Antigen Receptor T Cells Exhibit Potent Preclinical Activity Against Hodgkin's Lymphoma Cells. Front Cell Dev Biol. 2022 Jan 12;9:775599. doi:

10.3389/fcell.2021.775599. PMID: 35096811; PMCID: PMC8790321.

12. Braun T, Pruene A, Darguzyte M, Vom Stein AF, Nguyen PH, Wagner DL, Kath J, Roig-Merino A, Heuser M, Riehm LL, Schneider A, Awerkiew S, Talbot SR, Bleich A, Figueiredo C, Bornhäuser M, Stripecke R. Non-viral TRAC-knocked-in CD19KICAR-T and gp350KICAR-T cells tested against Burkitt lymphomas with type 1 or 2 EBV infection: In vivo cellular dynamics and potency. Front Immunol. 2023 Mar 24;14:1086433. doi: 10.3389/fimmu.2023.1086433. PMID: 37033919; PMCID: PMC10081580.

**Claims**

1. A genetically modified cytotoxic cell, comprising a chimeric antigen receptor (CAR) that binds CD30, for use in the treatment of an infection, in which infected cells express CD30.

2. The genetically modified cytotoxic cell for use according to claim 1, wherein the treatment comprises the cytotoxic cell binding to a human target cell, which is infected with a chronic inflammatory virus and expresses CD30, and activation of said cytotoxic cell, thereby inducing cytotoxic activity against said human target cell and/or a bystander inflammatory cell.

3. The genetically modified cytotoxic cell for use according to any of the preceding claims, wherein the treatment comprises cytotoxic activity of the cytotoxic cell against a reservoir of infected human CD30-expressing target cells in a subject with a chronic viral infection, said reservoir comprising a latent and/or lytic viral reservoir, preferably wherein said treatment prevents viral pathology or reactivation.

4. The genetically modified cytotoxic cell for use according to the preceding claim, wherein the reservoir comprises CD30-expressing B cells, T cells, myeloid cells, and/or epithelial cells.

5. The genetically modified cytotoxic cell for use according to claim 1, wherein the treatment comprises the cytotoxic cell binding to a human target cell, which is infected with an acute inflammatory virus and expresses CD30, and activation of said cytotoxic cell, thereby inducing cytotoxic activity against said human target cell and/or a bystander inflammatory cell.

6. The genetically modified cytotoxic cell for use according to any of the preceding claims, wherein the treatment comprises cytotoxic activity of the cytotoxic cell against a pre-cancerous and/or non-cancerous human CD30-expressing target cell.

7. The genetically modified cytotoxic cell for use according to any of the preceding claims, wherein the viral infection is a herpes virus infection, preferably Epstein-Barr virus (EBV) or human cytomegalovirus (HCMV) infection.

8. The genetically modified cytotoxic cell for use according to any of claims 1 to 5, wherein the viral infection is a retrovirus infection, preferably a human immunodeficiency virus (HIV) or human T-cell lymphotropic virus (HTLV) infection.

9. The genetically modified cytotoxic cell for use according to any of the preceding claims, wherein the viral infection is an Epstein-Barr virus infection and/or a human immunodeficiency virus (HIV) infection.

10. The genetically modified cytotoxic cell for use according to claim 1, wherein the infection is a bacterial infection, preferably a tuberculosis infection.

11. The genetically modified cytotoxic cell for use according to any of the preceding claims, wherein an autoimmune disease results from the infection, preferably rheumatoid arthritis, systemic lupus erythematosus (SLE), multiple sclerosis (scleroderma), Sjörgen's syndrome, Hashimoto's thyroiditis, Wegner's granulomatosis, primary biliary cirrhosis, anti-phospholipid syndrome, uveitis and/or vasculitis; an allergic condition, preferably atopic dermatitis, asthma, and/or allergic rhinitis; and/or a disease associated with inflammation.

12. The genetically modified cytotoxic cell for use according to any of the preceding claims, wherein the cytotoxic cell is an immune effector cell, preferably a lymphocyte, more preferably a T cell or natural killer (NK) cell.

13. The genetically modified cytotoxic cell for use according to any of the preceding claims, wherein a nucleic acid construct comprising a sequence encoding a CAR that binds CD30 (CD30-CAR) is integrated into the genome of said cytotoxic cell, and said sequence encoding the CD30-CAR is expressed from an endogenous promoter.

14. The genetically modified cytotoxic cell for use according to any of the preceding claims, wherein a nucleic acid construct comprising a sequence encoding a CD30-CAR is integrated into an endogenous T-cell receptor locus, preferably a TCR alpha (TRAC) locus, in the genome of said cytotoxic cell.

15. The genetically modified cytotoxic cell for use according to any of the preceding claims, wherein the cytotoxic cell is genetically modified by integrating a nucleic acid construct comprising a sequence encoding a CD30-CAR into the genome of said cytotoxic cell by gene editing, preferably by CRISPR/Cas gene editing, more preferably without a viral vector.

16. The genetically modified cytotoxic cell for use according to any of the preceding claims, wherein said cytotoxic cell is generated by gene editing and lacks expression of a functional endogenous T cell receptor and/or human leukocyte antigens and/or CD30, and preferably can be administered in an allogeneic setting, and preferably does not cause graft-versus-host disease and/or fratricide effects.

**Figure 1**

A

| TRAC homology 5' | P2A | SP | scFv and linker | IgG₄ hinge | Detection Domain | CD28 TMD | CD28 Endo | CD3 zeta | bGH Poly A | TRAC homology 3' |

Mouse anti-CD19: FMC63 (reference)
Mouse anti-CD30: HRS-3 (V$_H$V$_L$)
Humanized anti-CD30: 5F11 (V$_L$V$_H$), 2H9 (V$_L$V$_H$ or V$_H$V$_L$),

Human Fc IgG₁- CH₃ (CAR-FcIg)
Human truncated CD34 (CAR-tCD34)

B

| PBMC | Activation | Transfection/ | Expansion | Quality Control | Potency assays | Repeated |
| apheresis | R10 or | Electroporation | R10 or | flow cytometry | with targets | antigen |
| (frozen) | X-Vivo15 | gRNAs, Cas9, | X-Vivo15 | CD3--KO, CAR-KI, | EBV-negative | exposures |
| or buffy-coat | media plus | HDRT, PGA, | media plus | CD8, CD4, | EBV-positive | *in vitro* |
| (fresh or | anti-CD28, | Enhancer | anti-CD28, | phenotypipic | HIV-positive | |
| frozen) | IL-7, IL-15, IL-2 | | IL-7, IL-15, IL-2 | markers | | |

**Days -2 to -5: Pre-editing    Day 0-1: Editing        Days 2 onwards: expansion, QC, function**

C

Control No electroporation | TCR KO with gRNAs | KI FMC63 (V$_L$V$_H$) CD19CAR-FcIg | KI HRS-3 (V$_H$V$_L$) CD30CAR-FcIg | KI 5F11 (V$_L$V$_H$) CD30CAR-FcIg

(MID 015 N=1)

D

KI 2H9 (V$_L$V$_H$) CD30CAR-FcIg | KI 2H9 (V$_H$V$_L$) CD30CAR-FcIg

(MID 064 N=3)

E  KI 5F11 (V$_L$V$_H$) CD30CAR-tCD34 expansion in X-VIVO-15

(NES 002 N=1)

**Figure 2**

Figure 2 (cont.)

Figure 2 (cont.)

**D**  **KI 5F11 (V$_L$V$_H$) CD30CAR-tCD34 expansion in X-VIVO-15**

(NES 002 N=1)

**Figure 3**

**A** Parental cell line

L-540 KM-H2

Transduced, sorted, selected clones

L-540 KM-H2

**B**

L540/fLuc-GFP

KM-H2/fLuc-GFP

(JMK 016 N=1)

**Figure 3 (cont.)**

C

L540/fLuc-GFP

KM-H2/fLuc-GFP

(JMK 016 N=1)

**Figure 4**

**Figure 4 (cont.)**

B                    L591/fLuc GFP

(MID 020 N=1)

L591/fLuc GFP

(MID 037 N=1)

C                    L591/fLuc GFP

(MID 067 N=2)

**Figure 4 (cont.)**

LCL (B95.8-fLuc)

(MID 020 N=1)

LCL (M81-fLuc)

(MID 020 N=1)

**Figure 5**

Figure 5 (cont.)

Figure 5 (cont.)

Figure 6

**A**

**B**

Effector-to-target ratios

**C**

(MID 063 N=1)

Europäisches
Patentamt

European
Patent Office

Office européen
des brevets

# EUROPEAN SEARCH REPORT

**Application Number**

EP 24 18 5787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/167187 A1 (QUACH DAVID H [US] ET AL) 1 June 2023 (2023-06-01) | 1-13,15 | INV. A61K39/00 A61P31/18 A61P31/22 |
| Y | * paragraphs [0075] - [0080], [0088], [0107], [0555], [0556]; claim 1; example 5 * | 14,16 | |
| | ----- | | |
| Y | WO 2023/122337 A1 (SANA BIOTECHNOLOGY INC [US]) 29 June 2023 (2023-06-29) * paragraphs [0860], [1205] * | 14,16 | |
| | ----- | | |
| Y | WO 2019/149743 A1 (CELLECTIS [FR]) 8 August 2019 (2019-08-08) * page 4, paragraph 2; claim 11 * | 14,16 | |
| | ----- | | |

**TECHNICAL FIELDS SEARCHED (IPC)**

A61K
A61P
C12N

The present search report has been drawn up for all claims

| Place of search | Date of completion of the search | Examiner |
|---|---|---|
| The Hague | 15 December 2024 | Lonnoy, Olivier |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................
& : member of the same patent family, corresponding document

EPO FORM 1503 03.82 (P04C01)

# EP 4 674 430 A1

## ANNEX TO THE EUROPEAN SEARCH REPORT
## ON EUROPEAN PATENT APPLICATION NO.

EP 24 18 5787

15-12-2024

| Patent document cited in search report | | | Publication date | Patent family member(s) | | | Publication date |
|---|---|---|---|---|---|---|---|
| US 2023167187 | A1 | | 01-06-2023 | AU | 2021262833 | A1 | 08-12-2022 |
| | | | | AU | 2021263607 | A1 | 08-12-2022 |
| | | | | AU | 2021264089 | A1 | 08-12-2022 |
| | | | | CA | 3181371 | A1 | 04-11-2021 |
| | | | | CA | 3181374 | A1 | 04-11-2021 |
| | | | | CA | 3181377 | A1 | 04-11-2021 |
| | | | | CN | 115916225 | A | 04-04-2023 |
| | | | | CN | 115996734 | A | 21-04-2023 |
| | | | | CN | 117396598 | A | 12-01-2024 |
| | | | | EP | 4142747 | A1 | 08-03-2023 |
| | | | | EP | 4142748 | A1 | 08-03-2023 |
| | | | | EP | 4142750 | A1 | 08-03-2023 |
| | | | | JP | 2023523619 | A | 06-06-2023 |
| | | | | JP | 2023523620 | A | 06-06-2023 |
| | | | | JP | 2023523621 | A | 06-06-2023 |
| | | | | KR | 20230016183 | A | 01-02-2023 |
| | | | | KR | 20230017194 | A | 03-02-2023 |
| | | | | KR | 20230018376 | A | 07-02-2023 |
| | | | | TW | 202206453 | A | 16-02-2022 |
| | | | | US | 2023167187 | A1 | 01-06-2023 |
| | | | | US | 2023172986 | A1 | 08-06-2023 |
| | | | | US | 2023220097 | A1 | 13-07-2023 |
| | | | | WO | 2021221927 | A1 | 04-11-2021 |
| | | | | WO | 2021222927 | A1 | 04-11-2021 |
| | | | | WO | 2021222928 | A1 | 04-11-2021 |
| | | | | WO | 2021222929 | A1 | 04-11-2021 |
| WO 2023122337 | A1 | | 29-06-2023 | AU | 2022422147 | A1 | 04-07-2024 |
| | | | | CA | 3241438 | A1 | 29-06-2023 |
| | | | | CN | 119072319 | A | 03-12-2024 |
| | | | | EP | 4452285 | A1 | 30-10-2024 |
| | | | | IL | 313704 | A | 01-08-2024 |
| | | | | KR | 20240137574 | A | 20-09-2024 |
| | | | | WO | 2023122337 | A1 | 29-06-2023 |
| WO 2019149743 | A1 | | 08-08-2019 | AU | 2019216269 | A1 | 28-05-2020 |
| | | | | EP | 3694546 | A1 | 19-08-2020 |
| | | | | US | 2023158070 | A1 | 25-05-2023 |
| | | | | WO | 2019149743 | A1 | 08-08-2019 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017066122 A1
- WO 2017066122 A
- WO 2022136551 A
- US 4816567 A
- US 5807715 A
- US 5866692 A
- US 6331415 B
- US 5530101 A
- US 5693761 A
- US 5693762 A
- US 5585089 A
- US 6180370 B
- US 5225539 A
- US 6548640 B
- WO 9007861 A, Queen
- WO 9117271 A
- WO 9201047 A
- WO 9960846 A

- US 6352694 B
- US 6534055 B
- US 6905680 B
- US 6692964 B
- US 5858358 A
- US 6887466 B
- US 6905681 B
- US 7144575 B
- US 7067318 B
- US 7172869 B
- US 7232566 B
- US 7175843 B
- US 5883223 B
- US 6905874 B
- US 6797514 B
- US 6867041 B
- US 20060121005

### Non-patent literature cited in the description

- **VAN DER WEYDEN**. *Blood Cancer J.*, 2017
- **PRATOR**. *J Infect Dis*, 2020
- **WATANABE**. *Int J Hematol.*, 2023
- **BARBIERI**. *J Immunol Res*, 2015
- **KABAT et al.** Sequences of Proteins of Immunological Interest. National Institutes of Health, 1991
- **AL-LAZIKANI et al.** *J. Molec. Biol.*, 1997, vol. 273, 927-948
- **RAFTERY MJ**. *Ann Rev of Cancer Biol*, 2023
- **HONGGUANG F et al.** *Sci Rep*, 2020
- **BRAUN et al.** *Frontiers in Immunology*, 24 March 2023, vol. 14, 1086433
- **PATRICK J. HANLEY et al.** CMV-specific T cells generated from naive T cells recognize atypical epitopes and may be protective in vivo.. *Sci. Transl. Med.*, 2015, vol. 7, 285ra63-285ra63
- **VAN DER WEYDEN, C** ; **PILERI, S** ; **FELDMAN, A. et al.** Understanding CD30 biology and therapeutic targeting: a historical perspective providing insight into future directions.. *Blood Cancer J.*, 2017, vol. 7, e603, https://doi.org/10.1038/bcj.2017.85

- **PRATOR CA** ; **THANH C** ; **KUMAR S** ; **PAN T** ; **PELUSO MJ** ; **BOSCH R** ; **JONES N** ; **MILUSH JM** ; **BAKKOUR S** ; **STONE M**. Circulating CD30+CD4+ T Cells Increase Before Human Immunodeficiency Virus Rebound After Analytical Antiretroviral Treatment Interruption. *J Infect Dis.*, 16 March 2020, vol. 221 (7), 1146-1155
- **WATANABE M** ; **HATSUSE H** ; **NAGAO K** ; **TANAKA Y** ; **WATANABE T** ; **HORIE R.** CD30 stimulation induces multinucleation and chromosomal instability in HTLV-1-infected cell lines.. *Int J Hematol.*, 04 April 2023, vol. 118 (1), 75-87
- **BARBIERI A** ; **DOLCINO M** ; **TINAZZI E** ; **RIGO A** ; **ARGENTINE G** ; **PATUZZO G** ; **OTTRIA A** ; **BERI R** ; **PUCCETTI A** ; **LUNARDI C.** Characterization of CD30/CD30L(+) Cells in Peripheral Blood and Synovial Fluid of Patients with Rheumatoid Arthritis. *J Immunol Res.*, 19 May 2015, vol. 2015, 729654
- **SOLDAN, S.S.** ; **LIEBERMAN, P.M.** Epstein-Barr virus and multiple sclerosis.. *Nat Rev Microbiol*, 2023, vol. 21, 51-64, https://doi.org/10.1038/s41579-022-00770-5
- **BENGTSSON, A.** The role of CD30 in atopic disease. *Allergy*, 2001, vol. 56, 593-603, https://doi.org/10.1034/j.1398-9995.2001.00137.x

- **POLTE T** ; **BEHRENDT AK** ; **HANSEN G**. Direct evidence for a critical role of CD30 in the development of allergic asthma. *J Allergy Clin Immunol.*, 06 September 2006, vol. 118 (4), 942-8
- **DUMMER W** ; **ROSE C** ; **BRÖCKER EB.** Expression of CD30 on T helper cells in the inflammatory infiltrate of acute atopic dermatitis but not of allergic contact dermatitis.. *Arch Dermatol Res.*, November 1998, vol. 290 (11), 598-602
- **CHOI S** ; **PEGUES MA** ; **LAM N** ; **GELDRES C** ; **VANASSE D** ; **KOCHENDERFER JN.** Design and Assessment of Novel Anti-CD30 Chimeric Antigen Receptors with Human Antigen-Recognition Domains.. *Hum Gene Ther.*, 22 February 2021, vol. 32 (13-14), 730-743
- **GUO J** ; **HE S** ; **ZHU Y** ; **YU W** ; **YANG D** ; **ZHAO X.** Humanized CD30-Targeted Chimeric Antigen Receptor T Cells Exhibit Potent Preclinical Activity Against Hodgkin's Lymphoma Cells.. *Front Cell Dev Biol.*, 12 January 2022
- **BRAUN T** ; **PRUENE A** ; **DARGUZYTE M** ; **VOM STEIN AF** ; **NGUYEN PH** ; **WAGNER DL** ; **KATH J** ; **ROIG-MERINO A** ; **HEUSER M** ; **RIEHM LL**. Non-viral TRAC-knocked-in CD19KICAR-T and gp350KICAR-T cells tested against Burkitt lymphomas with type 1 or 2 EBV infection: In vivo cellular dynamics and potency.. *Front Immunol.*, 24 March 2023